# EUROPEAN PATENT APPLICATION

(11) **EP 1 650 194 A1**
(43) Date of publication of application: **26.04.2006**
(21) Application number: 04771303.7
(22) Date of filing: 30.07.2004
(51) Int. Cl.: C07D 231/56, C07D 401/06, A61K 31/416, A61K 31/4439, A61K 31/454, A61K 31/5377, A61K 31/547, A61P 35/00, A61P 35/02, A61P 43/00

(54) **PROTEIN KINASE INHIBITOR**

(30) Priority: 30.07.2003 JP 2003203509
(71) Applicant: KYOWA HAKKO KOGYO CO., LTD., Chiyoda-ku, Tokyo 100-8185 (JP)
(72) Inventor: SHIOTSU, Yukimasa, Kyowa Hakko Kogyo Co.,Ltd,, Shizuoka 411-8731 (JP); UMEHARA, Hiroshi, Kyowa Hakko Kogyo Co. Ltd,, Sunto-gun, Shizuoka 411-8731 (JP); KANAI, Fumihiko c/o Kyowa Hakko Kogyo Co., Ltd., Sunto-gun, Shizuoka 411-8731 (JP); NARA, Shinji, c/o Kyowa Hakko Kogyo Co., Ltd,, Sunto-gun, Shizuoka 411-8731 (JP); OHTA, Yoshihisa c/o Kyowa Hakko Kogyo Co., Ltd., Sunto-gun, Shizuoka 411-8731 (JP); KANDA, Yutaka, c/o Kyowa Hakko Kogyo Co., Ltd.,, Sunto-gun, Shizuoka 411-8731 (JP); AKINAGA, Shiro, c/o Head Office Kyowa Hakko, Tokyo 100-8185 (JP); NAOE, Tomoki, Nagoya-shi, Aichi 466-0016 (JP); KIYOI, Hitoshi, Nogaya-shi, Aichi 463-0037 (JP); NAKAZATO, Tomoyuki, c/o Kyowa Hakko Kogyo Co.,Ltd,, Sunto-gun, Shizuoka 411-8731 (JP); TASHIRO, Satoshi, c/o Kyowa Hakko Kogyo Co., Ltd,, Sunto-gun, Shizuoka 411-8731 (JP)
(74) Representative: Harding, Charles Thomas
(86) International application number: PCT/JP2004/011300
(87) International publication number: WO 2005/012258

(57) **Abstract**

The present invention provides a protein kinase inhibitor (excluding c-Jun N-terminal kinase inhibitor) which comprises, as an active ingredient, an indazole derivative represented by Formula (I) 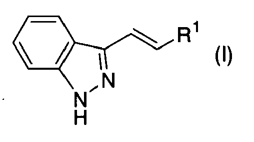 (wherein R¹ represents substituted or unsubstituted aryl or a substituted or unsubstituted heterocyclic group) or a pharmaceutically acceptable salt thereof.

## Description

### Technical Field

The present invention relates to a protein kinase inhibitor which comprises, as an active ingredient, an indazole derivative or a pharmaceutically acceptable salt thereof or the like.

### Background Art

Fms like tyrosine kinase 3 (hereinafter referred to as "Flt-3") is a receptor protein tyrosine kinase (PTK) and is a member of the platelet-derived growth factor receptor (PDGFR) family. Flt-3 is dimerized and thereby activated by the binding with Flt-3 ligand, phosphorylates a variety of proteins as intracellular substrates and is involved in cell proliferation and differentiation. Flt-3 or fetal liver kinase-2 (Flk-2) is known to express specifically in the hematopoietic stem cell and plays a significant role in the proliferation of the hematopoietic stem cell [Cell, vol.65, p.1143 (1991)]. Internal tandem duplication (ITD) within the juxtamembrane domain of Flt-3, in which a repetitive sequence of tyrosine residue is inserted, results in the activation of Flt-3 without the binding of ligand. This is revealed as a result of the investigations on specimens of leukemia patients [Leukemia, vol.11, p.1447 (1997)]. It is known that mutations including elongation or shorteining of the amino acid sequence within the juxtamembrane domain of Flt-3 yield similar activation of Flt-3 [Blood, vol.96, p.3907 (2000)]. In addition, a point mutation of amino acid in the kinase domain of Flt-3 results in constitutive activation of Flt-3 [Blood, vol.97, p.2434 (2001)]. The constitutive activation of Flt-3 based on these mutations is considered to induce infinite reproduction of cells by transmitting cell proliferation signals and be an important cause of leukemia.

As described above, examples of known mutations of Flt-3 include insertion of a repetitive sequence of tyrosine residue within the juxtamembrane domain, change in length of the juxtamembrane domain, and point mutation of amino acid in the kinase domain of Flt-3. Introducing any of these mutant genes into a cytokine-dependent cell strain, such as 32D cell, gives cytokine-independent proliferation capability to the cell strain. Accordingly, Flt-3 inhibitors are considered to be useful as therapeutic agents for a variety of cancers such as leukemia.

Staurosporine has been widely known as a kinase inhibitor [Biochemical & Biophysical Research Communications, vol.135, p.397 (1986)]. Staurosporine, however, inhibits a variety of kinases non-selectively and therefore is fatal on animals such as mice when administered. Imatinib, researched and developed as a selective kinase inhibitor, selectively inhibits Abl kinase and therefore shows low toxicity and high clinical effects on patients with chronic leukemia [New England Journal of Medicine, vol.345, p.645 (2002)]. As Flt-3 inhibitors, quinazoline derivatives or the like have been known (WO02/036587). As indazole derivatives, various compounds have been known [Japanese Published Unexamined Patent Application (Kokai) No. 32059/1990; WO01/53268; WO02/10137; and Khimiya Geterotsiklicheskikh Soedinenii, vol.7, pp.957-959 (1978)].

In Japanese published Unexamined Patent Application (Kokai) No. 32059/1990, compounds represented by Formula (IV) 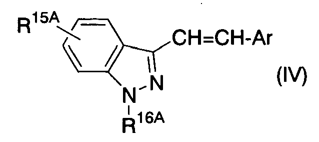 {wherein R^{15A} represents a hydrogen atom, nitro, NR^{15A1}R^{15A2} [wherein R^{15A1} and R^{15A2} may be the same or different and each represents a hydrogen atom, substituted or unsubstituted lower alkyl, lower alkanoyl (the carbon number in the lower alkanoyl is 1 to 6) or the like] or the like, R^{16A} represents a hydrogen atom or the like, Ar represents pyridyl, substituted or unsubstituted 2-oxochromenyl [the 2-oxochromenyl is bonded to ethenyl (-CH=CH-) on its benzene ring, and the substituent(s) on the 2-oxochromenyl is lower alkyl having 1 to 6 carbon atom(s) or lower alkoxy having 1 to 6 carbon atom(s)], phenyl or substituted phenyl [substituents Q¹, Q² and Q³ in the substituted phenyl may be the same or different and each represents a hydrogen atom, halogen, hydroxy, nitro, nitroso, carboxy, lower alkyl having 1 to 6 carbon atom(s), lower alkoxy having 1 to 6 carbon atom(s), lower alkoxycarbonyl having 1 to 6 carbon atom(s), NR^{17A1}R^{17A2} (wherein R^{17A1} and R^{17A2} have the same meanings as R^{15A1} and R^{15A2} defined above, respectively), or O(CH₂)_{nd}NR^{17A3}R^{17A4} (wherein nd represents an integer of 1 to 6 and R^{17A3} and R^{17A4} have the same meanings as R^{15A1} and R^{15A2} defined above, respectively), or any two from the groups Q¹, Q² and Q³ are combined together to form -O(CR^{17A5}R^{17A6)}O-(wherein two terminal oxygen atoms are bonded to the phenyl at adjacent carbon atoms on the phenyl and R^{17A5} and R^{17A6} may be the same or different and each represents a hydrogen atom or lower alkyl having 1 to 6 carbon atom(s), or R^{17A5} and R^{17A6} are combined together to form alkylene having 4 or 5 carbon atoms), provided that the Q¹, Q² and Q³ which are the subtituents in the substituted phenyl are not simultaneously hydrogen atoms] are disclosed.

In WO01/53268, compounds having suppressive activity on cell differentiation represented by Formula (V) 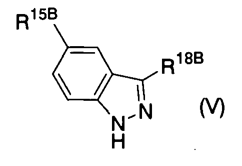 [wherein R^{18B} represents CH=CH-R^{18B1} (wherein R^{18B1} represents substituted or unsubstituted alkyl, substituted or unsubstituted aryl, a substituted or unsubstituted heterocyclic group or the like) and R^{15B} represents alkyl, aryl, CH=CH-R^{15B1} (wherein R^{15B1} represents substituted or unsubstituted aryl, substituted or unsubstituted heteroaryl or the like)] are disclosed.

In WO02/10137, compounds having inhibitory activity against c-jun N-terminal Kinase (JNK) represented by Formula (VI) 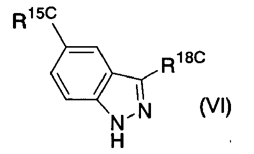 [wherein R^{18C} represents CH=CH-R^{18C1} (wherein R^{18C1} represents substituted or unsubstituted aryl, substituted or unsubstituted heteroaryl or the like) and R^{15C} represents halogen, hydroxy, amino or the like] are disclosed.

In Khimiya Geterotsiklicheskikh Soedinenii, vol.7, pp.957-959 (1978), compounds represented by Formula (VII) 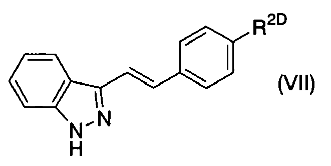 (wherein R^{2D} represents methoxy or nitro) are disclosed.

### Disclosure of the Invention

An object of the present invention is to provide a protein kinase inhibitor which comprises, as an active ingredient, an indazole derivative or a pharmaceutically acceptable salt thereof or the like.

The present invention relates to following (1) to (97).
(1) A protein kinase inhibitor (excluding c-jun N-terminal kinase inhibitor) which comprises, as an active ingredient, an indazole derivative represented by Formula (I) 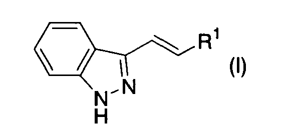 (wherein R¹ represents substituted or unsubstituted aryl or a substituted or unsubstituted heterocyclic group) or a pharmaceutically acceptable salt thereof.
(2) A protein kinase inhibitor (excluding c-jun N-terminal kinase inhibitor) which comprises, as an active ingredient, an indazole derivative represented by Formula (Ia) 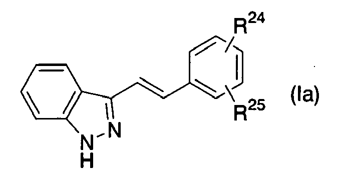 [wherein R²⁴ represents CONR^{24a}R^{24b} (wherein R^{24a} and R^{24b} may be the same or different and each represents a hydrogen atom, substituted or unsubstituted lower alkyl, substituted or unsubstituted aryl, substituted or unsubstituted aralkyl, or a substituted or unsubstituted heterocyclic group, or R^{24a} and R^{24b} are combined together with the adjacent nitrogen atom thereto to form a substituted or unsubstituted heterocyclic group) or NR^{24c}R^{24d} (wherein R^{24c} represents substituted or unsubstituted lower alkylsulfonyl or substituted or unsubstituted arylsulfonyl and R^{24d} represents a hydrogen atom or substituted or unsubstituted lower alkyl) and R²⁵ represents a hydrogen atom, halogen, cyano, nitro, hydroxy, carboxy, lower alkoxycarbonyl, substituted or unsubstituted lower alkyl, substituted or unsubstituted lower alkoxy, substituted or unsubstituted lower alkanoyl, CONR^{25a}R^{25b} (wherein R^{25a} and R^{25b} may be the same or different and each represents a hydrogen atom, substituted or unsubstituted lower alkyl, substituted or unsubstituted aryl, substituted or unsubstituted aralkyl or a substituted or unsubstituted heterocyclic group, or R^{25a} and R^{25b} are combined together with the adjacent nitrogen atom thereto to form a substituted or unsubstituted heterocyclic group) or NR^{25c}R^{25d} (wherein R^{25c} and R^{25d} may be the same or different and each represents a hydrogen atom, substituted or unsubstituted lower alkyl, substituted or unsubstituted lower alkanoyl, substituted or unsubstituted aroyl, substituted or unsubstituted heteroaroyl, substituted or unsubstituted aralkyl, substituted or unsubstituted lower alkylsulfonyl or substituted or unsubstituted arylsulfonyl)], or a pharmaceutically acceptable salt thereof.
(3) The protein kinase inhibitor (excluding c-jun N-terminal kinase inhibitor) according to (2), wherein R²⁴ is CONR^{24a}R^{25b} (wherein R^{24a} and R^{24b} have the same meanings as defined above, respectively) and R²⁵ is a hydrogen atom.
(4) The protein kinase inhibitor (excluding c-jun N-terminal kinase inhibitor) according to (2), wherein R²⁴ is NR^{24c}R^{24d} (wherein R^{24c} and R^{24d} have the same meanings as defined above, respectively) and R²⁵ is substituted or unsubstituted lower alkoxy.
(5) The protein kinase inhibitor (excluding c-jun N-terminal kinase inhibitor) according to (2), wherein R²⁴ is NR^{24c}R^{24d} (wherein R^{24c} and R^{24d} have the same meanings as defined above, respectively) and R²⁵ is a hydrogen atom.
(6) The protein kinase inhibitor according to any of (1) to (5), wherein protein kinase is Fms like tyrosine kinase 3.
(7) An anticancer agent which comprises, as an active ingredient, the indazole derivative or the pharmaceutically acceptable salt thereof described in (1).
(8) The anticancer agent according to (7), wherein cancer is cancer of hematopoietic tumor, mammary cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, oral cavity and pharynx cancer, osteosarcoma, melanoma or brain neoplasm.
(9) The anticancer agent according to (7), wherein cancer is leukemia, myeloma or lymphoma.
(10) The anticancer agent according to (7), wherein cancer is solid carcinoma.
(11) An antitumor agent, which comprises, as an active ingredient, the indazole derivative or the pharmaceutically acceptable salt thereof described in (1).
(12) The antitumor agent according to (11), wherein tumor is hematopoietic tumor.
(13) The antitumor agent according to (11), wherein tumor is solid tumor.
(14) Use of the indazole derivative or the pharmaceutically acceptable salt thereof described in any of (1) to (5) for the manufacture of a protein kinase inhibitor (excluding c-jun N-terminal kinase inhibitor).
(15) Use of the indazole derivative or the pharmaceutically acceptable salt thereof described in any of (1) to (5) for the manufacture of an Fms like tyrosine kinase 3 inhibitor.
(16) Use of the indazole derivative or the pharmaceutically acceptable salt thereof described in (1) for the manufacture of an anticancer agent.
(17) Use of the indazole derivative or the pharmaceutically acceptable salt thereof described in (1) for the manufacture of a therapeutic agent for cancer of hematopoietic tumor, mammary cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, oral cavity and pharynx cancer, osteosarcoma, melanoma or brain neoplasm.
(18) Use of the indazole derivative or the pharmaceutically acceptable salt thereof described in (1) for the manufacture of a therapeutic agent for leukemia, myeloma or lymphoma.
(19) Use of the indazole derivative or the pharmaceutically acceptable salt thereof described in (1) for the manufacture of a therapeutic agent for solid carcinoma.
(20) Use of the indazole derivative or the pharmaceutically acceptable salt thereof described in (1) for the manufacture of an antitumor agent.
(21) Use of the indazole derivative or the pharmaceutically acceptable salt thereof described in (1) for the manufacture of a therapeutic agent for hematopoietic tumor.
(22) Use of the indazole derivative or the pharmaceutically acceptable salt thereof described in (1) for the manufacture of a therapeutic agent for solid tumor.
(23) A method for inhibiting protein kinase (excluding c-jun N-terminal kinase), comprising a step of administering an effective amount of the indazole derivative or the pharmaceutically acceptable salt thereof described in any of (1) to (5).
(24) A method for inhibiting Fms like tyrosine kinase 3, comprising a step of administering an effective amount of the indazole derivative or the pharmaceutically acceptable salt thereof described in any of (1) to (5).
(25) A method for treating cancer, comprising a step of administering an effective amount of the indazole derivative or the pharmaceutically acceptable salt thereof described in (1).
(26) A method for treating cancer of hematopoietic tumor, mammary cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, oral cavity and pharynx cancer, osteosarcoma, melanoma or brain neoplasm, comprising a step of administering an effective amount of the indazole derivative or the pharmaceutically acceptable salt thereof described in (1).
(27) A method for treating leukemia, myeloma or lymphoma, comprising a step of administering an effective amount of the indazole derivative or the pharmaceutically acceptable salt thereof described in (1).
(28) A method for treating solid carcinoma, comprising a step of administering an effective amount of the indazole derivative or the pharmaceutically acceptable salt thereof described in (1).
(29) A method for treating tumor, comprising a step of administering an effective amount of the indazole derivative or the pharmaceutically acceptable salt thereof described in (1).
(30) A method for treating hematopoietic tumor, comprising a step of administering an effective amount of the indazole derivative or the pharmaceutically acceptable salt thereof described in (1).
(31) A method for treating solid tumor, comprising a step of administering an effective amount of the indazole derivative or the pharmaceutically acceptable salt thereof described in (1).
(32) An indazole derivative represented by Formula (II) 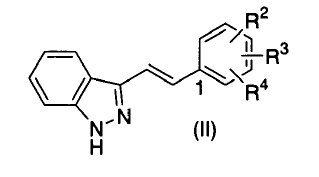 {wherein R², R³ and R⁴ may be the same or different and each represents a hydrogen atom, halogen, nitro, nitroso, carboxy, cyano, substituted or unsubstituted lower alkyl, substituted or unsubstituted lower alkanoyl, substituted or unsubstituted lower alkoxycarbonyl, substituted or unsubstituted aryl, NR⁵R⁶ (wherein R⁵ and R⁶ may be the same or different and each represents a hydrogen atom, substituted or unsubstituted lower alkyl, substituted or unsubstituted lower alkenyl, substituted or unsubstituted lower alkynyl, substituted or unsubstituted lower alkoxy, substituted or unsubstituted lower alkanoyl, substituted or unsubstituted aryl, substituted or unsubstituted aroyl, a substituted or unsubstituted heterocyclic group or substituted or unsubstituted heteroaroyl, or R⁵ and R⁶ are combined together with the adjacent nitrogen atom thereto to form a substituted or unsubstituted heterocyclic group) or OR⁷ (wherein R⁷ represents a hydrogen atom, substituted or unsubstituted lower alkyl, substituted or unsubstituted aryl, substituted or unsubstituted aroyl or a substituted or unsubstituted heterocyclic group) with the proviso that when two of R², R³ and R⁴ are hydrogen atoms, another one is not nitro, hydroxy, methoxy or 2-(dimethylamino)ethoxy bonding to 4-position of benzene ring [1-position of benzene ring is the position combined with (E)-2-(1H-indazol-3-yl)vinyl group]}, or a pharmaceutically acceptable salt thereof.
(33) The indazole derivative or the pharmaceutically acceptable salt thereof according to (32), wherein at least one of R², R³ and R⁴ is NR⁵R⁶ (wherein R⁵ and R⁶ have the same meanings as defined above, respesctively).
(34) The indazole derivative or the pharmaceutically acceptable salt thereof according to (32), wherein at least one of R², R³ and R⁴ is NR^{5a}R^{6a} (wherein R^{5a} and R^{6a} may be the same or different and each represents a hydrogen atom or substituted or unsubstituted lower alkanoyl, or R^{5a} and R^{6a} are combined together with adjacent nitrogen atom thereto to form a substituted or unsubstituted heterocyclic group).
(35) The indazole derivative or the pharmaceutically acceptable salt thereof according to (32), wherein at least one of R², R³ and R⁴ is OR⁷ (wherein R⁷ has the same meaning as defined above).
(36) The indazole derivative or the pharmaceutically acceptable salt thereof according to (32), wherein at least one of R², R³ and R⁴ is OR^{7a} (wherein R^{7a} represents substituted or unsubstituted lower alkyl).
(37) An indazole derivative represented by Formula (II-1) 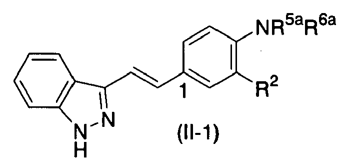 (wherein R², R^{5a} and R^{6a} have the same meanings as defind above, respectively) or a pharmaceutically acceptable salt thereof.
(38) An indazole derivative represented by Formula (II-2) 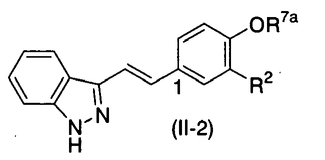 (wherein R² and R^{7a} have the same meanings as defind above, respectively) or a pharmaceutically acceptable salt thereof with the proviso that when R² is a hydrogen atom, R^{7a} is neither methyl nor 2-(dimethylamino)ethyl.
(39) A protein kinase inhibitor (excluding c-jun N-terminal kinase inhibitor) which comprises, as an active ingredient, the indazol derivative or the pharmaceutically acceptable salt thereof according to any of (32) to (38).
(40) The protein kinase inhibitor according to (39), wherein protein kinase is Fms like tyrosine kinase 3.
(41) A pharmaceutical composition which comprises, as an active ingredient, the indazol derivative or the pharmaceutically acceptable salt thereof according to any of (32) to (38).
(42) An anticancer agent which comprises, as an active ingredient, the indazol derivative or the pharmaceutically acceptable salt thereof according to any of (32) to (38).
(43) The anticancer agent according to (42), wherein cancer is cancer of hematopoietic tumor, mammary cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, oral cavity and pharynx cancer, osteosarcoma, melanoma or brain neoplasm.
(44) The anticancer agent according to (42), wherein cancer is leukemia, myeloma or lymphoma.
(45) The anticancer agent according to (42), wherein cancer is solid carcinoma.
(46) An antitumor agent, which comprises, as an active ingredient, the indazole derivative or the pharmaceutically acceptable salt thereof according to any of (32) to (38).
(47) The antitumor agent according to (46), wherein tumor is hematopoietic tumor.
(48) The antitumor agent according to (46), wherein tumor is solid tumor.
(49) Use of the indazole derivative or the pharmaceutically acceptable salt thereof according to any of (32) to (38) for the manufacture of a protein kinase inhibitor (excluding c-jun N-terminal kinase inhibitor).
(50) Use of the indazole derivative or the pharmaceutically acceptable salt thereof according to any of (32) to (38) for the manufacture of an Fms like tyrosine kinase 3 inhibitor.
(51) Use of the indazole derivative or the pharmaceutically acceptable salt thereof according to any of (32) to (38) for the manufacture of an anticancer agent.
(52) Use of the indazole derivative or the pharmaceutically acceptable salt thereof according to any of (32) to (38) for the manufacture of a therapeutic agent for cancer of hematopoietic tumor, mammary cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, oral cavity and pharynx cancer, osteosarcoma, melanoma or brain neoplasm.
(53) Use of the indazole derivative or the pharmaceutically acceptable salt thereof according to any of (32) to (38) for the manufacture of a therapeutic agent for leukemia, myeloma or lymphoma.
(54) Use of the indazole derivative or the pharmaceutically acceptable salt thereof according to any of (32) to (38) for the manufacture of a therapeutic agent for solid carcinoma.
(55) Use of the indazole derivative or the pharmaceutically acceptable salt thereof according to any of (32) to (38) for the manufacture of an antitumor agent.
(56) Use of the indazole derivative or the pharmaceutically acceptable salt thereof according to any of (32) to (38) for the manufacture of a therapeutic agent for hematopoietic tumor.
(57) Use of the indazole derivative or the pharmaceutically acceptable salt thereof according to any of (32) to (38) for the manufacture of a therapeutic agent for solid tumor.
(58) A method for inhibiting protein kinase (excluding c-jun N-terminal kinase), comprising a step of administering an effective amount of the indazole derivative or the pharmaceutically acceptable salt thereof according to any of (32) to (38).
(59) A method for inhibiting Fms like tyrosine kinase 3, comprising a step of administering an effective amount of the indazole derivative or the pharmaceutically acceptable salt thereof according to any of (32) to (38).
(60) A method for treating cancer, comprising a step of administering an effective amount of the indazole derivative or the pharmaceutically acceptable salt thereof according to any of (32) to (38).
(61) A method for treating cancer of hematopoietic tumor, mammary cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, oral cavity and pharynx cancer, osteosarcoma, melanoma or brain neoplasm, comprising a step of administering an effective amount of the indazole derivative or the pharmaceutically acceptable salt thereof according to any of (32) to (38).
(62) A method for treating leukemia, myeloma or lymphoma, comprising a step of administering an effective amount of the indazole derivative or the pharmaceutically acceptable salt thereof according to any of (32) to (38).
(63) A method for treating solid carcinoma, comprising a step of administering an effective amount of the indazole derivative or the pharmaceutically acceptable salt thereof according to any of (32) to (38).
(64) A method for treating tumor, comprising a step of administering an effective amount of the indazole derivative or the pharmaceutically acceptable salt thereof according to any of (32) to (38).
(65) A method for treating hematopoietic tumor, comprising a step of administering an effective amount of the indazole derivative or the pharmaceutically acceptable salt thereof according to any of (32) to (38).
(66) A method for treating solid tumor, comprising a step of administering an effective amount of the indazole derivative or the pharmaceutically acceptable salt thereof according to any of (32) to (38).
(67) An indazole derivative represented by Formula (III) 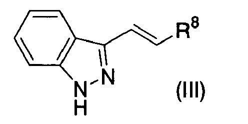 {wherein R⁸ represents a substituted or unsubstituted heterocyclic group [substituents in the substituted heterocyclic group may be the same or different, are 1 to 3 in number, and include oxo, formyl, carboxy, lower alkoxycarbonyl, substituted or unsubstituted lower alkyl, substituted or unsubstituted lower alkoxy, CONR⁹R¹⁰ (wherein R⁹ and R¹⁰ may be the same or different and each represents a hydrogen atom or substituted or unsubstituted lower alkyl), NR¹¹R¹² (wherein R¹¹ and R¹² may be the same or different and each represents a hydrogen atom, lower alkanoyl, lower alkoxycarbonyl, aralkyl, substituted or unsubstituted lower alkyl, substituted or unsubstituted aryl, substituted or unsubstituted aroyl or a substituted or unsubstituted heterocyclic group) or -O(CR¹³R¹⁴)ₙO- (wherein R¹³ and R¹⁴ may be the same or different and each represents a hydrogen atom or lower alkyl, n represents 2 or 3, and two terminal oxygen atoms are combined on the same carbon atom in the substituted heterocyclic group)]} or a pharmaceutically acceptable salt thereof.
(68) The indazole derivative or the pharmaceutically acceptable salt thereof according to (67), wherein substituents in the substituted heterocyclic group are amino, oxo, lower alkoxy, lower alkoxycarbonylamino, aroylamino or lower alkoxycarbonyl-lower alkyl.
(69) The indazole derivative or the pharmaceutically acceptable salt thereof according to (67), wherein R⁸ is 3-pyridyl.
(70) A protein kinase inhibitor (excluding c-jun N-terminal kinase inhibitor) which comprises, as an active ingredient, the indazol derivative or the pharmaceutically acceptable salt thereof according to any of (67) to (69).
(71) The protein kinase inhibitor according to (70), wherein protein kinase is Fms like tyrosine kinase 3.
(72) A pharmaceutical composition which comprises, as an active ingredient, the indazol derivative or the pharmaceutically acceptable salt thereof according to any of (67) to (69).
(73) An anticancer agent which comprises, as an active ingredient, the indazol derivative or the pharmaceutically acceptable salt thereof according to any of (67) to (69).
(74) The anticancer agent according to (73), wherein cancer is cancer of hematopoietic tumor, mammary cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, oral cavity and pharynx cancer, osteosarcoma, melanoma or brain neoplasm.
(75) The anticancer agent according to (73), wherein cancer is leukemia, myeloma or lymphoma.
(76) The anticancer agent according to (73), wherein cancer is solid carcinoma.
(77) An antitumor agent, which comprises, as an active ingredient, the indazole derivative or the pharmaceutically acceptable salt thereof according to any of (67) to (69).
(78) The antitumor agent according to (77), wherein tumor is hematopoietic tumor.
(79) The antitumor agent according to (77), wherein tumor is solid tumor.
(80) Use of the indazole derivative or the pharmaceutically acceptable salt thereof according to any of (67) to (69) for the manufacture of a protein kinase inhibitor (excluding c-jun N-terminal kinase inhibitor).
(81) Use of the indazole derivative or the pharmaceutically acceptable salt thereof according to any of (67) to (69) for the manufacture of an Fms like tyrosine kinase 3 inhibitor.
(82) Use of the indazole derivative or the pharmaceutically acceptable salt thereof according to any of (67) to (69) for the manufacture of an anticancer agent.
(83) Use of the indazole derivative or the pharmaceutically acceptable salt thereof according to any of (67) to (69) for the manufacture of a therapeutic agent for cancer of hematopoietic tumor, mammary cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, oral cavity and pharynx cancer, osteosarcoma, melanoma or brain neoplasm.
(84) Use of the indazole derivative or the pharmaceutically acceptable salt thereof according to any of (67) to (69) for the manufacture of a therapeutic agent for leukemia, myeloma or lymphoma.
(85) Use of the indazole derivative or the pharmaceutically acceptable salt thereof according to any of (67) to (69) for the manufacture of a therapeutic agent for solid carcinoma.
(86) Use of the indazole derivative or the pharmaceutically acceptable salt thereof according to any of (67) to (69) for the manufacture of an antitumor agent.
(87) Use of the indazole derivative or the pharmaceutically acceptable salt thereof according to any of (67) to (69) for the manufacture of a therapeutic agent for hematopoietic tumor.
(88) Use of the indazole derivative or the pharmaceutically acceptable salt thereof according to any of (67) to (69) for the manufacture of a therapeutic agent for solid tumor.
(89) A method for inhibiting protein kinase (excluding c-jun N-terminal kinase), comprising a step of administering an effective amount of the indazole derivative or the pharmaceutically acceptable salt thereof according to any of (67) to (69).
(90) A method for inhibiting Fms like tyrosine kinase 3, comprising a step of administering an effective amount of the indazole derivative or the pharmaceutically acceptable salt thereof according to any of (67) to (69).
(91) A method for treating cancer, comprising a step of administering an effective amount of the indazole derivative or the pharmaceutically acceptable salt thereof according to any of (67) to (69).
(92) A method for treating cancer of hematopoietic tumor, mammary cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, oral cavity and pharynx cancer, osteosarcoma, melanoma or brain neoplasm, comprising a step of administering an effective amount of the indazole derivative or the pharmaceutically acceptable salt thereof according to any of (67) to (69).
(93) A method for treating leukemia, myeloma or lymphoma, comprising a step of administering an effective amount of the indazole derivative or the pharmaceutically acceptable salt thereof according to any of (67) to (69).
(94) A method for treating solid carcinoma, comprising a step of administering an effective amount of the indazole derivative or the pharmaceutically acceptable salt thereof according to any of (67) to (69).
(95) A method for treating tumor, comprising a step of administering an effective amount of the indazole derivative or the pharmaceutically acceptable salt thereof according to any of (67) to (69).
(96) A method for treating hematopoietic tumor, comprising a step of administering an effective amount of the indazole derivative or the pharmaceutically acceptable salt thereof according to any of (67) to (69).
(97) A method for treating solid tumor, comprising a step of administering an effective amount of the indazole derivative or the pharmaceutically acceptable salt thereof according to any of (67) to (69).

The compounds represented by Formula (I), (Ia), (II) and (III) are hereinafter referred to as Compound (I), (Ia), (II) and (III), respectively. The same is true for compounds represented by other formula numbers.

In the definitions for each groups in Formula (I), (Ia), (II) and (III):
(i) The halogen includes fluorine, chlorine, bromine, and iodine atoms.
(ii) Examples of the lower alkyl and the lower alkyl moieties of the lower alkoxy, the lower alkoxycarbonyl, the lower alkoxycarbonylamino, the lower alkoxycarbonyl-lower alkyl and the lower alkylsulfonyl include, for example, linear, branched or cyclic alkyl or alkyl comprising these alkyls in combination, having 1 to 10 carbon atom(s). More specific examples thereof are as follows.
   (ii-a) Examples of the linear or branched lower alkyl include, for example, methyl, ethyl, *n*-propyl, isopropyl, *n*-butyl, isobutyl, sec-butyl, *tert*-butyl, *n-*pentyl, neopentyl, *n*-hexyl, *n*-heptyl, *n*-octyl, *n*-nonyl and *n*-decyl;
   (ii-b) examples of the cyclic lower alkyl include, for example, cyclopropyl, cyclobutyl, cyclopentyl, cyclohexyl, cycloheptyl, cyclooctyl, cyclodecyl, noradamantyl, adamantyl, bicyclo[2.2.1]heptyl, bicyclo[2.2.2]octyl, bicyclo[3.3.0]octyl and bicyclo[3.3.1]nonyl: and
   (ii-c) examples of the lower alkyl comprising linear or branched alkyl and cyclic alkyl include, for example, cyclopropylmethyl, cyclopentylmethyl and cyclooctylethyl.
(iii) The alkylene moiety of the lower alkoxycarbonyl-lower alkyl and the aralkyl has the same meaning as the group formed by removing one hydrogen atom from the linear or branched lower alkyl (ii-a) in the definition of the lower alkyl defined above.
(iv) Examples of the lower alkenyl include, for example, linear or branched alkenyl having 2 to 10 carbon atom(s) such as vinyl, allyl, 1-propenyl, 1-butenyl, 3-butenyl, 2-pentenyl, 4-pentenyl, 2-hexenyl, 5-hexenyl, 2-decenyl or 9-decenyl.
(v) Examples of the lower alkynyl include, for example, linear or branched alkynyl having 2 to 10 carbon atom(s) such as ethynyl, 2-propynyl, 3-butynyl, 4-pentynyl, 5-hexynyl or 9-decynyl.
(vi) Examples of the aryl and the aryl moieties of the aralkyl, the aroyl, the aroylamino and the arylsulfonyl include, for example, monocyclic aryl or fused aryl in which two or more rings are fused, and more specific examples include aryl having 6 to 14 carbon atoms as ring-constituting members, such as phenyl, naphthyl, indenyl or anthranyl.
(vii) Examples of the lower alkanoyl include, for example, linear, branched, or cyclic lower alkanyol, or lower alkanoyl comprising these lower alkanoyls in combination, having 1 to 8 carbon atom(s), such as formyl, acetyl, propionyl, butyryl, isobutyryl, valeryl, isovaleryl, pivaloyl, hexanoyl, heptanoyl, octanoyl, cyclopropylcarbonyl, cyclobutylcarbonyl, cyclopropylcarbonyl, cyclopropylmethylcarbonyl, cyclohexylcarbonyl, 1-methylcyclopropylcarbonyl or cycloheptylcarbonyl.
(viii) Examples of the heterocyclic group include, for example, heteroaromatic group and heteroalicyclic group.
   (viii-a) Examples of the heteroaromatic group include, for example, monocyclic heteroaromatic group or fused heteroaromatic group in which two or more rings are fused. The type and number of the heteroatom contained in heteroaromatic group are not specifically limited and the heteroaromatic group may contain, for example, one or more heteroatoms selected from the group consisting of nitrogen atom, sulfur atom and oxygen atom. More specific examples include heteroaromatic group having 5 to 14 atoms as ring-constituting members, such as furyl, thienyl, pyrrolyl, imidazolyl, pyrazolyl, triazolyl, tetrazolyl, oxazolyl, oxadiazolyl, thiazolyl, pyridyl, pyrazinyl, pyrimidinyl, pyridazinyl, triazinyl, indolyl, indazolyl, benzimidazolyl, benzoxazolyl, benzothiazolyl, quinolyl, isoquinolyl, phthalazinyl, naphthyridinyl, quinoxalinyl, quinazolinyl, cinnolinyl, purinyl or coumarinyl.
   (viii-b) Examples of the heteroalicyclic group include, for example, monocyclic heteroalicyclic group or fused heteroalicyclic group in which two or more rings are fused. The type and number of the heteroatom contained in heteroalicyclic groups are not specifically limited and the heteroalicyclic group may contain, for example, one or more heteroatoms selected from the group consisting of nitrogen atom, sulfur atom and oxygen atom. More specific examples include, for example, pyrrolidinyl, 2,5-dioxopyrrolidinyl, thiazolidinyl, oxazolidinyl, piperidyl, 1,2-dihydropyridyl, piperazinyl, homopiperazinyl, morpholinyl, thiomorpholinyl, pyrazolinyl, oxazolinyl, dioxolanyl, tetrahydropyranyl, tetrahydrothiopyranyl, tetrahydrofuryl, tetrahydroquinolyl, tetrahydroisoquinolyl, tetrahydroquinoxalinyl, octahydroquinolyl, dihydroindolyl and 1,3-dioxoisoindolinyl.
(ix) Examples of the heterocyclic group formed together with the adjacent nitrogen atom include 5- or 6-membered monocyclic heterocyclic group containing at least one nitrogen atom (the monocyclic heterocyclic group may further contain any other of a nitrogen atom, an oxygen atom and a sulfur atom) and bicyclic or tricyclic fused heterocyclic group containing at least one nitrogen atom in which 3- to 8-membered rings.are fused (the fused heterocyclic group may further contain any other of a nitrogen atom, an oxygen atom and a sulfur atom). More specific examples include, for example, pyrrolidinyl, piperidino, piperazinyl, morpholino, thiomorpholino, homopiperidino, homopiperazinyl, tetrahydropyridyl, tetrahydroquinolyl and tetrahydroisoquinolyl.
(x) The heteroaryl moiety in the heteroaroyl has the same meaning as the heteroaromatic group (viii-a) defined above.
(xi) Examples of the substituents in the substituted lower alkyl, the substituted lower alkoxy, the substituted lower alkenyl, the substituted lower alkynyl, the substituted lower alkanoyl, the substituted lower alkoxycarbonyl and the substituted lower alkylsulfonyl which may be the same or different and in number of 1 to 3, include
   (xi-a) hydroxy,
   (xi-b) lower alkoxy,
   (xi-c) oxo,
   (xi-d) carboxy,
   (xi-e) lower alkoxycarbonyl,
   (xi-f) heteroaroyl,
   (xi-g) arylsulfonyl,
   (xi-h) substituted or unsubstituted aryl (the substituent(s) in the substituted aryl is carboxy, lower alkoxy, lower alkoxycarbonyl or the like),
   (xi-i) a substituted or unsubstituted heterocyclic group (the substituent(s) in the substituted heterocyclic group is carboxy, lower alkoxy, lower alkoxycarbonyl or the like),
   (xi-j) CONR^{19a}R^{19b} {wherein R^{19a} and R^{19b} may be the same or different and each represents a hydrogen atom or substituted or unsubstituted lower alkyl [the substituent(s) in the substituted lower alkyl, which is 1 to 3 in number, is for example, halogen, hydroxy, oxo, nitro, cyano, carboxy, lower alkanoyl, lower alkoxycarbonyl, aroyl or substituted or unsubstituted lower alkoxy (the substituent(s) in the substituted lower alkoxy, which is 1 to 3 in number, is for example, hydroxy) or the like] or R^{19a} and R^{19b} are combined together with the adjacent nitrogen atom thereto to form a substituted or unsubstituted heterocyclic group [the substituent(s) in the substituted heterocyclic group formed together with the adjacent nitrogen atom, which is 1 to 3 in number, is for example, halogen, hydroxy, oxo, nitro, cyano, carboxy, lower alkanoyl, lower alkoxycarbonyl, aralkyl, aroyl, substituted or unsubstituted lower alkyl (the substituent(s) in the substituted lower alkyl, which is 1 to 3 in number, is for example, hydroxy), substituted or unsubstituted lower alkoxy (the substituent(s) in the substituted lower alkoxy, which is 1 to 3 in number, is for example, hydroxy) or the like]},
   (xi-k) NR^{20a}R^{20b} (wherein R^{20a} and R^{20b} have the same meanings as R^{19a} and R^{19b} defined above, respectively),
   (xi-l) lower alkanoylamino,
   (xi-m) N-(lower alkanoyl)-N-(lower alkyl)amino.
      In the definition of the substituents (xi) in the substituted lower alkyl, the substituted lower alkoxy, the substituted lower alkenyl, the substituted lower alkynyl, the substituted lower alkanoyl, the substituted lower alkoxycarbonyl and the substituted lower alkylsulfonyl, the halogen has the same meaning as (i) defined above; the lower alkyl and the lower alkyl moiety of the lower alkoxy, the lower alkoxycarbonyl and the N-(lower alkanoyl)-N-(lower alkyl)amino have the same meanings as (ii) defined above, respectively; the alkylene moiety of the aralkyl has the same meaning as (iii) defined above; the aryl and the aryl moiety of the aralkyl, the aroyl and the arylsulfonyl have the same meanings as (vi) defined above, respectively; the lower alkanoyl and the lower alkanoyl moiety of the lower alkanoylamino and the N-(lower alkanoyl)-N-(lower alkyl)amino have the same meanings as (vii) defined above, respectively; the heterocyclic group has the same meaning as (viii) defined above; the heterocyclic group formed together with the adjacent nitrogen atom has the same meaning as (ix) defined above; and the heteroaroyl has the same meaning as (x) defined above.
(xii) Examples of the substituents in the substituted aryl, the substituted aroyl, the substituted aralkyl, the substituted arylsulfonyl, the substituted heteroaroyl, the substituted heterocyclic group and the substituted heterocyclic group formed together with the adjacent nitrogen atom, which may be the same or different and is 1 to 3 in number, include
   (xii-a) halogen,
   (xii-b) nitro,
   (xii-c) nitroso,
   (xii-d) carboxy,
   (xii-e) substituted or unsubstituted lower alkyl [the substituent(s) in the substituted lower alkyl has the same meaning as (xi) defined above],
   (xii-f) substituted or unsubstituted lower alkenyl [the substituent(s) in the substituted lower alkenyl has the same meaning as (xi) defined above],
   (xii-g) substituted or unsubstituted lower alkynyl [the substituent(s) in the substituted lower alkynyl has the same meaning as (xi) defined above],
   (xii-h) substituted or unsubstituted lower alkoxycarbonyl [the substituent(s) in the substituted lower alkoxycarbonyl has the same meaning as (xi) defined above],
   (xii-i) substituted or unsubstituted lower alkanoyl [the substituent(s) in the substituted lower alkanoyl has the same meaning as (xi) defined above],
   (xii-j) substituted or unsubstituted aryl [the substituent(s) in the substituted aryl, which is 1 to 3 in number, is for example, halogen, hydroxy, nitro, cyano, carboxy, lower alkanoyl, lower alkoxycarbonyl, aralkyl, aroyl, substituted or unsubstituted lower alkyl (the substituent(s) in the substituted lower alkyl, which is 1 to 3 in number, is for example hydroxyl) and substituted or unsubstituted lower alkoxy (the substituent(s) in the substituted lower alkoxy, which is 1 to 3 in number, is for example hydroxyl)],
   (xii-k) NR^{21a}R^{21b} {wherein R^{21a} and R^{21b} may be the same or different and each represents a hydrogen atom, lower alkyl sulfonyl, substituted or unsubstituted lower alkyl [the substituent(s) in the substituted lower alkyl has the same meaning as (xi) defined above], substituted or unsubstituted lower alkenyl [the substituent(s) in the substituted lower alkenyl has the same meaning as (xi) defined above], substituted or unsubstituted lower alkynyl [the substituent(s) in the substituted lower alkynyl has the same meaning as (xi) defined above], substituted or unsubstituted lower alkoxy [the substituent(s) in the substituted lower alkoxy has the same meaning as (xi) defined above], substituted or unsubstituted lower alkanoyl [the substituent(s) in the substituted lower alkanoyl has the same meaning as (xi) defined above], substituted or unsubstituted aryl [the substituent(s) in the substituted aryl, which is 1 to 3 in number, is for example, halogen, hydroxy, nitro, cyano, carboxy, lower alkanoyl, lower alkoxycarbonyl, aralkyl, aroyl, substituted or unsubstituted lower alkyl (the substituent(s) in the substituted lower alkyl, which is 1 to 3 in number, is for example hydroxy) and substituted or unsubstituted lower alkoxy (the substituent(s) in the substituted lower alkoxy, which is 1 to 3 in number, is for example hydroxy)], substituted or unsubstituted aroyl [the substituent(s) in the substituted aroyl, which is 1 to 3 in number, is for example, halogen, hydroxy, nitro, cyano, carboxy, lower alkanoyl, lower alkoxycarbonyl, aralkyl, aroyl, substituted or unsubstituted lower alkyl (the substituent(s) in the substituted lower alkyl, which is 1 to 3 in number, is for example hydroxy) and substituted or unsubstituted lower alkoxy (the substituent(s) in the substituted lower alkoxy, which is 1 to 3 in number, is for example hydroxy)] or a substituted or unsubstituted heterocyclic group [the substituent(s) in the substituted heterocyclic group, which is 1 to 3 in number, is for example, halogen, hydroxy, nitro, cyano, carboxy, lower alkanoyl, lower alkoxycarbonyl, aralkyl, aroyl, substituted or unsubstituted lower alkyl (the substituent(s) in the substituted lower alkyl, which is 1 to 3 in number, is for example hydroxy) and substituted or unsubstituted lower alkoxy (the substituent(s) in the substituted lower alkoxy, which is 1 to 3 in number, is for example hydroxy)], or R^{21a} and R^{21b} are combined together with the adjacent nitrogen atom thereto to form a substituted or unsubstituted heterocyclic group [the substituent(s) in the substituted heterocyclic group formed with the adjacent nitrogen atom, which is 1 to 3 in number, is for example, halogen, amino, nitro, hydroxy, oxo, cyano, carboxy, lower alkoxycarbonyl, aralkyl, aroyl, heteroaroyl, substituted or unsubstituted lower alkyl (the substituent(s) in the substituted lower alkyl, which is 1 to 3 in number, is for example, hydroxy and lower alkoxy), substituted or unsubstituted lower alkoxy (the substituent(s) in the substituted lower alkoxy, which is 1 to 3 in number, is for example, hydroxy and lower alkoxy), substituted or unsubstituted lower alkanoyl (the substituent(s) in the substituted lower alkanoyl, which is 1 to 3 in number, is for example, amino, hydroxy, lower alkoxy, lower alkanoylamino and N-(lower alkanoyl)-N-(lower alkyl)amino) and substituted or unsubstituted heteroalicyclic-carbonyl (the substituent(s) in the substituted heteroalicyclic-carbonyl, which is 1 to 3 in number, is for example, halogen, hydroxy, oxo, lower alkyl and lower alkoxy)]},
   (xii-1) CONR^{21c}R^{21d} (wherein R^{21c} and R^{21d} have the same meanings as R^{21a} and R^{21b} defined above, respectively),
   (xii-m) OR²² {wherein R²² represents a hydrogen atom, substituted or unsubstituted lower alkyl [the substituent(s) in the substituted lower alkyl has the same meaning as (xi) defined above], substituted or unsubstituted aryl [the substituent(s) in the substituted aryl, which is 1 to 3 in number, is for example, halogen, hydroxy, nitro, cyano, carboxy, lower alkanoyl, lower alkoxycarbonyl, aralkyl, aroyl, substituted or unsubstituted lower alkyl (the substituent(s) in the substituted lower alkyl, which is 1 to 3 in number, is for example hydroxy) and substituted or unsubstituted lower alkoxy (the substituent(s) in the substituted lower alkoxy, which is 1 to 3 in number, is for example hydroxy)] or a substituted or unsubstituted heterocyclic group [the substituent(s) in the substituted heterocyclic group, which is 1 to 3 in number, is for example, halogen, hydroxy, nitro, cyano, carboxy, lower alkanoyl, lower alkoxycarbonyl, aralkyl, aroyl, substituted or unsubstituted lower alkyl (the substituent(s) in the substituted lower alkyl, which is 1 to 3 in number, is for example hydroxy) and substituted or unsubstituted lower alkoxy (the substituent(s) in the substituted lower alkoxy, which is 1 to 3 in number, is for example hydroxy)]},
   (xii-n) heteroaroyl,
   (xii-o) substituted or unsubstituted heteroalicyclic-carbonyl [the substituent(s) in the substituted heteroalicyclic-carbonyl, which is 1 to 3 in number, is for example, halogen, hydroxy, oxo, lower alkyl and lower alkoxy].
      The substituent(s) in the substituted heterocyclic group, and the substituent(s) in the substituted heterocyclic group formed with the adjacent nitrogen atom may be, in addition to (xii-a) to (xii-o), the following (xii-p) or (xii-q):
   (xii-p) oxo
   (xii-q) -O(CR^{23a}R^{23b})ₙO- (wherein R^{23a} and R^{23b} may be the same or different and each represents a hydrogen atom or lower alkyl, n represents 2 or 3, and the two terminal oxygen atoms are combined on the same carbon atom in the substituted heterocyclic group or the substituted heterocyclic group formed with the adjacent nitrogen atom)

In the definition of the substituents (xii) in the substituted aryl, the substituted aroyl, the substituted aralkyl, the substituted arylsulfonyl, the substituted heteroaroyl, the substituted heterocyclic group and the substituted heterocyclic group formed with the adjacent nitrogen atom, the halogen has the same meaning as (i) defined above; the lower alkyl and the lower alkyl moiety of the lower alkoxy, the lower alkoxycarbonyl and the lower alkylsulfonyl have the same meanings as (ii) defined above, respectively; the alkylene moiety of the aralkyl has the same meaning as (iii) defined above; the lower alkenyl has the same meaning as (iv) defined above; the lower alkynyl has the same meaning as (v) defined above; the aryl and the aryl moiety of the aroyl and the aralkyl have the same meanings as (vi) defined above, respectively; the lower alkanoyl and the lower alkanoyl moity of the lower alkanoylamino and the N-(lower alkanoyl)-N-(lower alkyl)amino have the same meaning as (vii) defined above, respectively; the heterocyclic group has the same meaning as (viii) defined above; the heteroalicyclic moiety of the heteroalicyclic-carbonyl has the same meaning as (viii-b) defined above; the heterocyclic group formed with the adjacent nitrogen atom has the same meaning as (ix) defined above; the heteroaroyl has the same meaning as (x) defined above.

Examples of the pharmaceutically acceptable salts of Compound (I), (Ia), (II) and (III) include, for example, pharmaceutically acceptable acid addition salts, metal salts, ammonium salts, organic amine addition salts, and amino acid addition salts. The acid addition salts include, for example, inorganic acid salts such as hydrochlorides, sulfates and phosphates; and organic acid salts such as acetate, maleate, fumarate, tartrates, citrates, lactates, aspartates, and glutamates. The metal salts include, for example, alkali metal salts such as sodium salts and potassium salts; alkaline earth metal salts such as magnesium salts and calcium salts; as well as aluminum salts and zinc salts. The ammonium salts include, for example, salts of ammonium and tetramethylammonium. The organic amine addition salts include, for example, morpholine salts and piperidine salts. The amino acid addition salts include, for example, lysine salts, glycine salts and phenylalanine salts.

Production methods of Compound (II) will be described below.

Me, Et, Pr, ⁱPr, ^{t}Bu, Ph, Ac, Bz and Boc in the following reaction processes, structural formulae and tables represent methyl, ethyl, propyl, isopropyl, *tert*-butyl, phenyl, acetyl, benzoyl and *tert*-butoxycarbonyl, respectively. The definitions of each groups in the following reaction processes have the same meanings as each groups defined above, unless otherwise noted.

When a defined group changes under the reaction conditions or is not suitable for carrying out the method in the following production methods, it is possible to obtain the targeted compound using a method for introduction and elimination of protective group commonly used in synthetic organic chemistry [for example, Protective Groups in Organic Synthesis, third edition, T. W. Greene, John Wiley & Sons Inc. (1999)]. If necessary, the order of reaction processes such as introduction of substituents can be changed.

Compound (II) can be produced according to the following reaction processes.

### Production Method 1

Compound (II) can be produced using Compound (A) obtained in a similar manner to the known method [e.g., J. Org. Chem., vol. 52, p. 19 (1987); Can. J. Chem., vol. 51, p. 792 (1973)] according to the following process: 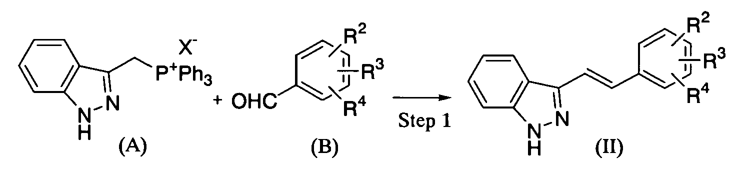 (wherein X represents each atoms of chlorine, bromine or iodine and R², R³ and R⁴ have the same meanings as defined above, respectively)

### Step 1

Compound (II) can be obtained by reacting Compound (A) with Compound (B) in the presence of a base, in a solvent such as methanol, ethanol, tetrahydrofuran (THF), *N,N-*dimethylformamide (DMF), and mixtures of these solvents.

Potassium carbonate, potassium *tert*-butoxide, sodium hydride and the like may be used as the base. To Compound (A), 1 to 10 equivalents of Compound (B) and the base are used, respectively. The reaction is usually performed at temperatures between 0 and 100°C for 1 to 72 hours.

### Production Method 2

Among Compound (II), Compound (IIa) which have a specific functional group in R², R³, and R⁴ may also be produced using Compound (C) which have other functional group in R², R³ and R⁴ obtained in Production Method 1 or other known method (for example, Japanese Published Unexamined Patent Application (kokai) No. 32059/1990) according to the following process.

Although all the compounds mentioned as Compound (IIa) and the like in the following Steps 2-1 to 2-3, are not always included in the scope of Compound (II), they are indicated as, for example, Compound (IIa) for the sake of convenience. Further, even among compounds referred to as Compound (C) in the following Steps 2-1 to 2-3, some compounds are included in Compound (II). 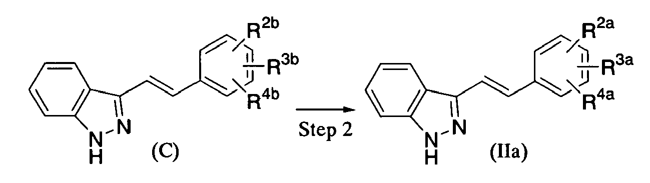 (wherein R^{2a}, R^{3a}, R^{4a}, R^{2b}, R^{3b} and R^{4b} represent the groups defined in following Steps 2-1 to 2-3, respectively. R^{2a}, R^{3a}, R^{4a}, R^{2b}, R^{3b} and R^{4b} have the same meaning as R², R³ and R⁴ defined above, respectively, unless otherwise defined in following Steps 2-1 to 2-3)

### Step 2-1

(In Step 2-1, at least one of R^{2a}, R^{3a} and R^{4a} represents carboxy and at least one of R^{2b}, R^{3b} and R^{4b} represents lower alkoxycarbonyl)

Compound (IIa) can be obtained by subjecting Compound (C) to hydrolysis in the presence of a base such as sodium hydroxide or an acid such as hydrochloric acid, in water or in a mixed solvent of water and another solvent such as methanol, ethanol or THF.

To Compound (C), 0.1 to 10 equivalent(s) of the acid or the base is preferably used. The reaction is usually performed at temperatures between 20 and 100°C for 1 to 24 hour(s).

### Step 2-2

(In Step 2-2, at least one of R^{2a}, R^{3a} and R^{4a} represents amino and at least one of R^{2b}, R^{3b} and R^{4b} represents nitro)

Compound (IIa) can be obtained by treating Compound (C) with a reducing agent such as tin or iron in the presence of an acid such as concentrated hydrochloric acid or acetic acid in a solvent such as water, ethanol or a mixed solvent thereof, or in the absence of the solvent; or by subjecting Compound (C) to reduction in the presence of a catalyst such as palladium/carbon, platinum dioxide or Raney nickel in an atmosphere of hydrogen gas or in the presence of a hydrogen donor such as hydrazine hydrate or ammonium formate in a solvent such as water, methanol, ethanol, THF, DMF, or a mixed solvent thereof.

To Compound (C), 1 to 20 equivalent(s) of the reducing agent such as tin or iron, 0.5 to 100 weight % of the catalyst and 1 to 100 equivalent(s) of the hydrogen donor are preferably used. The reaction is usually performed at temperatures between 0 to 100°C for 1 to 72 hours.

### Step 2-3

[In the Step 2-3, at least one of R^{2a}, R^{3a} and R^{4a} represents substituent including NHCOR²⁴ (wherein R²⁴ represents substituted or unsubstitued lower alkyl, substituted or unsubstituted aryl or heteroaryl) and at least one of R^{2b}, R^{3b} and R^{4b} represents amino]

In the definition of R²⁴, the lower alkyl and the aryl have the same meanings as (ii) and (vi) defined above, respectively, and the heteroaryl has the same meaning as heteroaromatic group (viii-a) defined above. The substituents in the substituted lower alkyl have the same meanings as (xi), and the substituents in the substituted aryl have the same meanings as (xii).

Compound (IIa) can be obtained by reacting Compound (C) with Compound (VIII) represented by R²⁴COCl (wherein R²⁴ has the same meaning as defined above) or Compound (IX) represented by (R²⁴CO)₂O (wherein R²⁴ has the same meaning as defined above) in the presence of a base such as triethylamine, pyridine, 4-dimethylaminopyridine, polyvinylpyridine, 4-morpholinomethylpolystyrene or 4-piperidinopolystyrene, in a solvent such as dichloromethane, THF, 1,4-dioxane, DMF, N-methylpiperidone or a mixed solvent thereof; or with Compound (X) represented by R²⁴CO₂H (wherein R²⁴ has the same meaning as defined above) in the presence of a condensing agent such as dicyclohexylcarbodiimide, 1-ethyl-3-(3-dimethylaminopropyl)carbodiimide hydrochloride or polymer bound-1-ethyl-3-(3-dimethylaminopropyl)carbodiimide, and a activating agent such as 1-hydroxybenztriazole or N-hydroxysuccinimide, in a solvent such as dichloromethane, THF, 1,4-dioxane, DMF, N-methylpiperidone or a mixed solvent thereof.

To Compound (C), 1 to 20 equivalent(s) of the base, Compound (VIII) or Compound (IX), the condensing agent, the activating agent and Compound (X) are preferably used, respectively. The reaction is usually performed at temperatures between -20 and 80°C for 30 minutes to 24 hours.

Transformation of functional groups in Compound (II) and the starting material can also be carried out by other known methods [for example, Comprehensive Organic Transformations, R. C. Larock, (1989)] in addition to the above processes.

Compound (II) having a desired functional group at a desired position can be obtained by carrying out the above processes in any suitable combination thereof.

Compound (I) an be synthesized in a similar manner to the Production methods of Compound (II) described above or a known method; or can be obtained by purchasing commercially available products. Compound (III) can be obtained in a similar manner to the Production methods of Compound (II) described above.

Isolation and purification of the Products in the above-mentioned production methods can be carried out by an appropriate combination of usual methods used in organic syntheses, such as filtration, extraction, washing, drying, concentration, crystallization and various chromatography. Intermediates can also be used in the subsequent reaction step without further purification.

There can be isomers such as positional isomers, geometrical isomers or optical isomers in Compound (I). All possible isomers including these isomers, and mixtures of the isomers in any ratio can be used as protein kinase inhibitor (excluding c-jun N-terminal kinase inhibitor) of the present invention.

There can be isomers such as positional isomers, geometrical isomers or optical isomers in Compound (II) or (III). All possible isomers including these isomers, and mixtures of the isomers in any ratio are comprised in the present invention.

When it is desired to obtain salts of Compound (I), (II) and (III), the salt may be purified as it is, if Compound (I), (II) or (III) are obtained in a form of a salt; and if Compound (I), (II) or (III) is obtained in a free form, it is dissolved or suspended in an appropriate solvent followed by adding an acid or a base thereto to form a salt.

Compound (I), (II) and (III), or a pharmaceutically acceptable salt thereof may exist in the form of adducts with water or solvents. These adducts are also included in the present invention.

As examples of Compound (I), Compound 2, Compound 60 to Compound 78 are listed; as examples of Compound (II), Compound 1, Compound 3 to Compound 20, Compound 28 to Compound 59 are listed; as examples of Compound (III), Compound 21 to Compound 27 are listed (Table 1 to 4).

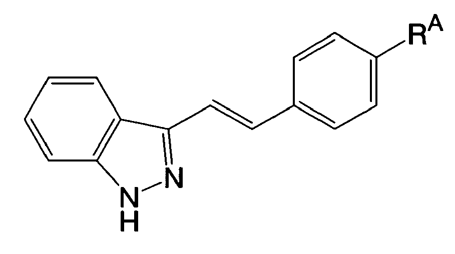

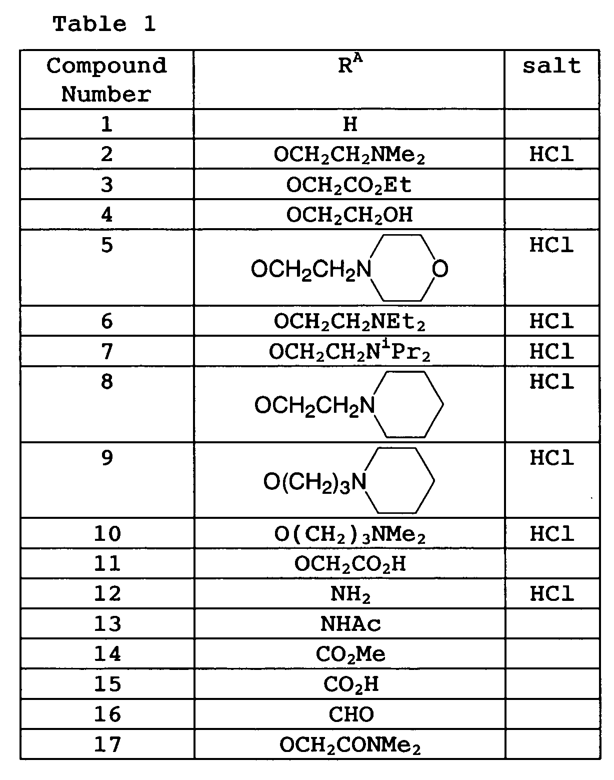

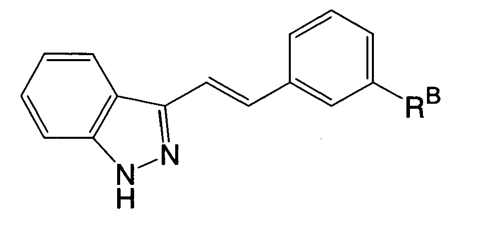

**Table 2**

| Compound Number | R^{B} | salt |
|---|---|---|
| 18 | OCH₂CH₂NMe₂ | HCl |
| 19 | OCH₂CO₂H | |
| 20 | OCH₂CH₂OH | |

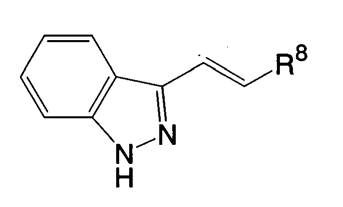

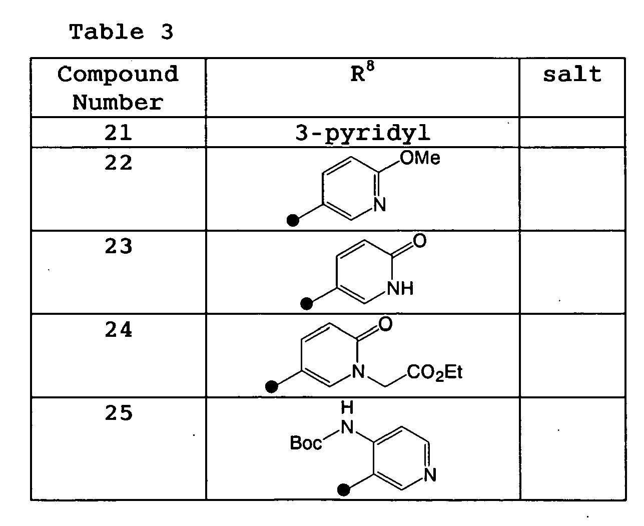

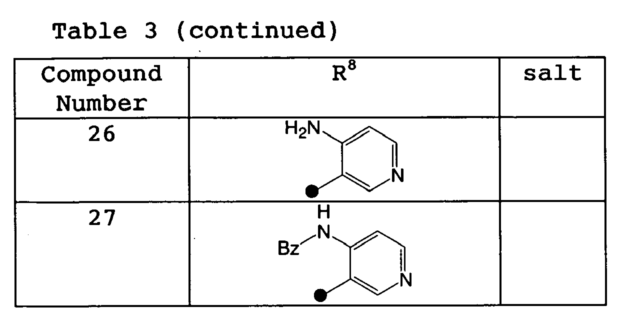

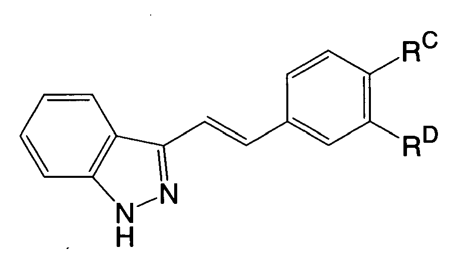

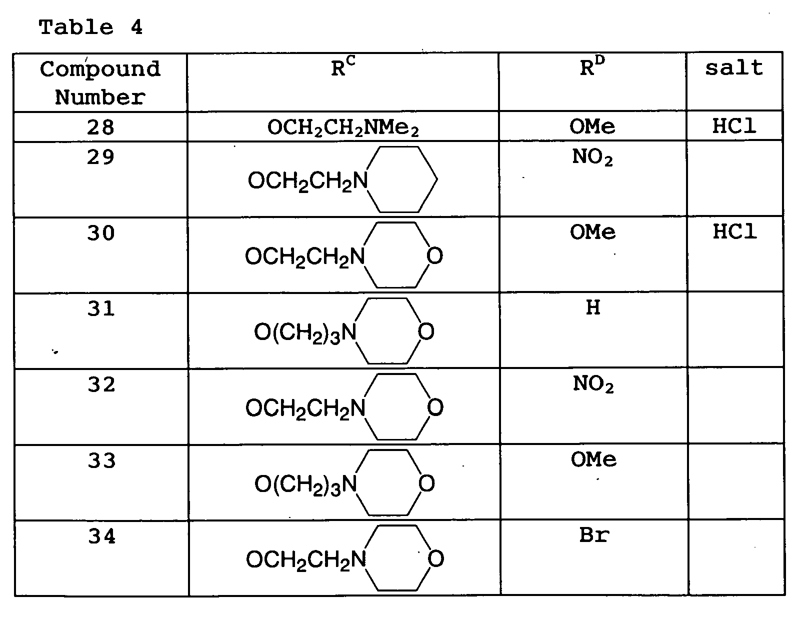

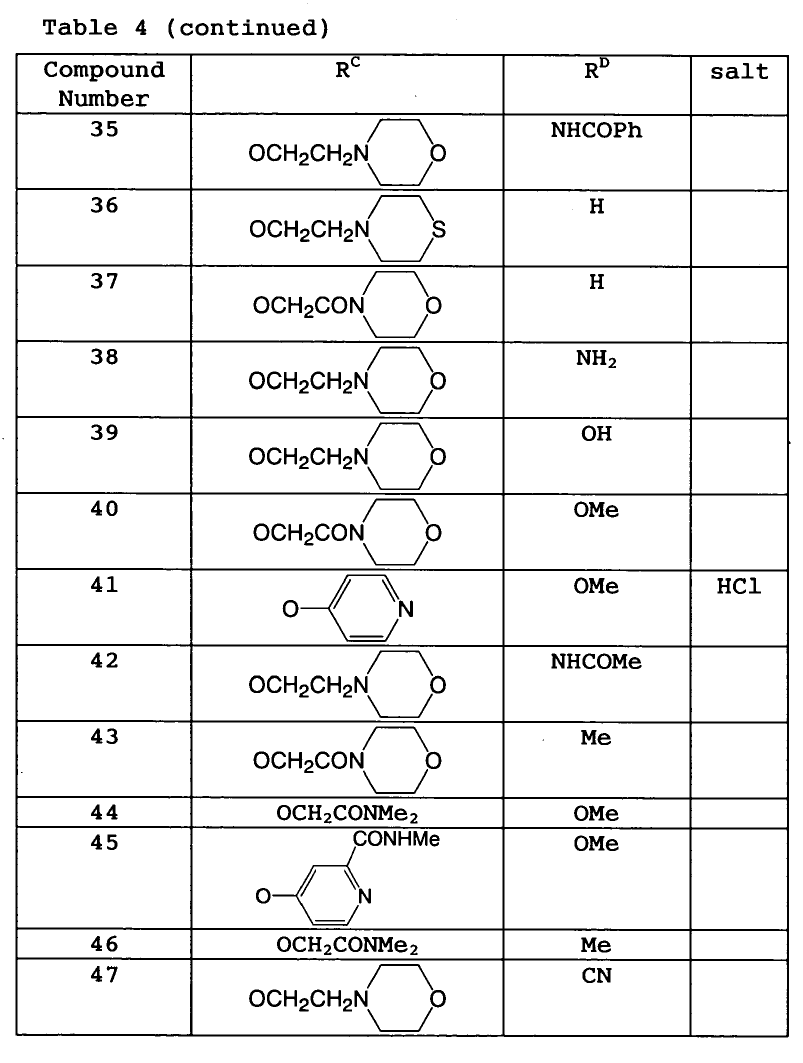

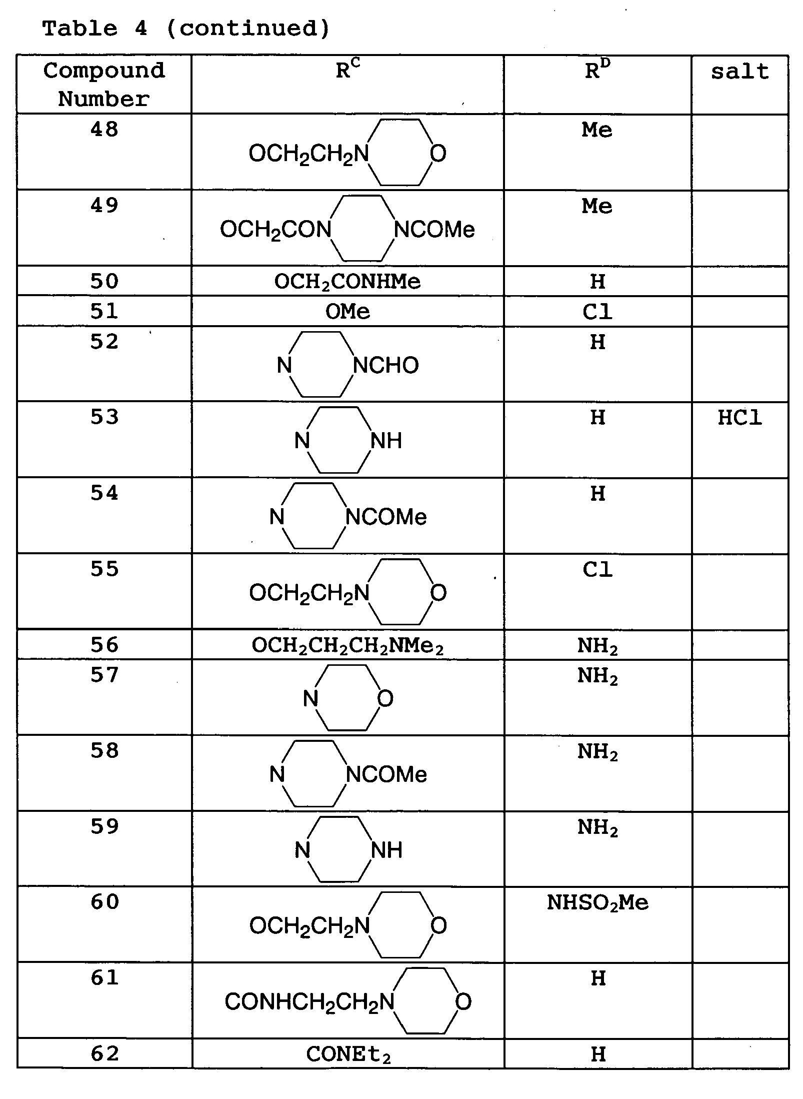

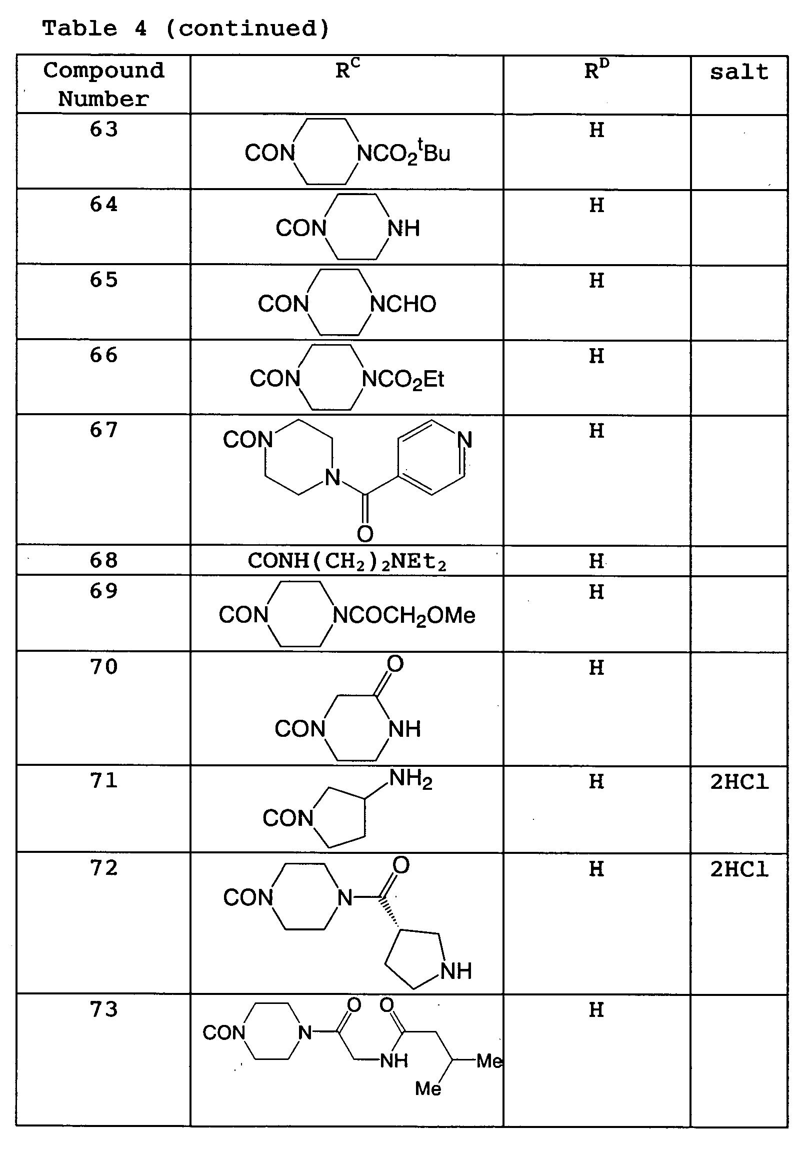

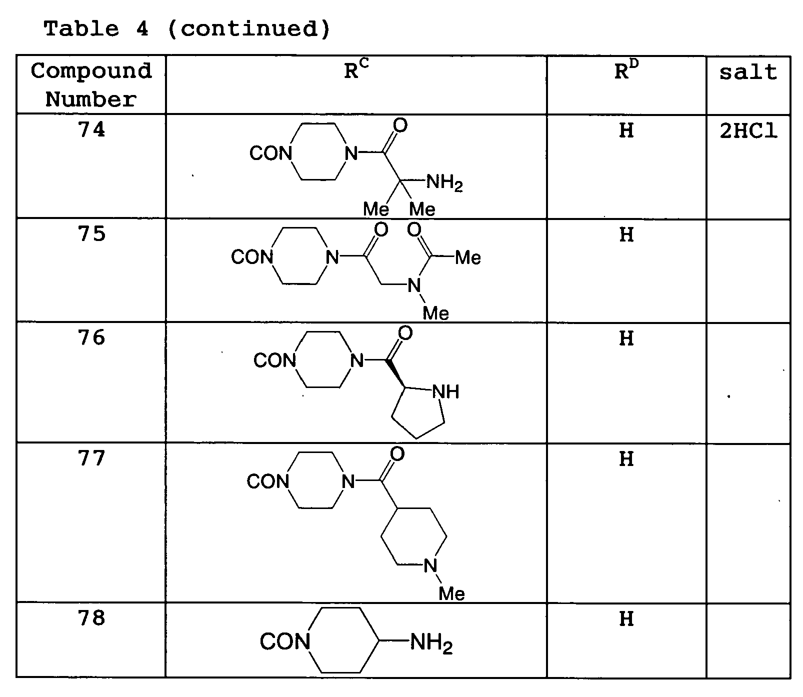

Next, pharmacological activities of Compound (I), (Ia), (II) and (III) will be illustrated below with reference to the test examples.

### Test Example 1: Cytostatic activity on leukemia cell line and solid carcinoma cell line in which activation of kinase by Flt-3 mutation or other mechanism is shown

The cytostatic rates of a test compound on human acute myeloid leukemia cell lines MV-4-11 and ML-1, and human chronic myeloid leukemia cell line K562 were determined in the following manner.

Each cell was cultured using Roswell Park Memorial Institute's Medium (RPMI) 1640 (Gibco, Catalog No. 11875-093) containing 10% fetal bovine serum (Gibco, Catalogue No. 10437-028) and penicillin/streptomycin (1:1) (Gibco, Catalogue No. 15140-122). Each 80 µL of the MV-4-11 cell having a concentration of 7.5×10⁴ cells/mL (or the ML-1 cell or K562 cell having a concentration of 2.5×10⁴ cells/mL) was inoculated to wells of a TC MICROWELL 96U plate (Nalge Nunc International, Catalogue No. 163320) and was cultured in a 5% carbon dioxide gas incubator at 37°C for 4 hours. As a blank, only RPMI medium (80 µL) was added to a well. Each 20 µL of a solution of the test compound in dimethyl sulfoxide (DMSO) which was prepared to make the final concentration to 3.16 µmol/L or 10 µmol/L, was added to the MV-4-11 cell, ML-1 cell and K562 cell, respectively. Each 20 µL of DMSO was added to the control well and the blank well to a final concentration of 0.1%. After adding the test compound, the cells were incubated in a 5% carbon dioxide gas incubator at 37°C for 72 hours. After adding 20 µL of WST-1 reagent {4-[3-(4-iodophenyl)-2-(4-nitrophenyl)-2*H*-5-tetrazolio]-1,3-benzene disulfonate sodium salt} (Roche Diagnostics K.K., Catalogue No. 1644807) diluted to 50% with RPMI medium, the cells were further incubated at 37°C for 2 hours. Then, the absorbances at 450 nm (reference wavelength: 690 nm) were determined with a microplate spectrophotometer SPECTRA max 340PC (Molecular Devices Corporation). The relative growth (%) of a well to which the test compound had been added was determined while setting the absorbance of a well to which not the test compound but DMSO alone had been added (control) at 100% and that of a well containing RPMI medium alone at 0%. The cytostatic rate (%) of the test compound was determined by subtracting the calculated relative growth from 100. The higher the cytostatic rate, the stronger the test compound exhibits cytostatic activity on the cell.

The cytostatic rate of a test compound on the human colon cancer cell line Colo205 was determined in the following manner.

The cell was cultured using Roswell Park Memorial Institute's Medium (RPMI) 1640 (Gibco, Catalog No. 11875-093) containing 10% fetal bovine serum (Gibco, Catalog No. 10437-028) and penicillin/streptomycin (1:1) (Gibco, Catalog No. 15140-122). Each 80 µL of the Colo205 having a concentration of 1.25×10⁴ cells/mL was inoculated to wells of a TC MICROWELL 96F plate (Nalge Nunc International, Catalog No. 167008) and was cultured in a 5% carbon dioxide gas incubator at 37°C for 4 hours. As a blank, only RPMI (80 µL) was added to a well. Each 20 µL of a solution of the test compound in DMSO which was prepared to make the final concentration to 10 µmol/L, was added to the Colo205 cell. Each 20 µL of DMSO was added to the control well and the blank well to a final concentration of 0.1%. After adding the test compound, the cell was incubated in a 5% carbon dioxide gas incubator at 37°C for 72 hours. The cytostatic rate (%) was then determined in the same manner as the method for the leukemia cell lines described above.

The results are shown in Table 5. Table 5 shows that Compound of the present invention exhibit effective cytostatic activities on leukemia cell line and solid carcinoma cell line in which activation of kinase by Flt-3 mutation or other mechanism is shown.

**Table 5**

| Compound | MV-4-11 (%) 3.16 µmol/L | ML-1 (%) 3.16 µmol/L | K562 (%) 10 µmol/L | Colo205(%) 10 µmol/L |
|---|---|---|---|---|
| 3 | 37 | - | 14 | - |
| 4 | 91 | 27 | 72 | - |
| 5 | 91 | 16 | 81 | 90 |
| 6 | 97 | 89 | 98 | - |
| 7 | 97 | 89 | 93 | - |
| 8 | 97 | 90 | 94 | - |
| 9 | 97 | 92 | 97 | - |
| 10 | 97 | 92 | 95 | - |
| 18 | 95 | 4.1 | 59 | - |
| 21 | 79 | 25 | 41 | - |
| 22 | 82 | 9.2 | 63 | - |
| 40 | 95 | 95 | 60 | 93 |
| 47 | 98 | 89 | 75 | 68 |
| 53 | 97 | 80 | 87 | 90 |
| 54 | 98 | 89 | 59 | 78 |
| 58 | 97 | 90 | 61 | 91 |
| 59 | 98 | 86 | 84 | 89 |

### Test Example 2: Inhibitory Activity on Flt-3

Flt-3 inhibitory activities were determined in the following manner.

Flt-3 was prepared by allowing baculovirus to infect insect cells, which baculovirus expresses a protein comprising human Flt-3 with glutathione S-transferase (GST) fused to the N-terminus of the intracellular domain (583-953 amino acids) thereof. Biotinylated polyglutamic acid-tyrosine peptide (Nihon Schering K.K., Catalogue No. 61GT0BAA) as a substrate was immobilized to a 96-well plate (FIA-PLATE BLACK 96 well FALT-BOTTOM HIGH BINDING, Greiner Bio-one Co., Ltd., Catalogue No. 655077) coated with Neutroavidin (Pierce Chemical Company, Catalogue No. 31000) and was then blocked with 0.25% gelatin to yield a plate for the determination of kinase reaction. Separately was prepared a solution containing, at final concentrations, GST-fused Flt-3 (100 µg/L), Tris-Cl (pH 7.5) (20 mmol/L), β-glycerophosphate (5 mmol/L), dithiothreitol (DTT) (1 mmol/L), Na₃VO₄ (0.1 mmol/L), MgCl₂ (10 mmol/L), MnCl₂ (10 mmol/L), ATP (10 µmol/L), bovine serum albumin (BSA) (0.1%), DMSO (0.1%), and a tested compound (10 µmol/L). The solution (each 60 µL) was placed into the wells of the plate for the determination of kinase reaction, followed by enzymatic reaction at room temperature for sixty minutes. After reacting, the reaction was stopped by the addition of 50 µL of a 25 mmol/L aqueous solution of ethylenediaminetetraacetic acid. The plate was washed with TBS-T [10 mmol/L Tris-Cl (pH 7.5), 150 mmol/L NaCl, 0.05% Tween 20 (Bio-Rad Laboratories, Catalogue No. 170-6531)] four times, was reacted with europium-labeled anti-phosphotyrosine antibody, was further washed with TBS-T four times, and was subjected to time-resolved fluoroimmunoassay at an excitation wavelength of 340 nm and a measuring wavelength of 615 nm. The relative activity (%) of a well in which the enzyme and tested compound were added was determined by calculation with the value in a well to which the tested compound was not added being 100% and the value in a well to which the enzyme and the tested compound were not added being 0%. The Flt-3 inhibitory activity (%) of the tested compound was determined by subtracting the relative activity (%) from 100.

The results are shown in Table 6. Table 6 demonstrates that the compounds according to the present invention exhibit effective Flt-3 inhibitory activities.

**Table 6**

| Compound | Inhibitory activity on Flt-3 (%) |
|---|---|
| 2 | 97.8 |
| 5 | 93.3 |
| 30 | 98.1 |
| 38 | 99.2 |
| 40 | 93.8 |
| 47 | 94.5 |
| 53 | 95.2 |
| 54 | 94.0 |
| 60 | 90.2 |
| 61 | 91.0 |
| 62 | 93.7 |
| 63 | 97.4 |
| 64 | 94.2 |
| 65 | 94.4 |
| 66 | 95.7 |
| 67 | 96.4 |
| 68 | 94.4 |
| 69 | 95.8 |
| 70 | 93.7 |
| 71 | 95.2 |
| 72 | 95.0 |
| 73 | 95.5 |
| 74 | 97.0 |
| 75 | 95.1 |
| 76 | 93.9 |
| 77 | 96.3 |
| 78 | 95.7 |

Compound (I), (Ia), (II), (III) or a pharmaceutically acceptable salt thereof may be used as it is or in various pharmaceutical forms depending upon the pharmacological effect, purpose of administration, etc. A pharmaceutical composition of the present invention can be manufactured by uniform mixing of Compound (I), (Ia), (II), (III) or a pharmaceutically acceptable salt thereof in an amount which is effective as an active ingredient with pharmaceutically acceptable carriers. These carriers can have forms in a wide range according to desired dosage form for administration. It is preferred that the pharmaceutical composition is in a unit dosage form for oral administration or parental administration such as injection.

In the manufacture of tablets, excipient such as lactose and mannitol, disintegrator such as starch, lubricant such as magnesium stearate, binder such as polyvinyl alcohol and hydroxypropyl cellulose, and surfactant such as sucrose fatty acid esters and sorbitol fatty acid esters, etc. may be used in accordance with a conventional procedure. Tablets containing 1 to 200 mg of an active ingredient per tablet are preferred.

In the manufacture of injections, water, physiological saline, vegetable oil such as olive oil and peanut oil, solvent such as ethyl oleate and propylene glycol, dissolving agent such as sodium benzoate, sodium salicylate and urethane, isotonizing agent such as sodium chloride and glucose, preservative such as phenol, cresol, *p-*hydroxybenzoate and chlorobutanol, and antioxidant such as ascorbic acid and sodium pyrosulfite, etc. may be used by a conventional procedure.

Compound (I), (Ia), (II), (III) or a pharmaceutically acceptable salt thereof can be administered either orally or parentally by means of injection solution, etc. The effective dose and frequency of administration vary depending on the dosage form, age, body weight and symptom of a patient, etc. In general, Compound (I), (Ia), (II), (III) or a pharmaceutically acceptable salt thereof may preferably be administered in an amount of 0.01 to 100 mg/kg per day.

### Best Mode for Carrying Out the Invention

The present invention will be illustrated in further detail with examples below which by no means limit the scope of the present invention.

In proton nuclear magnetic resonance spectra (¹H-NMR) used in the examples, exchangeable hydrogen may not be clearly observed in some compounds under some measuring conditions. With regard to indication of multiplicity of signals, the commonly used notation is here used, although br means a broad signal as judged by visual inspection.

### Example 1: (E)-3-(2-phenylvinyl)-1H-indazole (Compound 1) Step 1

1*H*-indazole-3-carboxylic acid (45.2 g, 279 mmol) was suspended in THF (500 mL), and to the suspension were added 1-hydroxybenzotriazole dimethylammonium salt (55.7 g, 308 mmol) prepared in a similar manner to the known method [for example, Synthesis, p. 285 (1992)] and 1-ethyl-3-(3-dimethylaminopropyl)carbodiimide hydrochloride (58.9 g, 307 mmol), followed by stirring at room temperature overnight. The reaction mixture was added with water, was extracted with ethyl acetate, and the organic layer was sequentially washed with a saturated aqueous sodium bicarbonate solution and brine, was dried over anhydrous sodium sulfate, and the solvent was evaporated under reduced pressure. The residue was recrystallized from a mixture of acetone/water to obtain *N,N*-dimethyl-1*H*-indazole-3-carboxamide (37.7 g, 71%).
¹H-NMR (270 MHz, CDCl₃) δ 3.22 (s, 3H), 3.40 (s, 3H), 7.24 (ddd, *J* = 1.3, 6.9, 8.1 Hz, 1H), 7.40 (ddd, *J* = 1.0, 6.9, 8.2 Hz, 1H), 7.48 (dd, *J* = 1.3, 8.2 Hz, 1H), 8.15 (dd, *J* = 1.0, 8.1 Hz, 1H), 10.7 (brs, 1H).
TOF-MS (m/z); 190 [M+1]⁺

### Step 2

Lithium aluminum hydride (13.7 g, 36.1 mmol) was suspended in THF (500 mL), and the suspension was added with a solution of *N,N*-dimethyl-1*H*-indazole-3-carboxamide obtained in Step 1 (34.2 g, 181 mmol) in THF (250 mL), followed by stirring at room temperature under the flow of nitrogen gas for 30 minutes. The reaction mixture was mixed with sodium sulfate decahydrate, was stirred for further one hour and was filtrated through Celite. The solvent was evaporated under reduced pressure to obtain 3-dimethylaminomethyl-1*H*-indazole (24.2 g, 76%).
¹H-NMR (270 MHz, CDCl₃) δ 2.33 (s, 6H), 3.85 (s, 2H), 7.16 (ddd, J = 1.3, 6.6, 7.9 Hz, 1H), 7.38 (ddd, J = 1.0, 6.6, 8.3 Hz, 1H), 7.45 (d, *J* = 8.3 Hz, 1H), 7.87 (dd, *J* = 1.0, 7.9 Hz, 1H), 10.0 (brs, 1H).

### Step 3

3-Dimethylaminomethyl-1*H*-indazole (25.6 g, 146 mmol) was dissolved in ethyl acetate (350 mL), and the solution was added with methyl iodide (33.3 mL, 535 mmol), followed by stirring at room temperature overnight. The resulting precipitates were collected by filtration to obtain (1*H-*indazol-3-ylmethyl)trimethylammonium iodide (45.0 g, 97%).
¹H-NMR (270 MHz, DMSO-*d*₆) δ 3.12 (s, 9H), 4.91 (s, 2H), 7.27 (dd, *J* = 7.3, 8.1 Hz, 1H), 7.45 (dd, *J* = 7.3, 8.1 Hz, 1H), 7.64 (d, *J* = 8.1 Hz, 1H), 8.02 (d, *J* = 8.1 Hz, 1H), 13.7 (brs, 1H).
TOF-MS (m/z); 190 [M-127]⁺

### Step 4

(1*H*-indazol-3-ylmethyl)trimethylammonium iodide (45.0 g, 142 mmol) was dissolved in DMF (220 mL), and the solution was mixed with triphenylphosphine (44.7 g, 170 mmol), followed by heating under reflux for 2.5 hours. After cooling to room temperature, the reaction mixture was added with diethyl ether and was stirred overnight. The resulting precipitates were collected by filtration to obtain (1*H-*indazol-3-ylmethyl)triphenylphosphonium iodide (68.3 g, 92%).
¹H-NMR (270 MHz, DMSO-*d*₆) δ 5.58 (d, *J* = 15.2 Hz, 2H), 6.98 (ddd, *J* = 1.0, 7.3, 8.3 Hz, 1H), 7.29 (ddd, *J* = 1.0, 7.3, 8.3 Hz, 1H), 7.43 (d, *J* = 8.3 Hz, 1H), 7.57 (d, *J* = 8.3 Hz, 1H), 7.64-7.85 (m, 15H), 13.1 (brs, 1H).
TOF-MS (m/z); 393 [M-127]⁺

### Step 5

(1*H*-indazol-3-ylmethyl)triphenylphosphonium iodide (0.15 g, 0.29 mmol) was dissolved in methanol (5 mL), and the solution was added with benzaldehyde (0.03 mL, 0.29 mmol) and potassium carbonate (0.12 g, 0.86 mmol), followed by stirring at room temperature overnight. The reaction mixture was added with water, and was extracted with ethyl acetate, then the organic layer was washed with brine, and was dried over anhydrous sodium sulfate, and the solvent was evaporated under reduced pressure. The residue was purified by thin-layer chromatography (chloroform/methanol=15/1), was further triturated in a mixture of ethyl acetate and diisopropyl ether to obtain Compound 1 (0.03 g, 54%).
¹H-NMR (270 MHz, DMSO-*d*₆) δ 7.20 (t, *J* = 7.6 Hz, 1H), 7.28 (t, *J* = 7.3 Hz, 1H), 7.36-7.39 (m, 3H), 7.42-7.55 (m, 3H), 7.71 (d, *J* = 7.9 Hz, 2H), 8.18 (d, *J* = 7.6 Hz, 1H), 13.1 (brs, 1H).
TOF-MS (m/z); 221 [M+1]⁺

### Example 2: (E)-3-{2-[4-(2-dimethylaminoethoxy)phenyl]vinyl}-1H-indazole hydrochloride (Compound 2)

### Step 1

In a similar manner to Step 5 of Example 1, a free base of Compound 2 (0.20 g) was obtained using (1*H*-indazol-3-ylmethyl)triphenylphosphonium iodide (1.00 g, 1.92 mmol), *p-*(2-dimethylaminoethoxy)benzaldehyde hydrochloride (0.45 g, 1.93 mmol) and potassium carbonate (1.33 g, 9.61 mmol).

### Step 2

A free base of Compound 2 (0.20 g) was dissolved in mixed solvent of ethyl acetate/chloroform (20 mL), and the solution was added with a 4 mol/L hydrogen chloride-ethyl acetate solution (0.28 mL, 1.10 mmol), followed by stirring at room temperature for 30 minutes. The resulting precipitate was collected by filtration to obtain Compound 2 (0.15 g, 2 steps 23%).
¹H-NMR (270 MHz, DMSO-*d*₆) δ 2.86 (s, 6H), 3.52 (brs, 2H), 4.39 (t, *J* = 5.0 Hz, 2H), 7.05 (d, *J* = 8.9 Hz, 2H), 7.19 (dd, *J* = 7.3, 8.1 Hz, 1H), 7.39 (dd, *J* = 7.3, 8.1 Hz, 1H), 7.43 (d, *J* = 15.7 Hz, 1H), 7.49 (d, *J* = 15.7 Hz, 1H), 7.54 (d, *J* = 8.1 Hz, 1H), 7.70 (d, *J* = 8.9 Hz, 2H), 8.17 (d, *J* = 8.1 Hz, 1H), 10.3 (brs, 1H), 13.1 (brs, 1H).
TOF-MS (m/z); 308 [M+1]⁺

### Example 3: ethyl (E)-4-[2-(1H-indaz-ol-3-yl)vinyl]phenoxyacetate (Compound 3)

In a similar manner to Step 5 of Example 1, Compound 3 (0.31 g, 59%) was obtained using (1*H*-indazol-3-ylmethyl)triphenylphosphonium iodide (1.08 g, 2.08 mmol), ethyl *p*-formylphenoxyacetate (0.34 g, 1.62 mmol) and potassium carbonate (0.87 g, 6.30 mmol).
¹H-NMR (270 MHz, DMSO-*d*₆) δ 1.31 (t, *J* = 7.3 Hz, 3H), 4.29 (q, *J* = 7.3 Hz, 2H), 4.66 (s, 2H), 6.94 (d, *J* = 8.7 Hz, 2H), 7.24 (m, 1H), 7.33 (d, *J* = 17.0 Hz, 1H), 7.41 (dd, *J* = 6.9, 8.3 Hz, 1H), 7.49 (d, *J* = 8.3 Hz, 1H), 7.487 (d, iJ = 17.1 Hz, 1H), 7.53 (d, *J* = 8.7 Hz, 2H), 8.02 (d, *J* = 8.3 Hz, 1H), 10.6 (brs, 1H).
TOF-MS (m/z); 323 [M+1]⁺

### Example 4: (E)-3-{2-[4-(2-hydroxyethoxy)phenyl]vinyl}-1H-indazole (Compound 4)

In a similar manner to Step 2 of Example 1, Compound 4 (0.12 g, 75%) was obtained using Compound 3 (0.18 g, 0.57 mmol) and lithium aluminum hydride (0.05 g, 1.20 mmol).
¹H-NMR (270 MHz, DMSO-*d*₆) δ 3.99 (t, *J* = 4.3 Hz, 2H), 4.14 (t, *J* = 4.3 Hz, 2H), 6.95 (d, *J* = 8.9 Hz, 2H), 7.24 (dd, *J* = 6.8, 8.3 Hz, 1H), 7.33 (d, *J* = 16.7 Hz, 1H), 7.42 (dd, *J* = 6.8, 8.3 Hz, 1H), 7.49 (d, *J* = 8.3 Hz, 1H), 7.50 (d, *J* = 16.7 Hz, 1H), 7.54 (d, *J* = 8.9 Hz, 2H), 8.04 (d, *J* = 8.3 Hz, 1H).
TOF-MS (m/z); 281 [M+1]⁺

### Example 5: (E)-3-{2-[4-(2-morpholinoethoxy)phenyl]vinyl}-1H-indazole hydrochloride (Compound 5)

In a similar manner to Step 5 of Example 1, a free base of Compound 5 was obtained using (1*H*-indazol-3-ylmethyl)triphenylphosphonium iodide (1.00 g, 1.92 mmol), p-(2-morpholinoethoxy)benzaldehyde hydrochloride (0.26 g, 0.96 mmol) and potassium carbonate (0.80 g, 5.79 mmol), and then the free base of Compound 5 was treated with 4 moL/L hydrogen chloride-ethyl acetate solution in a similar manner to Step 2 of Example 2, to obtain Compound 5 (0.14 g, 51%).
¹H-NMR (270 MHz, DMSO-d₆) δ 3.20 (m, 2H), 3.55 (m, 4H), 3.96 (m, 6H), 7.05 (d, *J* = 8.6 Hz, 2H), 7.19 (dd, *J* = 6.9, 8.3 Hz, 1H), 7.39 (dd, *J* = 6.9, 8.3 Hz, 1H), 7.43 (d, *J* = 16.2 Hz, 1H), 7.47 (d, *J* = 16.2 Hz, 1H), 7.54 (d, *J* = 8.3 Hz, 1H), 7.69 (d, J = 8.6 Hz, 2H), 8.16 (d, J = 8.3 Hz, 1H), 10.9 (brs, 1H), 13.1 (brs, 1H).
TOF-MS (m/z); 350 [M+1]⁺

### Example 6: (E)-3-{2-[4-(2-diethylaminoethoxy)phenyl]vinyl}-1H-indazole hydrochloride (Compound 6)

In a similar manner to Step 5 of Example 1, a free base of Compound 6 was obtained using (1*H*-indazol-3-ylmethyl)triphenylphosphonium iodide (1.00 g, 1.92 mmol), *p-*(2-diethylaminoethoxy)benzaldehyde hydrochloride (0.25 g, 0.97 mmol) and potassium carbonate (0.80 g, 5.79 mmol), and then the free base of Compound 6 was treated with 4 moL/L hydrogen chloride-ethyl acetate solution in a similar manner to Step 2 of Example 2, to obtain Compound 6 (0.11 g, 31%).
¹H-NMR (270 MHz, DMSO-*d*₆) δ 1.27 (t, *J* = 7.1 Hz, 6H), 3.22 (brm, 4H), 3.51 (brs, 2H), 4.42 (brm, 2H), 7.04 (d, *J* = 8.7 Hz, 2H), 7.19 (dd, *J* = 7.3, 7.6 Hz, 1H), 7.38 (dd, *J* = 7.3, 8.3 Hz, 1H), 7.43 (d, *J* = 16.5 Hz, 1H), 7.47 (d, *J* = 16.5 Hz, 1H), 7.54 (d, *J* = 8.3 Hz, 1H), 7.69 (d, *J* = 8.7 Hz, 2H), 8.16 (d, *J* = 7.6 Hz, 1H), 10.6 (brs, 1H).
TOF-MS (m/z); 336 [M+1]⁺

### Example 7: (E)-3-{2-[4-(2-diisopropylaminoethoxy)phenyl]vinyl}-1H-indazole hydrochloride (Compound 7)

In a similar manner to Step 5 of Example 1, a free base of Compound 7 was obtained using (1*H*-indazol-3-ylmethyl)triphenylphosphonium iodide (1.00 g, 1.92 mmol), p-(2-diisopropylaminoethoxy)benzaldehyde hydrochloride (0.27 g, 0.95 mmol) and potassium carbonate (0.80 g, 5.79 mmol), and then the free base of Compound 7 was treated with 4 moL/L hydrogen chloride-ethyl acetate solution in a similar manner to Step 2 of Example 2, to obtain Compound 7 (0.14 g, 36%).
¹H-NMR (270 MHz, DMSO-*d*₆) δ 1.33 (d, *J* = 6.6 Hz, 6H), 1.36 (d, *J* = 6.6 Hz, 6H), 3.55 (m, 4H), 4.36 (t, *J* = 4.8 Hz, 2H), 7.01 (d, *J* = 8.6 Hz, 2H), 7.19 (dd, *J* = 6.8, 8.1 Hz, 1H), 7.39 (dd, *J* = 6.8, 8.2 Hz, 1H), 7.42 (d, *J* = 16.8 Hz, 1H), 7.47 (d, *J* = 16.8 Hz, 1H), 7.54 (d, *J* = 8.2 Hz, 1H), 7.69 (d, *J* = 8.6 Hz, 2H), 8.17 (d, *J* = 8.1 Hz, 1H), 9.41 (brs, 1H).
TOF-MS (m/z); 364 [M+1]⁺

### Example 8: (E)-3-{2-[4-(2-piperidinoethoxy)phenyl]vinyl}-1H-indazole hydrochloride (Compound 8)

In a similar manner to Step 5 of Example 1, a free base of Compound 8 was obtained using (1*H*-indazol-3-ylmethyl)triphenylphosphonium iodide (1.00 g, 1.92 mmol), *p-*(2-piperidinoethoxy)benzaldehyde hydrochloride (0.26 g, 0.96 mmol) and potassium carbonate (0.80 g, 5.79 mmol), and then the free base of Compound 8 was treated with 4 moL/L hydrogen chloride-ethyl acetate solution in a similar manner to Step 2 of Example 2, to obtain Compound 8 (0.10 g, 27%).
¹H-NMR (270 MHz, DMSO-*d*₆) δ 1.80 (brm, 6H), 3.00 (brm, 2H), 3.49 (brm, 4H), 4.44 (m, 2H), 7.04 (d, *J* = 8.7 Hz, 2H), 7.19 (dd, *J* = 7.3, 7.9 Hz, 1H), 7.39 (dd, *J* = 7.3, 8.3 Hz, 1H), 7.43 (d, *J* = 16.5 Hz, 1H), 7.47 (d, *J* = 16.5 Hz, 1H), 7.54 (d, *J* = 8.3 Hz, 1H), 7.69 (d, *J* = 8.7 Hz, 2H), 8.17 (d, *J* = 7.9 Hz, 1H), 10.3 (brs, 1H), 13.1 (brs, 1H).
TOF-MS (m/z); 348 [M+1]⁺

### Example 9: (E)-3-{2-[4-(3-piperidinopropoxy)phenyl]vinyl}-1H-indazole hydrochloride (Compound 9)

In a similar manner to Step 5 of Example 1, a free base of Compound 9 was obtained using (1*H*-indazol-3-ylmethyl)triphenylphosphonium iodide (1.00 g, 1.92 mmol), *p-*(3-piperidinopropoxy)benzaldehyde hydrochloride (0.28 g, 0.98 mmol) and potassium carbonate (0.80 g, 5.79 mmol), and then the free base of Compound 9 was treated with 4 moL/L hydrogen chloride-ethyl acetate solution in a similar manner to Step 2 of Example 2, to obtain Compound 9 (0.11 g, 27%).
¹H-NMR (270 MHz, DMSO-*d*₆) δ 1.76 (brm, 6H), 2.18 (m, 2H), 2.89 (brm, 2H), 3.17 (brm, 4H), 4.10 (t, *J* = 5.9 Hz, 2H), 6.98 (d, *J* = 8.9 Hz, 2H), 7.19 (dd, *J* = 6.9, 8.1 Hz, 1H), 7.38 (m, 1H), 7.40 (d, *J* = 16.8 Hz, 1H), 7.46 (d, *J* = 16.8 Hz, 1H), 7.53 (d, *J* = 8.3 Hz, 1H), 7.66 (d, *J* = 8.9 Hz, 2H), 8.16 (d, *J* = 8.1 Hz, 1H), 9.87 (brs, 1H), 13.1 (brs, 1H).
TOF-MS (m/z); 362 [M+1]⁺

### Example 10: (E)-3-{2-[4-(3-dimethylaminopropoxy)phenyl]vinyl}-1H-indazole hydrochloride (Compound 10)

In a similar manner to Step 5 of Example 1, a free base of Compound 10 was obtained using (1*H*-indazol-3-ylmethyl)triphenylphosphonium iodide (1.00 g, 1.92 mmol), *p-*(3-dimethylaminopropoxy)benzaldehyde hydrochloride (0.24 g, 0.97 mmol) and potassium carbonate (0.80 g, 5.79 mmol), and then the free base of Compound 10 was treated with 4 moL/L hydrogen chloride-ethyl acetate solution in a similar manner to Step 2 of Example 2, to obtain Compound 10 (0.14 g, 31%).
¹H-NMR (270 MHz, DMSO-*d*₆) δ 2.07 (brm, 2H), 2.78 (s, 3H), 2.79 (s, 3H), 3.19 (brm, 2H), 4.09 (t, *J* = 5.9 Hz, 2H), 6.97 (d, *J* = 8.6 Hz, 2H), 7.15-7.54 (m, 5H), 7.65 (d, *J* = 8.6 Hz, 2H), 8.15 (d, *J* = 7.9 Hz, 1H), 10.9 (brs, 1H).
TOF-MS (m/z); 322 [M+1]⁺

### Example 11: (E)-4-[2-(1H-indazol-3-yl)vinyl]phenoxyacetic acid (Compound 11)

Compound 3 (0.14 g, 0.43 mmol) was dissolved in methanol (2.6 mL), and the solution was added with a 1 mol/L aqueous sodium hydroxide solution (0.86 mL, 0.86 mmol), followed by heating under reflux for 1.5 hours. Methanol was evaporated under reduced pressure, and the residue was added with 6 mol/L hydrochloric acid to adjust the pH of the residue to <3 under ice-cooling. The resulting precipitate was collected by filtration to obtain Compound 11 (0.12 g, 91%).
¹H-NMR (270 MHz, DMSO-*d*₆) δ 4.67 (s, 2H), 6.92 (d, *J* = 8.7 Hz, 2H), 7.17 (dd, *J* = 7.4, 7.8 Hz, 1H), 7.37 (dd, *J* = 7.4, 8.6 Hz, 1H), 7.39 (d, *J* = 16.7 Hz, 1H), 7.44 (d, *J* = 16.7 Hz, 1H), 7.52 (d, *J* = 8.6 Hz, 1H), 7.63 (d, *J* = 8.7 Hz, 2H), 8.15 (d, J = 7.8 Hz, 1H).
TOF-MS (m/z); 295 [M+1]⁺

### Example 12: (E)-3-[2-(4-aminophenyl)vinyl]-1H-indazole hydrochloride (Compound 12)

### Step 1

In a similar manner to Step 5 of Example 1, (*E*)-3-[2-(4-nitrophenyl)vinyl]-1*H*-indazole (1.12 g, 44%) was obtained using (1*H*-indazol-3-ylmethyl)triphenylphosphonium iodide (5.00 g, 9.61 mmol), *p*-nitrobenzaldehyde (1.45 g, 9.60 mmol) and potassium carbonate (3.98 g, 28.8 mmol).
¹H-NMR (270 MHz, DMSO-*d*₆) δ 7.23 (dd, *J* = 7.1, 7.8 Hz, 1H), 7.41 (dd, *J* = 7.1, 8.4 Hz, 1H), 7.57 (d, *J* = 8.4 Hz, 1H), 7.64 (d, *J* = 16.7 Hz, 1H), 7.82 (d, *J* = 16.7 Hz, 1H), 7.99 (d, *J* = 8.9 Hz, 2H), 8.23 (d, *J* = 8.9 Hz, 2H), 8.24 (d, *J* = 7.8 Hz, 1H).
TOF-MS (m/z); 266 [M+1]⁺

### Step 2

(*E*)-3-[2-(4-nitrophenyl)vinyl]-1*H*-indazole (0.83 g, 3.14 mmol) was added with concentrated hydrochloric acid (15 mL), ethanol (15 mL) and tin powder (0.75 g, 6.29 mmol), followed by heating under reflux for 3 hours. Ethanol was evaporated under reduced pressure, and the residue was alkalized with 10 mol/L aqueous sodium hydroxide solution, and was extracted with ethyl acetate. The organic layer was sequentially washed with water and saturated brine, and was dried over anhydrous sodium sulfate, and the solvent was evaporated under reduced pressure. In a similar manner to Step 2 of Example 2, the residue was treated with 4 mol/L aqueous hydrogen chloride-ethyl acetate solution to obtain Compound 12 (0.82 g, 96%).
¹H-NMR (270 MHz, DMSO-*d*₆) δ 7.20 (t, *J* = 7.6 Hz, 1H), 7.33 (d, *J* = 8.4 Hz, 2H), 7.39 (dd, *J* = 7.6, 8.3 Hz, 1H), 7.49 (d, *J* = 17.0 Hz, 1H), 7.55 (d, *J* = 8.3 Hz, 1H), 7.57 (d, *J* = 17.0 Hz, 1H), 7.80 (d, *J* = 8.4 Hz, 2H), 8.17 (d, *J* = 7.6 Hz, 1H).
TOF-MS (m/z); 236 [M+1]⁺

### Example 13: (E)-3-[2-(4-acetamidophenyl)vinyl]-1H-indazole (Compound 13)

Compound 12 (0.10 g, 0.37 mmol) was dissolved in pyridine (0.5 mL), and the solution was added with acetic anhydride (0.04 mL, 0.45 mmol), follwed by stirring at room temperature for 4 hours. The reaction mixture was added with ice, and was acidified with 1 mol/L hydrochloric acid, and was extracted with ethyl acetate. The organic layer was washed with saturated brine, and was dried over anhydrous sodium sulfate, and the solvent was evaporated under reduced pressure. The residue was purified by thin layer-chromatography (chloroform/methanol/aqueous ammonia=9/1/1), and then triturated with chloroform to obtain Compound 13 (0.08 g, 73%).
¹H-NMR (270 MHz, DMSO-*d*₆) δ 2.04 (s, 3H), 7.18 (dd, *J* = 6.9, 8.3 Hz, 1H), 7.37 (dd, *J* = 6.9, 8.3 Hz, 1H), 7.42 (s, 2H), 7.52 (d, *J* = 8.3 Hz, 1H), 7.53 (d, *J* = 9.1 Hz, 2H), 7.57 (d, *J* = 9.1 Hz, 2H), 8.13 (d, *J* = 8.3 Hz, 1H), 10.0 (s, 1H), 13.1 (brs, 1H).
TOF-MS (m/z); 278 [M+1]⁺

### Example 14: methyl (E)-4-[2-(1H-indazol-3-yl)vinyl]benzoate (Compound 14)

In a similar manner to Step 5 of Example 1, Compound 14 (0.87 g, 51%) was obtained using (1*H*-indazol-3-ylmethyl)triphenylphosphonium iodide (3.20 g, 6.15 mmol), methyl *p*-formylbenzoate (1.00 g, 6.09 mmol) and potassium carbonate (2.55 g, 18.5 mmol).
¹H-NMR (270 MHz, DMSO-*d*₆) δ 3.84 (s, 3H), 7.21 (dd, *J* = 7.1, 8.3 Hz, 1H), 7.40 (dd, *J* = 7.1, 8.3 Hz, 1H), 7.55 (d, *J* = 8.3 Hz, 1H), 7.55 (d, *J* = 16.5 Hz, 1H), 7.70 (d, *J* = 16.5 Hz, 1H), 7.84 (d, *J* = 8.1 Hz, 2H), 7.95 (d, *J* = 8.1 Hz, 2H), 8.19 (d, *J* = 8.3 Hz, 1H).
TOF-MS (m/z); 279 [M+1]⁺

### Example 15: (E)-4-[2-(1H-indazol-3-yl)vinyl]benzoic acid (Compound 15)

In a similar manner to Example 11, Compound 15 (0.70 g, quantitative yield) was obtained by treating Compound 14 (0.71 g, 2.54 mmol) with 1 mol/L aqueous sodium hydroxide solution (5.00 mL).
¹H-NMR (270 MHz, DMSO-*d*₆) δ 7.21 (dd, *J* = 7.1, 7.9 Hz, 1H), 7.40 (dd, *J* = 7.1, 8.6 Hz, 1H), 7.55 (d, *J* = 8.6 Hz, 1H), 7.57 (d, *J* = 16.5 Hz, 1H), 7.70 (d, *J* = 16.5 Hz, 1H), 7.83 (d, *J* = 8.3 Hz, 2H), 7.94 (d, *J* = 8.3 Hz, 2H), 8.21 (d, *J* = 7.9 Hz, 1H), 13.2 (brs, 1H).
TOF-MS (m/z); 265 [M+1]⁺

### Example 16: (E)-3-[2-(4-formylphenyl)vinyl]-1H-indazole (Compound 16)

In a similar manner to Step 5 of Example 1, (1*H-*indazol-3-ylmethyl)triphenylphosphonium iodide (2.50 g, 4.80 mmol) and terephthalaldehyde mono-(diethyl acetal) (1.00 g, 4.80 mmol) was reacted in the presence of potassium carbonate (2.00 g, 14.5 mmol), then the obtained product was treated with *p*-toluenesulfonic acid monohydrate (0.04 g, 0.20 mmol) in acetone, to obtain Compound 16 (0.08 g, 7%).
¹H-NMR (270 MHz, DMSO-*d*₆) δ 7.23 (dd, *J* = 7.6, 8.1 Hz, 1H), 7.40 (t, *J* = 7.6 Hz, 1H), 7.56 (d, *J* = 7.6 Hz, 1H), 7.60 (d, *J* = 16.3 Hz, 1H), 7.77 (d, *J* = 16.3 Hz, 1H), 7.92 (d, *J* = 8.6 Hz, 2H), 7.94 (d, *J* = 8.6 Hz, 2H), 8.23 (d, *J* = 8.1 Hz, 1H), 9.99 (s, 1H).
TOF-MS (m/z); 249 [M+1]⁺

### Example 17: (E)-N,N-dimethyl-4-[2-(1H-indazol-3-yl)vinyl]phenoxyacetamide (Compound 17)

In a similar manner to Step 1 of Example 1, Compound 17 (0.06 g, 66%) was obtained using Compound 11 (0.19 g, 0.30 mmol), 1-hydroxybenzotriazole dimethylammonium salt (0.08 g, 0.46 mmol) and 1-ethyl-3-(3-dimethylaminopropyl)carbodiimidehydrochloride (0.08 g, 0.43 mmol) .
¹H-NMR (270 MHz, DMSO-*d*₆) δ 2.85 (s, 3H), 3.00 (s, 3H), 4.83 (s, 2H), 6.93 (d, *J* = 8.6 Hz, 2H), 7.17 (dd, *J* = 7.6, 8.3 Hz, 1H), 7.37 (dd, *J* = 7.6, 8.3 Hz, 1H), 7.38 (d, *J* = 16.5 Hz, 1H), 7.44 (d, *J* = 16.5 Hz, 1H), 7.52 (d, *J* = 8.3 Hz, 1H), 7.61 (d, *J* = 8.6 Hz, 2H), 8.15 (d, *J* = 8.3 Hz, 1H), 13.1 (brs, 1H).
TOF-MS (m/z); 322 [M+1]⁺

### Example 18: (E)-3-{2-[3-(2-dimethylaminoethoxy)phenyl]vinyl}-1H-indazole hydrochloride (Compound 18)

In a similar manner to Step 5 of Example 1, a free base of Compound 18 was obtained using (1*H*-indazol-3-ylmethyl)triphenylphosphonium iodide (0.51 g, 0.97 mmol), *m-*(2-dimethylaminoethoxy)benzaldehyde (0.13 g, 0.67 mmol) and potassium carbonate (0.43 g, 3.09 mmol), and then the free base of Compound 18 was treated with 4 moL/L hydrogen chloride-ethyl acetate solution in a similar manner to Step 2 of Example 2, to obtain Compound 18 (0.19 g, 83%).
¹H-NMR (270 MHz, DMSO-*d*₆) δ 2.85 (s, 3H), 2.87 (s, 3H), 3.53 (m, 2H), 4.45 (t, *J* = 4.9 Hz, 2H), 6.93 (m, 1H), 7.21 (m, 1H), 7.34-7.43 (m, 4H), 7.51 (d, *J* = 16.8 Hz, 1H), 7.54 (m, 1H), 7.58 (d, *J* = 16.8 Hz, 1H), 8.19 (d, *J* = 8.3 Hz, 1H), 10.6 (brs, 1H).
TOF-MS (m/z); 308 [M+1]⁺

### Example 19: (E)-3-[2-(1H-indazol-3-yl)vinyl]phenoxyacetic acid (Compound 19)

In a similar manner to Example 11, Compound 19 (0.70 g, quantitative yield) was obtained by treating ethyl (E)-3-[2-(1*H*-indazol-3-yl)vinyl]phenoxyacetate (0.71 g, 2.54 mmol) obtained in a similar manner to Example 3 with a 1 mol/L aqueous sodium hydroxide solution (5.0 mL).
¹H-NMR (270 MHz, DMSO-d₆) δ 4.71 (s, 2H), 6.83 (m, 1H), 7.20 (dd, *J* = 7.1, 7.9 Hz, 1H), 7.30 (m, 3H), 7.38 (dd, *J* = 7.1, 8.3 Hz, 1H), 7.49-7.58 (m, 3H), 8.18 (d, *J* = 7.9 Hz, 1H).
TOF-MS (m/z); 295 [M+1]⁺

### Example 20: (E)-3-{2-[3-(2-hydroxyethoxy)phenyl]vinyl}-1H-indazole (Compound 20)

In a similar manner to Step 2 of Example 1, Compound 20 (0.09 g, 75%) was obtained using Compound 19 (0.13 g, 0.42 mmol) and lithium aluminum hydride (0.03 g, 0.82 mmol).
¹H-NMR (270 MHz, DMSO-*d*₆) δ 3.52 (m, 2H), 4.05 (t, *J* = 5.1 Hz, 2H), 4.88 (brm, 1H), 6.85 (m, 1H), 7.19 (dd, *J* = 7.1, 7.9 Hz, 1H), 7.27 (m, 3H), 7.38 (dd, *J* = 7.1, 8.3 Hz, 1H), 7.46 (d, *J* = 17.0 Hz, 1H), 7.54,(d, *J* = 8.3 Hz, 1H), 7.55 (d, *J* = 17.0 Hz, 1H), 8.18 (d, *J* = 7.9 Hz, 1H).
TOF-MS (m/z); 281 [M+1]⁺

### Example 21: (E)-3-[2-(3-pyridyl)vinyl]-1H-indazole (Compound 21)

In a similar manner to Step 5 of Example 1, Compound 21 (2.12 g, 50%) was obtained using (1*H*-indazol-3-ylmethyl)triphenylphosphonium iodide (10.0 g, 19.2 mmol), 3-pyridinecarboxaldehyde (1.80 mL, 19.0 mmol) and potassium carbonate (7.96 g, 57.6 mmol).
¹H-NMR (270 MHz, DMSO-*d*₆) δ 7.21 (dd, *J* = 7.3, 8.3 Hz, 1H), 7.40 (dd, *J* = 7.3, 8.6 Hz, 1H), 7.41 (m, 1H), 7.51 (d, *J* = 16.8 Hz, 1H), 7.55 (d, *J =* 8.6 Hz, 1H), 7.67 (d, *J* = 16.8 Hz, 1H), 8.16 (d, *J* = 10.2 Hz, 1H), 8.19 (d, *J* = 8.3 Hz, 1H), 8.45 (d, *J* = 4.6 Hz, 1H), 8.55 (s, 1H).
TOF-MS (m/z); 222 [M+1]⁺

### Example 22: (E)-3-[2-(6-methoxypyridin-3-yl)vinyl]-1H-indazole (Compound 22)

In a similar manner to Step 5 of Example 1, Compound 22 (0.03 g, 34%) was obtained using (1*H*-indazol-3-ylmethyl)triphenylphosphonium iodide (0.22 g, 0.42 mmol), 6-methoxy-3-pyridinecarboxaldehyde (0.06 g, 0.41 mmol) and potassium carbonate (0.18 g, 1.27 mmol).
¹H-NMR (270 MHz, DMSO-*d*₆) δ 3.87 (s, 3H), 6.85 (d, *J* = 8.7 Hz, 1H), 7.19 (dd, *J* = 7.1, 8.1 Hz, 1H), 7.38 (ddd, *J* = 1.0, 7.1, 8.4 Hz, 1H), 7.48 (m, 2H), 7.53 (d, *J* = 8.4 Hz, 1H), 8.14 (dd, *J* = 2.2, 8.7 Hz, 1H), 8.16 (d, *J* = 8.1 Hz, 1H), 8.42 (d, *J* = 2.2 Hz, 1H), 13.1 (brs, 1H).
TOF-MS (m/z); 252 [M+1]⁺

### Example 23: (E)-3-[2-(2-oxo-1,2-dihydropyridin-5-yl)vinyl]-1H-indazole (Compound 23)

Compound 22 (0.12 g, 0.46 mmol) was added with hydrobromic acid (1.6 mL), and then was heated under reflux for 1 hour. The reaction mixture was slowly poured into saturated aqueous sodium hydrogencarbonate solution, and the resulting precipitate was collected by filtration to obtain Compound 23 (0.11 g, 88%).
¹H-NMR (270 MHz, DMSO-*d*₆) δ 6.40 (d, *J* = 9.6 Hz, 1H), 7.15 (m, 1H), 7.21 (d, *J* = 16.5 Hz, 1H), 7.32 (d, *J* = 16.5 Hz, 1H), 7.36 (m, 1H), 7.50 (d, *J* = 8.6 Hz, 1H), 7.67 (d, *J* = 2.3 Hz, 1H), 8.01 (dd, *J* = 2.3, 9.6 Hz, 1H), 8.13 (d, *J* = 7.9 Hz, 1H), 11.7 (brs, 1H), 13.0 (brs, 1H).
TOF-MS (m/z); 238 [M+1]⁺

### Example 24: (E)-3-[2-(1-ethoxycarbonylmethyl-2-oxo-1,2-dihydropyridin-5-yl)vinyl]-1H-indazole (Compound 24)

In a similar manner to Step 5 of Example 1, Compound 24 (0.13 g, 36 %) was obtained using (1*H*-indazol-3 ylmethyl)triphenylphosphonium iodide (0.56 g, 1.08 mmol), 1-ethoxycarbonylmethyl-2-oxo-1,2-dihydropyridine-5-carboxaldehyde (0.23 g, 1.08 mmol) and potassium carbonate (0.45 g, 3.25 mmol).
¹H-NMR (270 MHz, DMSO-*d*₆) δ 1.21 (t, *J* = 7.1 Hz, 3H), 4.16 (q, *J* = 7.1 Hz, 2H), 4.70 (s, 2H), 6.51 (d, *J* = 9.6 Hz, 1H), 7.17 (t, *J* = 7.9 Hz, 1H), 7.28 (m, 2H), 7.37 (t, *J* = 7.9 Hz, 1H), 7.52 (d, *J* = 7.9 Hz, 1H), 7.99 (d, *J* = 2.6 Hz, 1H), 8.06 (dd, *J* = 2.6, 9.6 Hz, 1H), 8.10 (d, *J* =7.9 Hz, 1H), 13.1 (brs, 1H).

### Example 25: (E)-3-[2-(4-tert-butoxycarbonylaminopyridin-3-yl)vinyl]-1H-indazole (Compound 25)

In a similar manner to Step 5 of Example 1, Compound 25 (0.24 g, 80%) was obtained using (1*H*-indazol-3-ylmethyl)triphenylphosphonium iodide (0.46 g, 0.87 mmol), 4-tert-butoxycarbonylaminopyridine-3-carboxaldehyde (0.19 g, 0.87 mmol) and potassium carbonate (0.36 g, 2.62 mmol),
¹H-NMR (270 MHz, DMSO-*d*₆) δ 1.48 (s, 9H), 7.20 (dd, *J* = 7.3, 8.1 Hz, 1H), 7.40 (dd, *J* = 7.3, 8.6 Hz, 1H), 7.45 (d, *J* = 16.7 Hz, 1H), 7.55 (d, J = 8.6 Hz, 1H), 7.60 (d, J = 5.6 Hz, 1H), 7.66 (d, *J* = 16.7 Hz, 1H), 8.18 (d, *J* = 8.1 Hz, 1H), 8.34 (d, J = 5.6 Hz, 1H), 8.85 (s, 1H), 9.43 (brs, 1H), 13.2 (brs, 1H).
TOF-MS (m/z); 337 [M+1]⁺

### Example 26: (E)-3-[2-(4-aminopyridin-3-yl)vinyl]-1H-indazole (Compound 26)

Compound 25 (0.24 g, 0.70 mmol) was added with trifluoroacetic acid (2.38 mL, 30.9 mmol), and was stirred at 40°C for 2 hours. The residue was added with ice-water, and was alkalized with 10 mol/L aqueous sodium hydroxide solution, and the resulting precipitate was collected by filtration to obtain Compound 26 (0.16 g, 98%).
¹H-NMR (270 MHz, DMSO-*d*₆) δ 6.21 (brs, 2H), 6.56 (d, *J* = 5.6 Hz, 1H), 7.17 (dd, *J* = 7.3, 7.9 Hz, 1H), 7.34 (d, *J* = 16.7 Hz, 1H), 7.37 (m, 1H), 7.48 (d, *J* = 16.7 Hz, 1H), 7.52 (d, *J* = 8.3 Hz, 1H), 7.91 (d, *J* = 5.6 Hz, 1H), 8.21 (d, *J* = 7.9 Hz, 1H), 8.44 (s, 1H), 13.1 (brs, 1H).
TOF-MS (m/z); 237 [M+1]⁺

### Example 27: (E)-3-{2-[4-(benzoylamino)pyridin-3-yl]vinyl}-1H-indazole (Compound 27)

In a similar manner to Example 13, Compound 27 (0.01 g, 28%) was obtained using Compound 26 (0.03 g, 0.13 mmol), triethylamine (0.07 mL, 0.51 mmol) and benzoyl chloride (0.04 mL, 0.38 mmol).
¹H-NMR (270 MHz, DMSO-*d*₆) δ 7.18 (dd, *J* = 7.3, 8.3 Hz, 1H), 7.40 (t, *J* = 7.3 Hz, 1H), 7.56-7.68 (m, 4H), 7.66 (d, *J* = 16.3 Hz, 1H), 7.78 (d, *J* = 16.3 Hz, 1H), 8.04 (d, *J* = 6.9 Hz, 2H), 8.13 (d, *J* = 8.3 Hz, 1H), 8.26 (dd, *J* = 2.8, 6.3 Hz, 1H), 8.66 (d, *J* = 6.3 Hz, 1H), 9.26 (brs, 1H), 10.9 (brs, 1H).
TOF-MS (m/z); 341 [M+1]⁺

### Example 28: (E)-3-{2-[4-(2-dimethylaminoethoxy)-3-methoxyphenyl]vinyl}-1H-indazole hydrochloride (Compound 28)

In a similar manner to Step 5 of Example 1, a free base of Compound 28 was obtained using (1*H*-indazol-3-ylmethyl)triphenylphosphonium iodide (0.34 g, 0.64 mmol), 4-(2-dimethylaminoethoxy)-3-methoxybenzaldehyde hydrochloride (0.17 g, 0.64 mmol) and potassium carbonate (0.27 mg, 1.93 mmol), and then the free base of Compound 28 was treated with 4 moL/L hydrogen chloride-ethyl acetate solution in a similar manner to Step 2 of Example 2, to obtain Compound 28 (0.19 g, 78%).
¹H-NMR (270 MHz, DMSO-*d*₆) δ 2.87 (d, *J* = 4.6 Hz, 6H), 3.56 (m, 2H), 3.87 (s, 3H), 4.36 (t, *J* = 5.1 Hz, 2H), 7.06 (d, *J* = 8.3 Hz, 1H), 7.15-7.35 (m, 2H), 7.35-7.55 (m, 5H), 8.18 (d, *J* = 8.3 Hz, 1H), 10.5 (brs, 1H).

### Example 29: (E)-3-{2-[3-nitro-4-(2-piperidinoethoxy)phenyl]vinyl}-1H-indazole (Compound 29)

### Step 1

A DMF (20.0 mL) solution of 4-hydroxy-3-nitrobenzaldehyde (2.01 g, 12.0 mmol) was added with potassium carbonate (5.00 g, 36.8 mmol) and 2-piperidinoethyl chloride hydrochloride (2.88 g, 15.6 mmol), followed by stirring at 70°C for 4 hours. After cooling to room temperature, the reaction mixture was added with water (200 mL), and was extracted with ethyl acetate (100 mL X 4). The organic layer was dried over anhydrous sodium sulfate, and then was treated with 4 mol/L hydrogen chloride-ethyl acetate solution (7.0 mL, 28.0 mmol) in a similar manner to Step 2 of Example 2, to obtain 3-nitro-4-(2-piperidinoethoxy)benzaldehyde hydrochloride (1.51 g, 40%).
¹H-NMR (270 MHz, DMSO-*d*₆) δ 1.40 (m, 1H), 1.67-1.81 (m, 5H), 2.96-3.10 (m, 2H), 3.48-3.56 (m, 4H), 4.77 (t, *J* = 4.6 Hz, 2H), 7.62 (d, *J* = 8.9 Hz, 1H), 8.23 (dd, *J* = 2.0, 8.9 Hz, 1H), 8.48 (d, *J* = 2.0 Hz, 1H), 9.98 (s, 1H), 10.7 (brs, 1H). APCI-MS (m/z); 279 [M+H]⁺

### Step 2

A DMF (5.0 mL) solution of 3-nitro-4-(2-piperidinoethoxy)benzaldehyde hydrochloride (0.50 g, 1.59 mmol) obtained in Step 1 was added with (1*H*-indazol-3-ylmethyl)triphenylphosphonium iodide (0.83 g, 1.59 mmol) and potassium carbonate (0.66 g, 4.78 mmol), followed by stirring at room temperature for 2 hours and at 60°C for 11 hours. After cooling to room temperature, the reaction mixture was concentrated under reduced pressure, and the residue was added with mixed solvent of ethyl acetate/chloroform (4/1), and the resulting yellow solid was separated by filtration. The solid was added to water (40 mL), followed by stirring at 50°C for 0.5 hours. The suspension was cooled to room temperature, and was filtered to obtain Compound 29 (0.50 g, 81%).
¹H-NMR (270 MHz, DMSO-*d*₆) δ 1.36-1.48 (m, 6H), 2.43 (t, *J* = 5.1 Hz, 4H), 2.67 (t, *J* = 5.8 Hz, 2H), 4.29 (t, *J* = 5.8 Hz, 2H), 7.19 (dd, J = 7.3, 7.9 Hz, 1H), 7.35-7.58 (m, 4H), 7.59 (d, *J* = 16.8 Hz, 1H), 8.00 (dd, *J* = 2.0, 8.9 Hz, 1H), 8.20-8.23 (m, 2H).
APCI-MS (m/z); 393 [M+H]⁺

### Example 30: (E)-3-{2-[3-methoxy-4-(2-morpholinoethoxy)phenyl]vinyl}-1H-indazole hydrochloride (Compound 30)

### Step 1

In a similar manner to Step 1 of Example 29, 3-methoxy-4-(2-morpholinoethoxy)benzaldehyde hydrochloride (17.3 g, 87%) was obtained using vanillin (10.0 g, 65.7 mmol), potassium carbonate (27.2 g, 197 mmol) and 2-morpholinoethylchloride hydrochloride (14.7 g, 79.0 mmol).
¹H-NMR (270 MHz, DMSO-*d*₆) δ 3.16-3.32 (m, 2H), 3.36-3.58 (m, 4H), 3.74-4.00 (m, 4H), 3.85 (s, 3H), 4.53 (t, *J* = 4.9 Hz, 2H), 7.25 (d, *J* = 8.2 Hz, 1H), 7.44 (d, *J* = 1.8 Hz, 1H), 7.57 (dd, *J* = 1.8, 8.2 Hz, 1H), 9.86 (s, 1H).
ESI-MS (m/z); 266 [M+H]⁺

### Step 2

In a similar manner to Step 2 of Example 29, a free base of Compound 36 was obtained using 3-methoxy-4-(2-morpholinoethoxy)benzaldehyde (0.50 g, 1.66 mmol) obtained in Step 1, (1*H*-indazol-3-ylmethyl)triphenylphosphonium iodide (0.82 g, 1.58 mmol) and potassium carbonate (0.63 g, 4.56 mmol), and then the free base of Compound 36 was treated with 4 moL/L hydrogen chloride-ethyl acetate solution (4.0 mL, 16.0 mmol) in a similar manner to Step 2 of Example 2, to obtain Compound 30 (0.25 g, 37%).
¹H-NMR (270 MHz, DMSO-*d*₆) δ 3.15-3.30 (m, 2H), 3.53-3.57 (m, 4H), 3.80-4.00 (m, 4H), 3.87 (s, 3H), 4.40-4.46 (m, 2H), 7.07 (d, *J* = 7.4 Hz, 1H), 7.16-7.26 (m, 2H), 7.35-7.55 (m, 5H), 8.18 (d, *J* = 7.2 Hz, 1H).
APCI-MS (m/z); 380 [M+H]⁺

### Example 31: (E)-3-{2-[4-(3-morpholinopropoxy)phenyl]vinyl}-1H-indazole (Compound 31)

### Step 1

In a similar manner to Step 1 of Example 29, 4-(3-morpholinopropoxy)benzaldehyde (1.45 g, 70%) was obtained using 4-hydroxybenzaldehyde (1.20 g, 9.83 mmol), potassium carbonate (4.07 g, 29.4 mmol) and 3-morpholinopropyl chloride (1.36 g, 8.34 mmol).
¹H-NMR (300 MHz, CDCl₃) δ 1.98-2.03 (m, 2H), 2.47 (brt, *J* = 4.4 Hz, 4H), 2.53 (t, *J* = 7.2 Hz, 2H), 3.72 (brt, *J* = 4.4 Hz, 4H), 4.12 (t, *J* = 6.2 Hz, 2H), 7.00 (d, *J* = 8.8 Hz, 2H), 7.83 (d, *J* = 8.8 Hz, 2H), 9.88 (s, 1H).
APCI-MS (m/z); 250 [M+H]⁺

### Step 2

In a similar manner to Step 2 of Example 29, Compound 31 (0.13 g, 18%) was obtained using 4-(3-morpholinopropoxy)benzaldehyde (0.51 g, 2.04 mmol) obtained in Step 1, (1*H*-indazol-3-ylmethyl)triphenylphosphonium iodide (1.00 g, 1.92 mmol) and potassium carbonate (0.56 g, 4.08 mmol).
¹H-NMR (270 MHz, DMSO-*d*₆) δ 1.89 (m, 2H), 2.36-2.39 (m, 4H), 2.43 (t, *J* = 7.1 Hz, 2H), 3.56-3.59 (m, 4H), 4.04 (t, *J* = 6.4 Hz, 2H), 6.95 (d, *J* = 8.6 Hz, 2H), 7.18 (t, *J* = 8.3 Hz, 1H), 7.36 (d, *J* = 16.8 Hz, 1H), 7.38 (t, *J* = 8.3 Hz, 1H), 7.46 (d, *J* = 16.8 Hz, 1H), 7.53 (d, *J* = 8.3 Hz, 1H), 7.63 (d, *J* = 8.6 Hz, 2H), 8.15 (d, *J* = 8.3 Hz, 1H), 13.1 (brs, 1H).
APCI-MS (m/z); 364 [M+H]⁺

### Example 32: (E)-3-{2-[4-(2-morpholinoethoxy)-3-nitrophenyl]vinyl}-1H-indazole (Compound 32)

### Step 1

In a similar manner to Step 1 of Example 29, the crude product was obtained using 4-hydroxy-3-nitrobenzaldehyde (6.37 g, 38.1 mmol), potassium carbonate (15.8 g, 114 mmol) and 2-morpholinoethyl chloride hydrochloride (7.09 g, 38.1 mmol), and then the crude product was treated with 4 moL/L hydrogen chloride-ethyl acetate solution (10.0 mL, 40.0 mmol) in a similar manner to Step 2 of Example 2, to obtain 4-(2-morpholino)-3-nitrobenzaldehyde hydrochloride (6.07 g, 50%).
¹H-NMR (300 MHz, DMSO-*d*₆) δ 3.24-4.00 (m, 10H), 4.78 (t, *J* = 4.7 Hz, 2H), 7.62 (d, *J* = 8.8 Hz, 1H), 8.22 (dd, *J* = 2.0, 8.8 Hz, 1H), 8.48 (d, *J* = 2.0 Hz, 1H), 9.97 (s, 1H), 11.6 (brs, 1H).
ESI-MS (m/z); 281 [M+H]⁺

### Step 2

The crude product obtained using 4-(2-morpholino)-3-nitrobenzaldehyde hydrochloride (0.32 g, 1.02 mmol) obtained in Step 1, (1*H*-indazol-3-ylmethyl)triphenylphosphonium iodide (0.70 g, 1.35 mmol) and potassium carbonate (0.28 g, 2.00 mmol) in a similar manner to Step 2 of Example 29, was purified by silica gel chromatography [amino-modified chemically bound silica gel Chromatorex (registered trademark) NH, Fuji Silysia Chemical Ltd.; ethyl acetate, then methanol/ethyl acetate=1/19], and was crystallized from ethyl acetate to obtain Compound 32 (0.26 g, 49%).
¹H-NMR (270 MHz, DMSO-*d*₆) δ 2.46-2.51 (m, 4H), 2.71 (t, *J* = 5.6 Hz, 2H), 3.54-3.58 (m, 4H), 4.32 (t, *J* = 5.6 Hz, 2H), 7.20 (t, *J* = 7.5 Hz, 1H), 7.40 (t, *J* = 7.5 Hz, 1H), 7.42 (d, *J* = 8.9 Hz, 1H), 7.51 (d, *J* = 16.9 Hz, 1H), 7.55 (d, *J* = 7.5 Hz, 1H), 7.59 (d, *J* = 16.9 Hz, 1H), 8.01 (dd, *J* = 2.3, 8.9 Hz, 1H), 8.22 (d, *J* = 7.5 Hz, 1H), 8.23 (d, *J* = 2.3 Hz, 1H).
APCI-MS (m/z); 395 [M+H]⁺

### Example 33: (E)-3-{2-[3-methoxy-4-(3-morpholinopropoxy)phenyl]vinyl}-1H-indazole (Compound 33)

### Step 1

In a similar manner to Step 1 of Example 29, the crude product was obtained using vanillin (1.27 g, 8.35 mmol), potassium carbonate (1.92 g, 13.9 mmol) and 3-morpholinopropyl chloride (1.21 g, 7.42 mmol), and then the crude product was treated with 4 moL/L hydrogen chloride-ethyl acetate solution (4.0 mL, 16.0 mmol) in a similar manner to Step 2 of Example 2, to obtain 3-methoxy-4-(3-morpholinopropoxy)benzaldehyde hydrochloride (1.15 g, 56%).
¹H-NMR (270 MHz, CDCl₃) δ 2.04-2.12 (m, 2H), 2.54 (brt, *J* = 4.6 Hz, 4H), 2.60 (t, *J* = 7.3 Hz, 2H), 3.74 (brt, *J* = 4.6 Hz, 4H), 3.92 (s, 3H), 4.18 (t, *J* = 6.6 Hz, 2H), 6.99 (d, *J* = 8.1 Hz, 1H), 7.41 (d, *J* = 2.0 Hz, 1H), 7.43 (dd, *J* = 2.0, 8.1 Hz, 1H), 9.85 (s, 1H).
ESI-MS (m/z); 280 [M+H]⁺

### Step 2

In a similar manner to Step 2 of Example 29, Compound 33 (0.32 g, 30%) was obtained using 3-methoxy-4-(3-morpholinopropoxy)benzaldehyde hydrochloride (0.76 g, 2.72 mmol) obtained in Step 1, (1*H*-indazol-3-ylmethyl)triphenylphosphonium iodide (1.42 g, 2.73 mmol) and potassium carbobate (0.75 g, 5.46 mmol).
¹H-NMR (300 MHz, CDCl₃) δ 2.00-2.09 (m, 2H), 2.48 (brt, *J* = 4.6 Hz, 4H), 2.55 (t, *J* = 7.3 Hz, 2H), 3.73 (brt, *J* = 4.6 Hz, 4H), 3.94 (s, 3H), 4.13 (t, *J* = 6.7 Hz, 2H), 6.92 (d, *J* = 8.3 Hz, 1H), 7.11 (dd, *J* = 2.0, 8.3 Hz, 1H), 7.16 (d, *J* = 1.8 Hz, 1H), 7.22-7.27 (m, 1H), 7.33 (d, *J* = 16.7 Hz, 1H), 7.40-7.51 (m, 2H), 7.48 (d, *J* = 16.7 Hz, 1H), 8.06 (d, *J* = 8.1 Hz, 1H), 10.1 (brs, 1H).
ESI-MS (m/z); 394 [M+H]⁺

### Example 34: (E)-3-{2-[3-bromo-4-(2-morpholinoethoxy)phenyl]vinyl}-1H-indazole (Compound 34)

### Step 1

A chloroform (100 mL) solution of 4-hydroxybenzaldehyde (10.0 g, 81.9 mmol) was added with bromine (4.30 mL, 83.5 mmol), follwed by stirring at room temperature for 2 hours. The reaction mixture was added with water (100 mL), and the organic layer was concentrated under reduced pressure to obtain 3-bromo-4-hydroxybenzaldehyde (quantitative yield) as pale yellow solid.
¹H-NMR (270 MHz, CDCl₃) δ 6.27 (s, 1H), 7.15 (d, *J* = 8.4 Hz, 1H), 7.78 (dd, *J* = 1.9, 8.4 Hz, 1H), 8.04 (d, *J* = 1.9 Hz, 1H), 9.83 (s, 1H).
ESI-MS (m/z); 199, 201 [M-H]⁻

### Step 2

In a similar manner to Step 1 of Example 29, 3-bromo-4-(2-morpholinoethoxy)benzaldehyde (10.9 g, 49%) was obtained using 3-bromo-4-hydroxybenzaldehyde (14.3 g, 71.0 mmol) obtained in Step 1, potassium carbonate (20.0 g, 145 mmol) and 2-morpholinoethyl chloride hydrochloride (13.5 g, 72.6 mmol).
¹H-NMR (270 MHz, CDCl₃) δ 2.65 (brt, *J* = 4.7 Hz, 4H), 2.91 (t, *J* = 5.6 Hz, 2H), 3.73 (brt, *J* = 4.7 Hz, 4H), 4.26 (t, *J* = 5.6 Hz, 2H), 7.00 (d, *J* = 8.4 Hz, 1H), 7.80 (dd, *J* = 2.0, 8.4 Hz, 1H), 8.08 (d, *J* = 2.0 Hz, 1H), 9.84 (s, 1H).
ESI-MS (m/z); 314, 316 [M+H]⁺

### Step 3

In a similar manner to Step 2 of Example 32, Compound 34 (1.23 g, 59%) was obtained using 3-bromo-4-(2-morpholinoethoxy)benzaldehyde (2.76 g, 8.78 mmol) obtained in Step 2, potassium carbonate (0.20 g, 1.45 mmol) and (1*H-*indazol-3-ylmethyl)triphenylphosphonium iodide (2.54 g, 4.88 mmol).
¹H-NMR (270 MHz, CDCl₃) δ 2.66 (brt, *J* = 4.6 Hz, 4H), 2.89 (t, *J* = 5.7 Hz, 2H), 3.74 (brt, *J* = 4.6 Hz, 4H), 4.21 (t, *J* = 5.7 Hz, 2H), 6.91 (d, *J* = 8.6 Hz, 1H), 7.22-7.28 (m, 1H), 7.31 (d, *J* = 16.8 Hz, 1H), 7.41 (d, *J* = 16.8 Hz, 1H), 7.38-7.51 (m, 3H), 7.80 (d, *J* = 2.0 Hz, 1H), 8.00 (d, *J* = 8.1 Hz, 1H), 10.0 (brs, 1H).
ESI-MS (m/z); 428, 430 [M+H]⁺

### Example 35: (E)-N-{5-[2-(1H-indazol-3-yl)vinyl]-2-(2-morpholinoethoxy)phenyl}benzamide (Compound 35)

### Step 1

A dichloromethane (10.0 mL) solution of 4-(2-morpholinoethoxy)-3-nitrobenzaldehyde (2.97 g, 9.38 mmol) obtained in Step 1 of Example 32, was added with trimethyl orthoformate (15.0 mL, 13.7 mmol) and 10% hydrogen chloride-methanol solution (5.00 mL), followed by stirring at room temperature for 4 hours. The reaction mixture was added with potassium carbonate (10.0 g, 72.4 mmol), followed by stirring at room temperature for 5 hours, and then was concentrated under reduced pressure. The residue was added with water (50 mL), and was extracted with ethyl acetate (50 mL X 3). The organic layer was dried over anhydrous sodium sulfate, and was concentrated under reduced pressure, to obtain 4-(2-morpholinoethoxy)-3-nitrobenzaldehyde dimethyl acetal (2.98 g, 97%).
¹H-NMR (300 MHz, CDCl₃) δ 2.60 (brt, *J* = 4.7 Hz, 4H), 2.85 (t, *J* = 5.6 Hz, 2H), 3.32 (s, 6H), 3.71 (brt, *J* = 4.7 Hz, 4H), 4.25 (t, *J* = 5.6 Hz, 2H), 5.39 (s, 1H), 7.07 (d, *J* = 8.6 Hz, 1H), 7.59 (dd, *J* = 2.2, 8.6 Hz, 1H), 7.93 (d, *J* = 2.2 Hz, 1H).
ESI-MS (m/z); 327 [M+H]⁺

### Step 2

An ethanol (30.0 mL) solution of 4-(2-morpholinoethoxy)-3-nitrobenzaldehyde dimethyl acetal (2.97 g, 9.10 mmol) obtained in Step 1 was added with platinum oxide (0.02 g, 0.10 mmol), followed by stirring at room temperature for 2 hours under hydrogen atmosphere. The reaction mixture was filtered, and the filtrate was concentrated under reduced pressure to obtain the crude product. The crude product was crystallized from mixed solvent of ethyl acetate/hexane (1/1) to obtain 3-amino-4-(2-morpholinoethoxy)benzaldehyde dimethyl acetal (0.70 g, 26%).
¹H-NMR (300 MHz, CDCl₃) δ 2.57 (brt, *J* = 4.7 Hz, 4H), 2.78 (t, *J* = 5.7 Hz, 2H), 3.30 (s, 6H), 3.72 (brt, *J* = 4.7 Hz, 4H), 3.91 (brs, 2H), 4.12 (t, *J* = 5.7 Hz, 2H), 5.25 (s, 1H), 6.76-6.81 (m, 3H).
ESI-MS (m/z); 297 [M+H]⁺

### Step 3

A dichloromethane (2.00 mL) solution of 3-amino-4-(2-morpholinoethoxy)benzaldehyde dimethyl acetal (0.21 g, 0.70 mmol) obtained in Step 2 was added with benzoyl chloride (0.10 mL, 0.86 mmol) and pyridine (0.10 mL, 1.24 mmol), followed by stirring at room temperature for 10 hours. The reaction mixture was added with 3 mol/L hydrochloric acid (3.00 mL), followed by stirring at room temperature for 20 minutes. The reaction mixture was neutralized with saturated aqueous sodium hydrogencarbonate solution (50 ml), and was extracted with ethyl acetate (50 mL X 3). The organic layer was dried over anhydrous sodium sulfate, and was concentrated under reduced pressure. The crude product was purified by silica gel column chromatography (chloroform, then methanol/chloroform 1/5) to obtain 3-benzoylamino-4-(2-morpholinoethoxy)benzaldehyde (0.24 g, 96%).
¹H-NMR (300 MHz, CDCl₃) δ 2.55 (brt, *J* = 4.6 Hz, 4H), 2.86 (t, *J* = 5.5 Hz, 2H), 3.65 (brt, *J* = 4.6 Hz, 4H), 4.31 (t, *J* = 5.5 Hz, 2H), 7.06 (d, *J* = 8.6 Hz, 1H), 7.48-7.69 (m, 4H), 7.91-7.94 (m, 2H), 8.81 (brs, 1H), 9.07 (d, *J* = 2.0 Hz, 1H), 9.94 (s, 1H).

### Step 4

In a similar manner to Step 2 of Example 32, Compound 35 (0.11 g, 33%) was obtained using 3-benzoylamino-4-(2-morpholinoethoxy)benzaldehyde (0.24 g, 0.69 mmol) obtained in Step 3, (1*H*-indazol-3-ylmethyl)triphenylphosphonium iodide (0.36 g, 0.69 mmol) and potassium carbonate (0.19 g, 1.38 mmol).
¹H-NMR (300 MHz, CDCl₃) δ 2.54 (brt, *J* = 4.7 Hz, 4H), 2.81 (t, *J* = 5.4 Hz, 2H), 3.60 (brt, *J* = 4.7 Hz, 4H), 4.26 (t, *J* = 5.4 Hz, 2H), 6.98 (d, *J* = 8.4 Hz, 1H), 7.23-7.28 (m, 1H), 7.33 (dd, *J* = 2.2, 8.4 Hz, 1H), 7.43 (d, *J* = 16.7 Hz, 1H), 7.40-7.60 (m, 6H), 7.94-7.98 (m, 2H), 8.08 (d, *J* = 8.0 Hz, 1H), 8.88 (d, *J* = 2.0 Hz, 1H), 8.92 (s, 1H), 10.0 (brs, 1H).
ESI-MS (m/z); 469 [M+H]⁺

### Example 36: (E)-3-{2-[4-(2-thiomorpholinoethoxyphenyl]vinyl}-1H-indazole (Compound 36)

### Step 1

A toluene (25 mL) solution of thiomorpholine (5.23 mL, 52.0 mmol) was added with 2-chlorobromoethane (2.90 mL, 34.7 mmol), followed by heating under reflux for 24 hours. After filtration of the reaction mixture, the filtrate was added with 1 moL/L hydrochloric acid, and was extracted.

The aqueous layer was adjusted to pH 8 with 2 moL/L aqueous sodium hydroxide solution, and was extracted with diethyl ether. The organic layer was washed with saturated brine, and was dried over anhydrous sodium sulfate, and then was concentrated to obtain *N*-(2-chloroethyl)thiomorpholine (1.25 g, 22%).
¹H-NMR (270 MHz, CDCl₃) δ 2.67 (dt, *J* = 4.4, 5.5 Hz, 2H), 2.71-2.81 (m, 8H), 3.56 (t, *J* = 6.9 Hz, 2H).
ESI-MS (m/z); 166 [M+H]⁺

### Step 2

In a similar manner to Step 1 of Example 29, the crude product was obtained using 4-hydroxybenzaldehyde (0.60 g, 4.91 mmol), potassium carbonate (2.00 g, 14.5 mmol) and *N-*(2-chloroethyl)thiomorpholine (1.20 g, 7.27 mmol) obtained in Step 1, and the crude product was treated with 4 moL/L hydrogen chloride-ethyl acetate solution in a similar manner to Step 2 of Example 2, to obtain 4-(2-thiomorpholinoethoxy)benzaldehyde hydrochloride (1.81 g, 87%).
¹H-NMR (270 MHz, CDCl₃) δ 2.67-2.75 (m, 6H), 2.81-2.92 (m, 4H), 4.16 (t, *J* = 5.8 Hz, 2H), 7.00 (d, *J* = 8.7 Hz, 2H), 7.83 (d, *J* = 8.7 Hz, 2H), 9.88 (s, 1H)
ESI-MS (m/z); 252 [M+H]⁺

### Step 3

In a similar manner to Step 4 of Example 1, Compound 36 (0.03 g, 3%) was obtained using 4-(2-thiomorpholinoethoxy)benzaldehyde hydrochloride (1.00 g, 3.48 mmol) obtained in Step 2, (1*H*-indazol-3-ylmethyl)triphenylphosphonium iodide (1.81 g, 3.48 mmol) and potassium carbonate (1.44 g, 10.4 mmol).
¹H-NMR (270 MHz, CDCl₃) δ 2.53-2.95 (m, 10H), 4.13 (t, *J* = 5.7 Hz, 2H), 6.92 (d, *J* = 8.8 Hz, 1H), 7.23-7.56 (m, 8H), 8.02 (d, J = 8.1 Hz, 1H).
ESI-MS (m/z); 366 [M+H]⁺

### Example 37: N-{4-[(E)-2-(1H-indazol-3-yl)vinyl]phenoxy}acetylmorpholine (Compound 37)

### Step 1

A toluene (30 mL) solution of morpholine (1.00 mL, 11.5 mmol) was added with potassium carbonate (3.17 g, 22.9 mmol) and chloroacetyl chloride (0.91 mL, 11.4 mmol), followed by stirring at 60°C for 2 hours. The reaction mixture was filtered and the filtrate was concentrated to obtain *N-*chloroacetylmorpholine (1.87 g, quantitative yield).
¹H-NMR (270 MHz, CDCl₃) δ 3.51-3.54 (m, 2H), 3.62-3.65 (m, 2H), 3.68-3.74 (m, 4H), 4.07 (s, 2H).

### Step 2

A THF (10 mL) solution of 4-hydroxybenzaldehyde (0.50 g, 4.09 mmol) was added with *N*-chloroacetylmorpholine (1.00 g, 6.12 mmol) obtained in Step 1 and potassium carbonate (1.70 g, 12.3 mmol), followed by heating under reflux at 100°C for 24 hours. The reaction mixture was concentrated, and the residue was added with ethyl acetate and saturated brine, and then was extracted. The organic layer was dried over anhydrous sodium sulfate, and was concentrated to obtain *N-*(4-formylphenoxy)acetylmorpholine (1.24 g, quantitative yield).
¹H-NMR (270 MHz, CDCl₃) δ 3.51-3.74 (m, 8H), 4.80 (s, 2H), 7.07 (d, *J* = 8.9 Hz, 2H), 7.85 (d, *J* = 8.9 Hz, 2H), 9.90 (s, 1H).

### Step 3

In a similar manner to Step 4 of Example 1, Compound 37 (0.24 g, 56%) was obtained using *N*-(4-formylphenoxy)acetylmorpholine (0.30 g, 1.20 mmol) obtained in Step 2, (1*H*-indazol-3-ylmethyl)triphenylphosphonium iodide (0.75 g, 1.44 mmol) and potassium carbonate (0.50 g, 3.62 mmol).
¹H-NMR (270 MHz, DMSO-*d*₆) δ 3.34-3.40 (brm, 4H), 3.58-3.70 (brm, 4H), 4.86 (s, 2H), 6.95 (d, *J* = 8.8 Hz, 2H), 7.18 (t, *J* = 7.6 Hz, 1H), 7.35-7.53 (m, 4H), 7.62 (d, *J* = 8.8 Hz, 2H), 8.14 (d, *J* = 8.1 Hz, 1H).
ESI-MS (m/z); 362 [M-H]⁻

### Example 38: (E)-3-{2-[3-amino-4-(2-morpholinoethoxy)phenyl]vinyl}-1H-indazole (Compound 38)

In a similar manner to Step 2 of Example 12, Compound 38 (0.04 g, 47%) was obtained using Compound 29 (0.10 g, 0.25 mmol), concentrated hydrochloric acid (2.0 mL) and tin (0.06 g, 0.51 mmol).
¹H-NMR (270 MHz, CDCl₃) δ 2.60 (brs, 4H), 2.83 (t, *J* = 5.7 Hz, 2H), 3.75 (t, *J* = 4.6 Hz, 4H), 4.18 (t, *J* = 5.7 Hz, 2H), 6.81 (d, *J* = 8.4 Hz, 1H), 6.94 (dd, *J* = 2.0, 8.2 Hz, 1H), 7.01 (d, *J* = 2.0 Hz, 1H), 7.21 (dd, *J* = 1.3, 6.7 Hz, 2H), 7.27 (d, *J* = 16.6 Hz, 1H), 7.41 (d, *J* = 16.6 Hz, 1H), 7.41-7.64 (m, 3H), 8.02 (d, *J* = 8.1 Hz, 1H), 9.90 (brs, 1H).
APCI-MS (m/z); 365 [M+H]⁺

### Example 39: (E)-3-{2-[3-hydroxy-4-(2-morpholinoethoxy)phenyl]vinyl}-1H-indazole (Compound 39)

### Step 1

A mixture of 3-methoxy-4-(2-morpholinoethoxy)benzaldehyde hydrochloride (2.79 g, 9.25 mmol) obtained in Step 1 of Example 30, D-methionine (1.52 g, 10.2 mmol) and concentrated sulfric acid (17.0 mL) was stirred at 70°C for 4 hours. After cooling to room temperature, the reaction mixture was added to water (150 mL), and a 4 mol/L aqueous sodium hydroxide solution (130 mL) was added to the solution. The reaction mixture was neutralized with a saturated sodium hydrogencarbonate solution, and then was filtered. The filtrate was extracted with ethyl acetate (100 mL X 4), and the organic layer was dried over anhydrous sodium sulfate, and then was evaporated under reduced pressure. The crude product was purified by silica gel column chromatography (ethyl acetate, then methanol/ethyl acetate = 1/9) to obtain 3-hydroxy-4-(2-morpholinoethoxy)benzaldehyde (1.48 g, 64%).
¹H-NMR (270 MHz, CDCl₃) δ 2.64-2.71 (m, 6H), 3.82 (t, *J* = 4.7 Hz, 4H), 4.17 (t, *J* = 5.1 Hz, 2H), 7.10 (d, *J* = 8.1 Hz, 1H), 7.35 (dd, *J* = 2.0, 8.1 Hz, 1H), 7.73 (d, *J* = 2.0 Hz, 1H), 9.87 (s, 1H), 10.34 (brs, 1H).
APCI-MS (m/z); 250 [M-H]⁻

### Step 2

In a similar manner to Step 5 of Example 1, Compound 39 (0.04 g, 15%) was obtained using 3-hydroxy-4-(2-morpholinoethoxy)benzaldehyde (0.31 g, 1.23 mmol) obtained in Step 1, (1*H*-indazol-3-ylmethyl)triphenylphosphonium iodide (0.40 g, 0.77 mmol) and potassium carbonate (0.15 g, 1.09 mmol).
¹H-NMR (270 MHz, CDCl₃) δ 2.63-2.68 (m, 6H), 3.85 (t, *J* = 4.8 Hz, 4H), 4.14 (t, *J* = 5.2 Hz, 2H), 7.02 (d, *J* = 1.2 Hz, 2H), 7.21-7.27 (m, 1H), 7.34 (d, J = 16.5 Hz, 1H), 7.39-7.50 (m, 3H), 7.45 (d, J = 16.5 Hz, 1H), 8.01 (d, J = 8.9 Hz, 1H).
APCI-MS (m/z); 366 [M+H]⁺

### Example 40: (E)-N-{4-[2-(1H-indazol-3-yl)vinyl]-2-methoxyphenoxy}acetylmorpholine (Compound 40)

### Step 1

In a similar manner to Step 2 of Example 37, *N*-(4-formyl-2-methoxyphenoxy)acetylmorpholine (0.85 g, 97%) was obtained using vanillin (0.50 g, 3.29 mmol), N-chloroacetylmorpholine (0.82 g, 5.02 mmol) obtained in Step 1 of Example 37 and potassium carbonate (1.36 g, 9.84 mmol). ¹H-NMR (270 MHz, CDCl₃) δ 3.51-3.74 (m, 8H), 3.93 (s, 3H), 4.86 (s, 2H), 7.05 (d, *J* = 8.9 Hz, 1H), 7.42 (d, *J* = 8.9 Hz, 2H), 9.85 (s, 1H).

### Step 2

In a similar manner to Step 5 of Example 1, Compound 40 (0.11 g, 12%) was obtained using *N*-(4-formyl-2-methoxyphenoxy)acetylmorpholine (0.85 g, 3.23 mmol) obtained in Step 1, (1*H*-indazol-3-ylmethyl)triphenylphosphonium iodide (2.00 g, 3.85 mmol) and potassium carbonate (1.34 g, 9.70 mmol).
¹H-NMR (270 MHz, CDCl₃) δ 3.66-3.68 (m, 8H), 3.94 (s, 3H), 4.79 (s, 2H), 6.98 (d, *J* = 8.3 Hz, 2H), 7.09-7.51 (m, 5H), 8.05 (d, *J* = 7.9 Hz, 2H).
ESI-MS (m/z); 394 [M+H]⁺

### Example 41: (E)-3-{2-[3-methoxy-4-(4-pyridyloxy)phenyl]vinyl}-1H-indazole hydrochloride (Compound 41)

### Step 1

A xylene (30 mL) solution of vanillin (1.00 g, 6.57 mmol) was added with 4-chloropyridine hydrochloride (1.18 g, 7.87 mmol) and 4-dimethylaminopyridine (0.52 g, 4.26 mmol), followed by heating under reflux at 140°C for 3 days. The reaction mixture was concentrated, and then the residue was added with ethyl acetate and saturated brine, and was extracted. The organic layer was dried over anhydrous sodium sulfate, and was concentrated under reduced pressure. The crude product was purified by silica gel column chromatography (ethyl acetate/n-hexane = 1/1, then ethyl acetate/methanol = 5/1) to obtain 3-methoxy-4-(4-pyridyloxy)benzaldehyde (0.27 g, 18%).
¹H-NMR (300 MHz, CDCl₃) δ 3.88 (s, 3H), 6.82 (d, *J* = 8.3 Hz, 2H), 7.25 (d, *J* = 8.2 Hz, 1H), 7.52 (d, *J* = 8.3 Hz, 1H), 7.53 (s, 1H), 8.49 (d, *J* = 8.1 Hz, 2H), 9.99 (s, 1H).

### Step 2

In a similar manner to Step 5 of Example 1, a free base of Compound 41 was obtained using 3-methoxy-4-(4-pyridyloxy)benzaldehyde (0.27 g, 1.37 mmol) obtained in Step 1, (1*H*-indazol-3-ylmethyl)triphenylphosphonium iodide (0.85 g, 1.63 mmol) and potassium carbonate (0.57 g, 4.10 mmol), and then the free base of Compound 41 was treated with 4 moL/L hydrogen chloride-ethyl acetate solution in a similar manner to Step 2 of Example 2, to obtain Compound 41 (0.33 g, 64%).
¹H-NMR (300 MHz, DMSO-*d*₆) δ 3.85 (s, 3H), 7.22 (t, *J* = 8.0 Hz, 1H), 7.33-7.70 (m, 10H), 8.23 (d, *J* = 8.2 Hz, 1H), 8.77 (d, *J* = 8.3 Hz, 1H).
ESI-MS (m/z); 344 [M+H]⁺

### Example 42: (E)-N-{5-[2-(1H-indazol-3-yl)vinyl]-2-(2-morpholinoethoxy)phenyl}acetamide (Compound 42)

### Step 1

In a similar manner to Step 3 of Example 35, *N*-[5-formyl-2-(2-morpholinoethoxy)phenyl]acetamide (0.28 g, 91%) was obtained using 3-amino-4-(2-morpholinoethoxy)benzaldehyde dimethyl acetal (0.31 g, 1.05 mmol) obtained in Step 2 of Example 35, acetic anhydride (0.50 mL, 5.30 mmol) and pyridine (0.10 mL, 1.24 mmol).
¹H-NMR (270 MHz, CDCl₃) δ 2.24 (s, 3H), 2.57 (brt, *J* = 4.7 Hz, 4H), 2.83 (t, *J* = 5.7 Hz, 2H), 3.74 (brt, *J* = 4.7 Hz, 4H), 4.27 (t, *J* = 5.7 Hz, 2H), 7.02 (d, *J* = 8.4 Hz, 1H), 7.64 (dd, *J* = 2.0, 8.4 Hz, 1H), 8.05 (brs, 1H), 8.87 (d, *J* = 2.0 Hz, 1H), 9.90 (s, 1H).
APCI-MS (m/z); 293 [M+H]⁺

### Step 2

In a similar manner to Step 5 of Example 1, Compound 42 (0.15 g, 50%) was obtained using *N*-[5-formyl-2-(2-morpholinoethoxy)phenyl]acetamide (0.22 g, 0.75 mmol) obtained in Step 1, (1*H*-indazol-3-ylmethyl)triphenylphosphonium iodide (0.34 g, 0.65 mmol) and potassium carbonate (0.18 g, 1.30 mmol).
¹H-NMR (300 MHz, CDCl₃) δ 2.58 (brt, *J* = 4.7 Hz, 4H), 2.79 (t, *J* = 5.4 Hz, 2H), 3.76 (brt, *J* = 4.7 Hz, 4H), 4.22 (t, *J* = 5.4 Hz, 2H), 6.93 (d, *J* = 8.6 Hz, 1H), 7.21-7.30 (m, 2H), 7.37 (d, *J* = 16.5 Hz, 1H), 7.38-7.49 (m, 2H), 7.49 (d, *J* = 16.5 Hz, 1H), 8.05 (d, *J* = 8.1 Hz, 1H), 8.19 (brs, 1H), 8.63 (brs, 1H), 10.0 (brs, 1H).
APCI-MS (m/z); 407 [M+H]⁺

### Example 43: (E)-N-{4-[2-(1H-indazol-3-yl)vinyl]-2-methylphenoxy}acetylmorpholine (Compound 43)

### Step 1

In a similar manner to Step 2 of Example 37, *N*-(4-formyl-2-methylphenoxy)acetylmorpholine (0.50 g, 52%) was obtained using 4-hydroxy-3-methylbenzaldehyde (0.50 g, 3.67 mmol), *N*-chloroacetylmorpholine (0.90 g, 5.55 mmol) obtained in Step 1 of Example 37 and potassium carbonate (1.52 g, 11.0 mmol).
¹H-NMR (270 MHz, CDCl₃) δ 2.30 (s, 3H), 3.63-3.70 (m, 8H), 4.83 (s, 2H), 6.95 (d, *J* = 8.9 Hz, 1H), 7.66-7.68 (m, 2H), 9.84 (s, 1H).

### Step 2

In a similar manner to Step 5 of Example 1, Compound 43 (0.16 g, 22%) was obtained using *N*-(4-formyl-2-methylphenoxy)acetylmorpholine (0.50 g, 1.90 mmol) obtained in Step 1, (1*H*-indazol-3-ylmethyl)triphenylphosphonium iodide (1.19 g, 2.29 mmol) and potassium carbonate (0.79 g, 5.70 mmol).
¹H-NMR (270 MHz, CDCl₃) δ 2.27 (s, 3H), 3.56-3.78 (m, 8H), 4.73 (s, 2H), 6.85 (d, *J* = 8.4 Hz, 2H), 7.18-7.48 (m, 5H), 8.02 (d, *J* = 8.1 Hz, 2H).
ESI-MS (m/z); 378 [M+H]⁺

### Example 44: (E)-N,N-dimethyl-4-[2-(1H-indazol-3-yl)vinyl]-2-methoxyphenoxyacetamide (Compound 44)

### Step 1

A dichlorometane (200 mL) solution of dimethylamine hydrochloride (10.2 g, 128 mmol) was added with triethylamine (35.0 mL, 251 mmol), chloroacetylchloride (10.0 mL, 126 mmol), followed by stirring st room temperature for 6 hours. The reaction mixture was filtered and the filtrate was concentrated to obtain *N*,*N-*dimethylchloroacetamide (15.2 g).
¹H-NMR (270 MHz, CDCl₃) δ 2.99 (s, 3H), 3.10 (s, 3H), 4.09 (s, 2H).

### Step 2

In a similar manner to Step 2 of Example 37, *N,N-*dimethyl-(4-formyl-2-methoxyphenoxy)acetamide (0.16 g, 10%) was obtained using vanillin (1.00 g, 6.57 mmol), *N,N-*dimethylchloroacetamide (1.20 g, 9.88 mmol) obtained in Step 1 and potassium carbonate (3.40 g, 24.6 mmol).
¹H-NMR (270 MHz, CDCl₃) δ 2.97 (s, 3H), 3.11 (s, 3H), 3.94 (s, 3H), 4.86 (s, 2H), 7.01 (d, *J* = 8.9 Hz, 1H), 7.40-7.43 (m, 2H), 9.85 (s, 1H).

### Step 3

In a similar manner to Step 5 of Example 1, Compound 44 (0.14 g, 60%) was obtained using *N*,*N*-dimethyl-(4-formyl-2-methoxyphenoxy)acetamide (0.16 g, 0.68 mmol) obtained in Step 2, (1*H*-indazol-3-ylmethyl)triphenylphosphonium iodide (0.42 g, 0.81 mmol) and potassium carbonate (0.28 g, 2.03 mmol).
¹H-NMR (270 MHz, DMSO-*d*₆) δ 2.85 (s, 3H), 3.01 (s, 3H), 3.86 (s, 3H), 4.79 (s, 2H), 6.86 (d, *J* = 8.4 Hz, 1H), 7.19 (m, 1H), 7.36-7.53 (m, 5H), 8.17 (d, *J* = 8.1 Hz, 2H), 13.1 (brs, 1H).
ESI-MS (m/z); 352 [M+H]⁺

### Example 45: (E)-3-{2-[3-methoxy-4-(2-(methylaminocarbonyl)pyridin-4-yloxy)phenyl]vinyl}-1H-indazole (Compound 45)

### Step 1

Thionyl chloride (6.00 mL, 82.3 mmol) was added with DMF (0.20 mL, 2.58 mmol), followed by stirring at 45°C for 15 minutes. The reaction mixture was added with picolinic acid (2.00 g, 16.3 mmol), followed by stirring at 45°C for 45 minutes and at 80°C for 3 days. The reaction mixture was concentrated, and then the residue was added with chloroform and sodium hydrogencarbonate, and was extracted. The organic layer was washed with saturated brine and was dried over anhydrous sodium sulfate, and the solvent was evaporated to obtain 4-chloropicolinic acid (2.78 g).
¹H-NMR (300 MHz, CDCl₃) δ 7.27 (s, 1H), 7.62 (brd, *J* = 5.5 Hz, 1H), 8.12 (s, 1H), 8.73 (d, *J* = 5.1 Hz, 1H).

### Step 2

4-chloropicolinic acid (2.78 g, 17.7 mmol) obtained in Step 1 was dissolved in THF (50 mL), and the solution was added with methylamine hydrochloride (2.41 g, 35.7 mmol), 1-hydroxybenzotriazole monohydrate (3.61 g, 26.7 mmol) and 1-ethyl-3-(3-dimethylaminopropyl)carbodiimide hydrochloride (5.13 g, 26.8 mmol), followed by stirring at room temperature for 2 hours. The reaction mixture was added with a saturated aqueous sodium hydrogencarbonate solution, and was extracted with ethyl acetate. The organic layer was sequentially washed with water and saturated brine, and was dried over anhydrous magnesium sulfate, and the solvent was evaporated under reduced pressure. The residue was purified by silica gel column chromatography (chloroform/methanol=25/1) to obtain *N*-methyl-4-chloropicolinamide (2.04 g, 2 steps 74%).
¹H-NMR (300 MHz, CDCl₃) d 3.04 (d, *J* = 5.1 Hz, 3H), 7.43 (m, 1H), 7.96 (br, 1H), 8.21 (s, 1H), 8.44 (d, *J* = 5.4 Hz, 1H). Step 3

In a similar manner to Step 1 of Example 41, *N*-methyl-4-(4-formyl-2-methoxyphenoxy)picolinamide (0.05 g, 6%) was obtained using vanillin (0.45 g, 2.94 mmol), *N*-methyl-4-chloropicolinamide (0.50 g, 2.94 mmol) obtained in Step 2 and 4-dimethylaminopyridine (0.26 g, 2.13 mmol).
¹H-NMR (300 MHz, CDCl₃) δ 3.00 (d, *J* = 5.2 Hz, 3H), 3.85 (s, 3H), 6.98 (m, 1H), 7.25 (s, 1H), 7.42 (m, 1H), 7.56 (s, 1H), 7.62 (brs, 1H), 8.02 (br, 1H), 8.40 (d, *J* = 5.6 Hz, 1H), 9.98 (s, 1H).

### Step 4

In a similar manner to Step 5 of Example 1, Compound 45 (0.02 g, 32%) was obtained using *N*-methyl-4-(4-formyl-2-methoxyphenoxy)picolinamide (0.05 g, 0.18 mmol) obtained in Step 3, (1*H*-indazol-3-ylmethyl)triphenylphosphonium iodide (0.11 g, 0.22 mmol) and potassium carbonate (0.08 g, 0.55 mmol).
¹H-NMR (300 MHz, DMSO-*d*₆) δ 2.77 (d, *J* = 5.1 Hz, 3H), 3.82 (s, 3H), 7.10-7.38 (m, 6H), 7.53-7.61 (m, 4H), 8.23 (d, *J* = 8.1 Hz, 1H), 8.47 (d, *J* = 5.4 Hz, 1H), 8.74 (brm, 1H), 13.2 (br, 1H).
ESI-MS (m/z); 400 [M]⁺

### Example 46: (E)-N,N-dimethyl-4-[2-(1H-indazol-3-yl)vinyl]-2-methylphenoxyacetamide (Compound 46)

### Step 1

In a similar manner to Step 2 of Example 44, *N,N-*dimethyl-(4-formyl-2-methylphenoxy)acetamide (0.86 g, quantitative yield) was obtained using 4-hydroxy-3-methylbenzaldehyde (0.50 g, 3.67 mmol), *N,N-*dimethylchloroacetamide (0.75 g, 6.13 mmol) obtained in a similar manner to Step 1 of Example 44 and potassium carbonate (1.52 g, 11.0 mmol).
¹H-NMR (270 MHz, CDCl₃) δ 2.32 (s, 3H), 2.98 (s, 3H), 3.10 (s, 3H), 4.83 (s, 2H), 6.93 (d, *J* = 8.0 Hz, 1H), 7.63-7.67 (m, 2H), 9.83 (s, 1H).

### Step 2

In a similar manner to Step 5 of Example 1, Compound 46 (0.52 g, 40%) was obtained using *N,N*-dimethyl-(4-formyl-2-methylphenoxy)acetamide (0.68 g, 3.89 mmol) obtained in Step 1, (1*H*-indazol-3-ylmethyl)triphenylphosphonium iodide (2.40 g, 4.62 mmol) and potassium carbonate (1.61 g, 11.7 mmol).
¹H-NMR (270 MHz, DMSO-*d*₆) δ 2.23 (s, 3H), 2.85 (s, 3H), 3.01 (s, 3H), 4.85 (s, 2H), 6.85 (d, *J* = 8.4 Hz, 1H), 7.17 (t, *J* = 7.9 Hz, 1H), 7.32-7.53 (m, 5H), 8.14 (d, *J* = 8.1 Hz, 2H), 13.0 (br, 1H).
ESI-MS (m/z); 336 [M+H]⁺

### Example 47: (E)-3-{2-[3-cyano-4-(2-morpholinoethoxy)phenyl]vinyl}-1H-indazole (Compound 47)

### Step 1

A THF (10.0 mL) solution of 3-bromo-4-(2-morpholinoethoxy)benzaldehyde (0.65 g, 1.80 mmol) obtained in Step 2 of Example 34 was cooled to -78°C, and *n-*butyllithium (1.60 mol/L, 2.40 mL, 3.84 mmol) was dropped to the solution over 3 minutes, followed by stirring at -78°C for 5 minutes. The reaction mixture was added with *p-*toluensulfonylcyanide (0.78 g, 4.32 mmol), and was stirred for 3 hours while temperature was raised to room temperature. The reaction mixture was concentrated under reduced pressure, and the residue was added with 1 mol/L aqueous ammonium acetate solution (30 mL), and was purified by HP-20 column chromatography (water, then acetnitrile/water=2/1), followed by crystallization from mixed solvent of ethyl acetate/hexane (1/1) to obtain 3-cyano-4-(2-morpholinoethoxy)benzaldehyde (0.24 g, 51%).
¹H-NMR (300 MHz, CDCl₃) δ 2.65 (brt, *J* = 4.7 Hz, 4H), 2.92 (t, *J* = 5.6 Hz, 2H), 3.73 (brt, *J* = 4.7 Hz, 4H), 4.33 (t, *J* = 5.6 Hz, 2H), 7.12 (d, *J* = 8.6 Hz, 1H), 8.07 (dd, *J* = 2.2, 8.6 Hz, 1H), 8.10 (d, *J* = 2.2 Hz, 1H), 9.90 (s, 1H).
ESI-MS (m/z); 261 [M+H]⁺

### Step 2

In a similar manner to Step 5 of Example 1, Compound 47 (0.11 g, 33%) was obtained using 3-cyano-4-(2-morpholinoethoxy)benzaldehyde (0.24 g, 0.91 mmol) obtained in Step 1, (1*H*-indazol-3-ylmethyl)triphenylphosphonium iodide (0.45 g, 0.87 mmol) and potassium carbobate (0.25 g, 1.82 mmol).
¹H-NMR (270 MHz, CDCl₃) δ 2.65 (brt, *J* = 4.7 Hz, 4H), 2.90 (t, *J* = 5.6 Hz, 2H), 3.74 (brt, *J* = 4.7 Hz, 4H), 4.26 (t, *J* = 5.6 Hz, 2H), 7.00 (d, *J* = 8.6 Hz, 1H), 7.24-7.30 (m, 1H), 7.33 (d, *J* = 16.4 Hz, 1H), 7.40-7.52 (m, 2H), 7.43 (d, *J* = 16.4 Hz, 1H), 7.72-7.77 (m, 2H), 8.00 (d, *J* = 8.1 Hz, 1H), 10.0 (brs, 1H).
APCI-MS (m/z); 375 [M+H]⁺

### Example 48: (E)-3-{2-[3-methyl-4-(2-morpholinoethoxy)phenyl]vinyl}-1H-indazole (Compound 48)

### Step 1

A DMF (12 mL) solution of 4-hydroxy-3-methylbenzaldehyde (1.00 g, 7.34 mmol) was added with potassium carbonate (2.03 g, 14.7 mmol) and 4-(2-chloroethyl)morpholine hydrochloride (1.50 g, 8.07 mmol), followed by stirring at 70°C for 4 hours. The reation mixture was concentrated, and the residue was added with ethyl acetate and water, and then was extracted. The organic layer was washed with saturated brine and was dried over anhydrous sodium sulfate, and the solvent was evaporated. The residue was purified by silica gel column chromatography (chloroform/methanol=10/1) to obtain 3-methyl-4-(2-morpholinoethoxy)benzaldehyde (1.37 g, 75%).
¹H-NMR (300 MHz, CDCl₃) δ 2.26 (s, 3H) , 2.62 (t, *J* = 4.77 Hz, 4H), 2.87 (t, *J* = 5.69 Hz, 2H), 3.73 (t, *J* = 4.77 Hz, 4H), 4.21 (t, *J* = 5.69 Hz, 2H), 6.91 (d, *J* = 8.99 Hz, 1H), 7.69-7.72 (m, 2H), 9.85 (s, 1H).
EI-MS (m/z); 250 [M+H]⁺

### Step 2

In a similar manner to Step 5 of Example 1, Compound 48 (0.21 g, 71%) was obtained using 3-methyl-4-(2-morpholinoethoxy)benzaldehyde (0.30 g, 1.2 mmol) obtained in Step 1, (1*H*-indazol-3-ylmethyl)triphenylphosphonium iodide (0.42 g, 0.81 mmol) and potassium carbonate (0.50 g, 3.6 mmol).
¹H-NMR (300 MHz, CDCl₃) δ 2.27 (s, 3H), 2.63 (t, *J* = 4.77 Hz, 4H), 2.86 (t, *J* = 5.50 Hz, 2H), 3.75 (t, *J* = 4.77 Hz, 4H), 4.17 (t, *J* = 5.50 Hz, 2H), 6.83 (d, *J* = 8.44 Hz, 1H), 7.21-7.50 (m, 7H), 8.04 (d, *J* = 8.07 Hz, 1H).
APCI-MS (m/z); 364 [M+H]⁺

### Example 49: (E)-4-acetyl-1-{4-[2-(1H-indazol-3-yl)vinyl]-2-methylphenoxy}acetylpiperazine (Compound 49)

### Step 1

A THF (8.0 mL) solution of 4-hydroxy-3-methylbenzaldehyde (400 mg, 2.94 mmol) was added with potassium carbonate (813 mg, 5.88 mmol) and 1-acetyl-4-chloroacetylpiperazine (1.69 g, 8.26 mmol), followed by stirring at 50°C for 3 days. The reaction mixture was added with ethyl acetate and a saturated aqueous ammonium chloride solution, and was extracted. The organic layer was washed with saturated brine and was dried over anhydrous sodium sulfate, and the solvent was evaporated. The residue was purified by silica gel column chromatography (chloroform/methanol=10/1) to obtain 4-(4-acetylpiperazin-1-ylcarbonyl)methoxy-3-methylbenzaldehyde (304 mg, 34%).
¹H-NMR (270 MHz, CDCl₃) δ 2.12 (s, 3H), 2.30 (s, 3H), 3.43-3.50 (br, 2H), 3.60-3.70 (br, 6H), 4.84 (s, 2H), 6.97 (d, *J* = 8.44 Hz, 1H), 7.69-7.71 (m, 2H), 9.87 (s, 1H).
ESI-MS (m/z); 305 [M+H]⁺

### Step 2

In a similar manner to Step 5 of Example 1, Compound 49 (0.029 g, 16%9 was obtained using 4-(4-acetylpiperazin-1-ylcarbonyl)methoxy-3-methylbenzaldehyde (0.19 g, 0.64 mmol) obtained in Step 1, (1*H*-indazol-3-ylmethyl)triphenylphosphonium iodide (0.22 g, 0.43 mmol) and potassium carbonate (0.27 g, 1.9 mmol).
¹H-NMR (270 MHz, CDCl₃) δ 2.13 (s, 3H), 2.30 (s, 3H), 3.43-3.55 (br, 2H), 3.63-3.75 (br, 6H), 4.78 (s, 2H), 6.85-6.95 (br, 1H), 7.25-7.53 (br, 7H), 8.00-8.10 (br, 1H).
ESI-MS (m/z); 419 [M+H]⁺

### Example 50: (E)-3-{2-[4-(N-methylcarbamoylmethoxy)phenyl]vinyl}-1H-indazole (Compound 50)

The crude product of Compound 11 (706 mg, 2.4 mmol) obtained in Step 11 was dissolevd in THF (20 mL), and the solution was added with methylamine hydrochloride (0.24 mL, 3.60 mmol), 1-hydroxybenzotriazole monohydrate (0.42 g, 3.11 mmol), 1-ethyl-3-(3-dimethylaminopropyl)carbodiimide hydrochloride (0.65 g, 3.39 mmol) and *N*-methylmorpholine (0.54 mL, 4.80 mmol), followed by stirring at 60°C for 5 hours. The reaction mixture was added with a saturated aqueous sodium hydrogencarbonate solution, and was extracted with ethyl acetate. The organic layer was sequentially washed with water and saturated brine, and was dried over anhydrous magnesium sulfate, and the solvent was evaporated under reduced pressure. The residue was triturated with mixed solvent of ethyl acetate/diethylether to obtain Compound 50 (0.073 g, 10%).
¹H-NMR (270 MHz, DMSO-*d*₆) δ 2.66 (d, *J* = 7.1 Hz, 3H), 4.99 (s, 2H), 6.98 (d, *J* = 8.8 Hz, 2H), 7.18 (t, *J* = 7.8 Hz, 1H), 7.36 (d, *J* = 8.8 Hz, 1H), 7.39 (d, *J* = 16.5 Hz, 1H), 7.41 (d, *J* = 16.5 Hz, 1H), 7.52 (d, *J* = 8.8 Hz, 1H), 7.65 (d, *J* = 8.8 Hz, 2H), 8.04 (br, 1H), 8.15 (d, *J* = 7.8 Hz, 1H), 13.1 (br, 1H).
APCI-MS (m/z); 308 [M+1]⁺

### Example 51: (E)-3-[2-(3-chloro-4-methoxyphenyl)vinyl]-1H-indazole (Compound 51)

In a similar manner to Step 5 of Example 1, Compound 51 (130 mg, 31%) was obtained using 3-chloro-4-methoxybenzaldehyde (272 mg, 1.59 mmol), (1*H*-indazol-3-ylmethyl)triphenylphosphonium iodide (764 mg, 1.47 mmol) and potassium carbonate (506 mg, 3.66 mmol).
¹H-NMR (300 MHz, CDCl₃) δ 3.94 (s, 3H), 6.96 (d, *J* = 8.62 Hz, 1H), 7.23-7.51 (m, 6H), 7.64 (d, *J* = 2.02 Hz, 1H), 8.02 (d, *J* = 8.25 Hz, 1H).
APCI-MS (m/z); 285 [M+H]⁺

### Example 52: (E)-3-{2-[4-(4-formylpiperazin-1-yl)phenyl]vinyl}-1H-indazole (Compound 52)

(1*H*-indazol-3-ylmethyl)triphenylphosphonium iodide (6.85 g, 13.2 mmol) obtained in Step 4 of Example 1 was dissolved in methanol (100 mL), and the solution was added with 4-(4-formylpiperazin-1-yl)benzaldehyde (2.39 g, 11.0 mmol) and potassium carbonate (4.55 g, 33.0 mmol), followed by stirring at room temperature. The solvent was evaporated under reduced pressure, and the residue was added with a saturated aqueous sodium hydrogencarbonate solution, and was extracted with ethyl acetate. The organic layer was sequentially washed with water and sarurated brine and was dried over magnesium sulfate, and the solvent was evaporated under reduced pressure to obtain Compound 52 (3.28 g, 90%). ¹H-NMR (270 MHz, DMSO-*d*₆) δ 3.14-3.32 (m, 4H), 3.49-3.54 (m, 4H), 6.99 (d, *J* = 8.6 Hz, 2H), 7.16 (t, *J* = 8.6 Hz, 1H), 7.32 (d, *J* = 8.6 Hz, 1H), 7.33 (d, *J* = 16.7 Hz, 1H), 7.42 (d, *J* = 16.7 Hz, 1H), 7.53 (d, *J* = 8.6 Hz, 1H), 7.57 (d, *J* = 8.6 Hz, 2H), 8.08 (br, 1H), 8.13 (d, *J* = 8.1 Hz, 1H).
APCI-MS (m/z); 333 [M+1]⁺

### Example 53: (E)-3-{2-[4-(piperazin-1-yl)phenyl]vinyl}-1H-indazole hydrochloride (Compound 53)

Compound 52 (2.78 g, 8.37 mmol) was dissolved in methanol (100 mL), and the solution was added with a 10% hydrogen chloride-methanol solution (20 mL), followed by stirring at 60°C for 3 hours. The solvent was evaporated under reduced pressure, and the residue was crystallized from acetone and ethyl acetate to obtain Compound 53 (3.16 g, 100%).
¹H-NMR (270 MHz, DMSO-d₆) δ 3.22 (br, 4H), 3.43 (br, 4H), 7.01 (d, *J* = 8.6 Hz, 2H), 7.17 (t, *J* = 8.6 Hz, 1H), 7.32 (d, *J* = 8.6 Hz, 1H), 7.35 (d, *J* = 16.7 Hz, 1H), 7.44 (d, *J* = 16.7 Hz, 1H), 7.52 (d, *J* = 8.6 Hz, 1H), 7.60 (d, *J* = 8.6 Hz, 2H), 8.14 (d, *J* = 8.6 Hz, 1H), 9.14 (br, 1H).
APCI-MS (m/z); 305 [M+1]⁺

### Example 54: (E)-3-{2-[4-(4-acetylpiperazin-1-yl)phenyl]vinyl}-1H-indazole (Compound 54)

Compound 53 (138 mg, 0.45 mmol) was dissolved in THF (5 mL), and the solution was added with acetic acid (0.023 mL, 0.41 mmol), 1-hydroxybenzotriazole monohydrate (71 mg, 0.53 mmol), 1-ethyl-3-(3-dimethylaminopropyl)carbodiimide hydrochloride (110 mg, 0.57 mmol) and *N*-methylmorpholine (0.20 mL, 1.64 mmol), followed by stirring at 60°C for 3.3 hours. The reaction mixture was added with a saturated aqueous sodium hydrogencarbonate solution, and was extracted with ethyl acetate. The organic layer was sequentially washed with water and saturated brine, and was dried over anhydrous sodium sulfate, and the solvent was evaporated under reduced pressure. The residue was crystallized to obtain Compound 54 (45.7 mg, 30%).
¹H-NMR (270 MHz, DMSO-*d*₆) δ 2.04 (s, 3H), 3.13-3.24 (m, 4H), 3.56-3.57 (m, 4H), 6.97 (d, *J* = 8.1 Hz, 2H), 7.17 (t, *J* = 7.4 Hz, 1H), 7.32 (d, *J* = 7.4 Hz, 1H), 7.33 (d, *J* = 16.7 Hz, 1H), 7.47 (d, *J* = 16.7 Hz, 1H), 7.51 (d, *J* = 8.1 Hz, 1H), 7.57 (d, *J* = 8.1 Hz, 2H), 8.14 (d, *J* = 8.1 Hz, 1H), 13.0 (br, 1H).
APCI-MS (m/z); 347 [M+H]⁺

### Example 55: (E)-3-{2-[3-chloro-4-(2-morpholinoethoxy)phenyl]vinyl}-1H-indazole (Compound 55)

In a similar manner to Step 5 of Example 1, Compound 55 (0.072 g, 69%) was obtained using (1*H*-indazol-3-ylmethyl)triphenylphosphonium iodide (0.17 g, 0.33 mmol), 3-chloro-4-(2-morpholinoethoxy)benzaldehyde (0.074 g, 0.27 mmol) and potassium carbonate (0.11 g, 0.82 mmol).
¹H-NMR (270 MHz, CDCl₃) δ 2.65 (t, *J* = 4.79 Hz, 4H), 2.89 (t, *J* = 5.61 Hz, 2H), 3.75 (t, *J* = 4.79 Hz, 4H), 4.22 (t, *J* = 5.61 Hz, 2H), 6.95 (d, *J* = 8.59 Hz, 1H), 7.23-7.51 (m, 6H), 7.63 (d, *J* = 2.15 Hz, 1H), 9.98 (brs, 1H), 8.01 (d, *J* = 8.26 Hz, 1H).
APCI-MS (m/z); 384 [M+H]⁺

### Example 56: (E)-3-{2-[3-amino-4-(3-dimethylaminopropyloxy)phenyl]vinyl}-1H-indazole (Compound 56)

### Step 1

In a similar manner to Step 5 of Example 1, (*E*)-3-{2-[4-(3-dimethylaminopropyloxy)-3-nitrophenyl]vinyl}-1*H-*indazole (0.21 g, 68%) was obtained using (1*H*-indazol-3-ylmethyl)triphenylphosphonium iodide (1.07 g, 5.16 mmol) 3-nitro-4-(3-dimethylaminopropyloxy)benzaldehyde (0.22 g, 0.86 mmol) and potassium carbonate (0.36 g, 2.6 mmol).
¹H-NMR (300 MHz, CDCl₃) d 2.05 (t, *J* = 4.77 Hz, 2H), 2.28 (s, 6H), 2.52 (t, *J* = 6.97 Hz, 4H), 4.22 (t, *J* = 6.24 Hz, 2H), 7.12 (d, *J* = 8.80 Hz, 1H), 7.24-7.34 (m, 1H), 7.40-7.52 (m, 4H), 7.71 (dd, *J* = 8.72, 2.20 Hz, 1H), 8.01 (d, *J* = 8.07 Hz, 1H), 8.04 (d, *J* = 2.39 Hz, 1H), 10.4 (brs, 1H).
ESI-MS (m/z); 367 [M+H]⁺

### Step 2

An ethanol (4.5 mL) solution of (*E*)-3-{2-[4-(3-dimethylaminopropyloxy)-3-nitrophenyl]vinyl}-1*H*-indazole (210 mg, 0.57 mmol) was added with concentrated hydrochloric acid (4.5 mL) and tin (200 mg, 1.68 mmol) at room temperature, followed by stirring at 60°C for 30 minutes. The resulting precipitate was collected by filtration, and was added with ethyl acetate and a saturated aqueous sodium hydrogencarbonate solution, and was extracted. The organic layer was washed with saturated brine and was dried over anhydrous sodium sulfate, and the solvent was evaporated. The residue was added with ethyl acetate and methanol, and the resulting precipitate was collected by filtration to obtain Compound 56 (206 mg).
¹H-NMR (270 MHz, CDCl₃) δ 1.99-2.05 (m, 2H), 2.28 (s, 6H), 2.50 (t, *J* = 7.25 Hz, 2H), 4.06-4.16 (m, 2H), 6.81 (d, *J* = 8.07 Hz, 1H), 6.92 (d, *J* = 2.14 Hz, 1H), 7.01 (d, *J* = 1.81 Hz, 1H), 7.23-7.29 (m, 2H), 7.39-7.50 (m, 3H), 8.03 (d, *J* = 7.91 Hz, 1H), 10.0 (brs, 1H).
ESI-MS (m/z); 337 [M+H]⁺

### Example 57: (E)-3-{2-[3-amino-4-morpholinophenyl]vinyl}-1H-indazole (Compound 57)

### Step 1

A dichloromethane (10 mL) solution of 4-fluoro-3-nitrobenzaldehyde (0.44 g, 2.6 mmol) was added with diisopropylethylamine (0.91 mL, 5.2 mmol) and morpholine (0.34 mL, 3.9 mmol) at 0°C, followed by stirring for 5.0 hours. After the reaction was finished, the reaction mixture was added with water and ethyl acetate, and was extracted. The organic layer was concentrated under reduced pressure. The residue was purified by silica gel column chromatography (chloroform/methanol=9/1) to obtain 4-morpholino-3-nitrobenzaldehyde (0.61 g, quantitative yield).
¹H-NMR (270 MHz, CDCl₃) δ 3.24 (brt, *J* = 4.6 Hz, 4H), 3.86 (brt, *J* = 4.6 Hz, 4H), 7.37 (d, *J* = 8.7 Hz, 1H), 7.96 (dd, *J* = 8.7, 2.0 Hz, 1H), 8.29 (d, *J* = 2.0 Hz, 1H), 9.87 (s, 1H). APCI-MS (m/z); 237 [M+H]⁺

### Step 2

A methanol (50 mL) solution of 4-morpholino-3-nitrobenzaldehyde (1.3 g, 5.4 mmol) was added with (1*H-*indazol-3-ylmethyl)triphenylphosphonium iodide (2.8 g, 5.4 mmol) and potassium carbonate (2.3 g, 16 mmol), followed by stirring at 50°C for 20 hours. The reaction mixture was concentrated under reduced pressure, the residue was added with ethyl acetate and water, and was extracted. The organic layer was concentrated, and the residue was added with ethyl acetate, and the resulting precipitate was collected by filtration to obtain (*E*)-3-{2-[4-morpholino-3-nitrophenyl]vinyl}-1*H*-indazole (0.57 g, 30%).
¹H-NMR (270 MHz, DMSO-d₆) δ 3.00 (brt, 4H), 3.69 (brt, 4H), 7.19 (t, *J* = 7.6 Hz, 1H), 7.32 (d, *J* = 8.4 Hz, 1H), 7.38 (t, *J* = 7.6 Hz, 1H), 7.50 (d, *J* = 16.8 Hz, 1H), 7.53 (d, *J* = 8.4 Hz, 1H), 7.58 (d, *J* = 16.8 Hz, 1H), 7.95 (dd, *J* = 8.4, 2.2 Hz, 1H), 8.17 (d, *J* = 2.2 Hz, 1H), 8.21 (d, *J* = 8.1 Hz, 1H), 13.18 (s, 1H).
APCI-MS (m/z); 351 [M+H]⁺

### Step 3

A ethanol (6.0 mL) solution of (*E*)-3-{2-[4-morpholino-3-nitrophenyl]vinyl}-1*H*-indazole (0.25 mg, 0.71 mmol) was added with concentrated hydrochloric acid (6.0 mL) and tin (0.25 g, 2.1 mmol) at 0°C, followed by stirring at 60°C for 1.0 hour. The reaction mixture was concentrated under reduced pressure, and the residue was added with ethyl acetate and a saturated aqueous sodium hydrogencarbonate solution, and was extracted. The organic layer was concentrated, and the residue was added with ethyl acetate and methanol, and the resulting precipitate was collected by filtration to obtain Compound 57 (0.17 mg, 76%).
¹H-NMR (270 MHz, CDCl₃) δ 2.90 (brt, 4H), 3.87 (brt, 4H), 7.01 (s, 2H), 7.15-7.29 (m, 4H), 7.32 (d, *J* = 16.8 Hz, 1H), 7.42 (t, *J* = 6.6 Hz, 1H), 7.43 (d, *J* = 16.8 Hz, 1H), 7.49 (d, *J* = 8.3 Hz, 1H), 8.03 (d, *J* = 8.6 Hz, 1H).
APCI-MS (m/z); 321 [M+H]⁺

### Example 58: (E)-3-{2-[4-(4-acetylpiperazin-1-yl)-3-aminophenyl]vinyl}-1H-indazole (Compound 58)

### Step 1

In a similar manner to Step 1 of Example 57, 4-(4-acetylpiperazin-1-yl)-3-nitrobenzaldehyde (0.86 g, quantitative yield) was obtained using 4-fluoro-3-nitrobenzaldehyde (0.52 g, 3.1 mmol), 1-acetylpiperazine (0.59 g, 4.6 mmol) and diisopropylethylamine (1.1 mL, 6.2 mmol).
¹H-NMR (270 MHz, CDCl₃) δ 2.05 (s, 3H), 3.06 (brt, *J* = 4.8 Hz, 4H), 3.61 (brt, *J* = 4.8 Hz, 4H), 7.37 (d, *J* = 8.7 Hz, 1H), 7.71 (dd, *J* = 8.7, 2.1 Hz, 1H), 8.00 (s, 1H), 8.02 (d, *J* = 5.9 Hz, 1H).
APCI-MS (m/z); 278 [M+H]⁺

### Step 2

A methanol (20 mL) solution of 4-(4-acetylpiperazin-1-yl)-3-nitrobenzaldehyde (0.86 g, 3.1 mmol) was added with (1H-indazol-3-ylmethyl)triphenylphosphonium iodide (1.9 g, 3.7 mmol) and potassium carbonate (1.3 g, 9.3 mmol), followed by stirring at room temperature for 6 hours. The reaction mixture was concentrated under reduced pressure, and the residue was added with ethyl acetate and water, and was extracted. The organic layer was concentrated, and the residue was added with ethyl acetate, and the resulting precipitate was collected by filtration to obtain (*E*)-3-{2-[4-(4-acetylpiperazin-1-yl)-3-nitrophenyl]vinyl}-1*H*-indazole (0.93 g, 77%).
¹H-NMR (270 MHz, DMSO-d₆) δ 2.03 (s, 3H), 2.97-3.08 (brm, 4H), 3.56 (brt, 4H), 7.20 (t, *J* = 7.5 Hz, 1H), 7.34 (d, *J* = 8.3 Hz, 1H), 7.39 (t, *J* = 7.5 Hz, 1H), 7.51 (d, *J* = 17.1 Hz, 1H), 7.54 (d, *J* = 8.3 Hz, 1H), 7.59 (d, *J* = 17.1 Hz, 1H), 7.97 (dd, *J* = 8.8, 2.0 Hz, 1H), 8.19 (d, *J* = 2.0 Hz, 1H), 8.22 (d, *J* = 7.9 Hz, 1H), 13.18 (s, 1H).
APCI-MS (m/z); 392 [M+H]⁺

### Step 3

An ethanol (15 mL) solution of (*E*)-3-{2-[4-(4-acetylpiperazin-1-yl)-3-nitrophenyl]vinyl}-1*H*-indazole (0.93 g, 2.4 mmol) was added with concentrated hydrochloric acid (15 mL) and tin (0.84 g, 7.1 mmol) at 0°C, followed by stirring at room temperature for 40 minutes. The reaction mixture was concentrated under reduced pressure, and the residue was added with ethyl acetate and a saturated aqueous sodium hydrogencarbonate solution, and was extracted. The organic layer was concentrated, and the residue was added with ethyl acetate, and the resulting precipitate was collected by filtration to obtain Compound 58 (0.28 g, 32%).
¹H-NMR (270 MHz, CDCl₃) δ 2.35 (s, 3H), 3.20-3.32 (brs, 2H), 3.32-3.43 (brs, 2H), 3.44-3.59 (brs, 2H), 3.70-3.87 (brs, 2H), 7.19 (d, *J* = 8.4 Hz, 1H), 7.26 (d, *J* = 19.1 Hz, 1H), 7.22-7.34 (m, 3H), 7.48 (s, 2H), 7.48 (d, *J* = 19.1 Hz, 1H), 7.40-7.52 (m, 2H), 8.04 (d, *J* = 8.1 Hz, 1H).
APCI-MS (m/z); 362 [M+H]⁺

### Example 59: (E)-3-{2-[3-amino-4-(piperazin-1-yl)phenyl]vinyl}-1H-indazole (Compound 59)

### Step 1

In a similar manner to Step 1 of Example 57, 4-[4-(tert-butoxycarbonyl)piperazin-1-yl]-3-nitrobenzaldehyde (1.1 g, quantitative yield) was obtained using 4-fluoro-3-nitrobenzaldehyde (0.57 g, 3.4 mmol), 1-*tert-*butoxycarbonylpiperazine (0.94 g, 5.0 mmol) and diisopropylethylamine (1.2 mL, 6.7 mmol).
¹H-NMR (270 MHz, CDCl₃) δ 1.41 (s, 9H), 3.20-3.50 (brm, 8H), 7.39 (d, *J* = 8.7 Hz, 1H), 7.97 (dd, *J* = 8.7, 2.0 Hz, 1H), 8.35 (d, *J* = 2.0 Hz, 1H), 9.85 (s, 1H).

### Step 2

In a similar manner to Step 2 of Example 57, (*E*)-3-{2-[4-(4-(*tert*-butoxycarbonyl)piperazin-1-yl)-3-nitrophenyl]vinyl}-1*H*-indazole (0.60 g, 40%) was obtained using 4-[4-(*tert*-butoxycarbonyl)piperazin-1-yl]-3-nitrobenzaldehyde (1.1 g, 3.4 mmol), (1*H*-indazol-3-ylmethyl)triphenylphosphonium iodide (1.9 g, 3.7 mmol) and potassium carbonate (1.4 g, 10 mmol).
¹H-NMR (270 MHz, CDCl₃) δ 1.49 (s, 9H), 3.06 (brt, 4H), 3.56 (brt, 4H), 7.15 (t, *J* = 8.4 Hz, 1H), 7.27 (t, *J* = 7.3 Hz, 1H), 7.40 (d, *J* = 17.2 Hz, 1H), 7.44 (t, *J* = 7.3 Hz, 1H), 7.47 (d, *J* = 17.2 Hz, 1H), 7.51 (d, *J* = 8.4 Hz, 1H), 7.71 (dd, *J* = 8.4, 2.2 Hz, 1H), 8.01 (d, *J* = 2.2 Hz, 1H), 8.02 (d, *J* = 8.4 Hz, 1H), 10.11 (s, 1H).

### Step 3

In a similar manner to Step 3 of Example 57, Compound 59 (29 mg, 6.7%) was obtained using (*E*)-3-{2-[4-(4-(*tert-*butoxycarbonyl)piperazin-1-yl)-3-nitrophenyl]vinyl}-1*H-*indazole (0.61 g, 1.4 mmol) obtained in Step 2, concentrated hydrochloric acid (10 mL) and tin (0.48 g, 4.0 mmol).
¹H-NMR (270 MHz, DMSO-*d*₆) δ 2.53 (brs, 4H), 2.81 (brs, 4H), 4.71 (s, 2H), 6.88 (brs, 2H), 7.00 (s, 1H), 7.17 (t, *J* = 7.6 Hz, 1H), 7.24 (d, *J* = 17.6 Hz, 1H), 7.28 (d, *J* = 6.9 Hz, 1H), 7.30 (d, *J* = 17.6 Hz, 1H), 7.36 (t, *J* = 7.6 Hz, 1H), 7.52 (d, *J* = 8.4 Hz, 1H), 8.09 (d, *J* = 8.6 Hz, 1H), 13.0 (s, 1H).
APCI-MS (m/z); 320 [M+H]⁺

### Example 60: (E)-N-{5-[2-(1H-Indazol-3-yl)vinyl]-2-(2-morpholinoethoxy)phenyl}methanesulfonamide (Compound 60)

### Step 1

A solution of 4-hydroxy-3-nitrobenzaldehyde (6.37 g, 38.1 mmol) in DMF (10.0 mL) was added with potassium carbonate (15.8 g, 114 mmol) and 2-(N-morpholino)ethyl chloride hydrochloride (7.09 g, 38.1 mmol), followed by stirring at 80°C for 7 hours. After cooling to room temperature, the reaction mixture was added with water (40 mL), was extracted with ethyl acetate (100 mL X 4), the organic layer was dried over anhydrous sodium sulfate, was treated with a 4 mol/L solution of hydrogen chloride in ethyl acetate to obtain 4-(2-morpholinoethoxy)-3-nitrobenzaldehyde hydrochloride (6.07 g, 50%).
¹H-NMR (270 MHz, DMSO-*d*₆) δ 3.24-4.00 (m, 10H), 4.78 (t, *J* = 4.7 Hz, 2H), 7.62 (d, *J* = 8.8 Hz, 1H), 8.22 (dd, *J* = 2.0, 8.8 Hz, 1H), 8.48 (d, *J* = 2.0 Hz, 1H), 9.97 (s, 1H), 11.6 (brs, 1H).
APCI-MS (m/z); 281 [M+H]⁺

### Step 2

A solution of 4-(2-morpholinoethoxy)-3-nitrobenzaldehyde hydrochloride obtained in Step 1 (2.97 g, 9.38 mmol) in dichloromethane (10.0 mL) was added with trimethyl orthoformate (15.0 mL, 13.7 mmol) and a 10% solution of hydrogen chloride in methanol (5.00 mL), followed by stirring at room temperature for 4 hours. The reaction mixture was added with potassium carbonate (10.0 g, 72.4 mmol), then stirred at room temperature for 5 hours, and the solvent was evaporated under reduced pressure. The residue was added with water (50 mL) and was extracted with ethyl acetate (50 mL X 3). After drying over anhydrous sodium sulfate, the solvent was evaporated under reduced pressure to obtain 4-dimethoxymethyl-1-(2-morpholinoethoxy)-2-nitrobenzene (2.98 g, 97%).
¹H-NMR (300 MHz, CDCl₃) δ 2.60 (brt, *J* = 4.7 Hz, 4H), 2.85 (t, *J* = 5.6 Hz, 2H), 3.32 (s, 6H), 3.71 (brt, *J* = 4.7 Hz, 4H), 4.25 (t, *J* = 5.6 Hz, 2H), 5.39 (s, 1H), 7.07 (d, *J* = 8.6 Hz, 1H), 7.59 (dd, *J* = 2.2, 8.6 Hz, 1H), 7.93 (d, *J* = 2.2 Hz, 1H).
ESI-MS (m/z); 327 [M+H]⁺

### Step 3

A solution of 4-dimethoxymethyl-1-(2-morpholinoethoxy)-2-nitrobenzene obtained in Step 2 (2.97 g, 9.10 mmol) in ethanol (30.0 mL) was added with platinum oxide (0.02 g, 0.10 mmol), followed by stirring at room temperature under hydrogen atmosphere for 2 hours. The reaction mixture was filtrated, the solvent of filtrate was evaporated under reduced pressure, the resulting crude product was crystallized from mixed solvent of ethyl acetate/hexane (1/1) to obtain 5-dimethoxymethyl-2-(2-morpholinoethoxy)aniline (0.70 g, 26%).
¹H-NMR (300 MHz, CDCl₃) δ 2.57 (brt, *J* = 4.7 Hz, 4H), 2.78 (t, *J* = 5.7 Hz, 2H), 3.30 (s, 6H), 3.72 (brt, *J* = 4.7 Hz, 4H), 3.91 (brs, 2H), 4.12 (t, *J* = 5.7 Hz, 2H), 5.25 (s, 1H), 6.76-6.81 (m, 3H).
ESI-MS (m/z); 297 [M+H]⁺

### Step 4

A solution of 5-dimethoxymethyl-2-(2-morpholinoethoxy)aniline obtained in Step 3 (0.27 g, 0.91 mmol) in dichloromethane (2.00 mL) was added with methanesulfonyl chloride (0.11 mL, 1.42 mmol) and pyridine (0.50 mL, 6.18 mmol), followed by stirring at room temperature for 10 hours. The reaction mixture was added with hydrochloric acid (3 mol/L, 3.00 mL), was stirred at room temperature for 20 minutes, was neutralized with a saturated aqueous sodium bicarbonate solution (50 ml) and was extracted with ethyl acetate (50 mL X 3). The organic layer was dried over anhydrous sodium sulfate, the solvent was evaporated under reduced pressure. The crude product was purified by silica gel column chromatography (chloroform, then methanol/chloroform=1/6) to obtain 3-methanesulfonylamino-4-(2-morpholinoethoxy)benzaldehyde (0.28 g, 95%).
¹H-NMR (300 MHz, CDCl₃) δ 2.56 (brt, *J* = 4.6 Hz, 4H), 2.70 (t, *J* = 5.5 Hz, 2H), 2.99 (s, 3H), 3.80 (brt, *J* = 4.6 Hz, 4H), 4.28 (t, *J* = 5.5 Hz, 2H), 7.14 (d, *J* = 8.4 Hz, 1H), 7.71 (dd, *J* = 2.0, 8.4 Hz, 1H), 8.03 (d, *J* = 2.0 Hz, 1H), 9.91 (s, 1H).
ESI-MS (m/z); 327 [M-H]⁻

### Step 5

A solution of 3-methanesulfonylamino-4-(2-morpholinoethoxy)benzaldehyde obtained in Step 4 (0.27 g, 0.83 mmol) in DMF (5.0 mL) was added with (1*H*-indazol-3-ylmethyl)triphenylphosphonium iodide obtained in Step 4 of Example 1 (0.40 g, 0.77 mmol) and potassium carbonate (0.23 g, 1.66 mmol), followed by stirring at room temperature for 2 hours, and further stirring at 60°C for 11 hours. After cooling to room temperature, the solvent was evaporated under reduced pressure, the resulting crude product was purified by silica gel chromatography [amino-modified chemically bound silica gel Chromatorex (registered trademark) NH, Fuji Silysia Chemical Ltd.; ethyl acetate, then methanol/ethyl acetate=1/19], and was crystallized from ethyl acetate to obtain Compound 60 (0.05 g, 14%).
¹H-NMR (300 MHz, CDCl₃) δ 2.58-2.61 (m, 6H), 2.94 (s, 3H), 3.87 (brt, *J* = 4.7 Hz, 4H), 4.23 (t, *J* = 5.6 Hz, 2H), 7.07 (d, *J* = 8.4 Hz, 1H), 7.23-7.29 (m, 1H), 7.39 (d, *J* = 16.5 Hz, 1H), 7.48 (d, *J* = 16.5 Hz, 1H), 7.40-7.51 (m, 3H), 7.77 (d, *J* = 2.2 Hz, 1H), 8.04 (d, *J* = 8.2 Hz, 1H).
ESI-MS (m/z); 443 [M+H]⁺

### Example 61: (E)-N-(2-morpholinoethyl)-4-[2-(1H-indazol-3-yl)vinyl]benzamide (Compound 61)

(*E*)-4-[2-(1*H*-indazol-3-yl)vinyl]benzoic acid (0.30 g, 1.14 mmol) obtained in Example 15 was dessolved in THF (20 mL), and the solution was added with 2-morpholinoethylamine (0.22 mL, 1.71 mmol), 1-hydroxybenzotriazole monohydrate (0.20 g, 1.31 mmol) and 1-ethyl-3-(3-dimethylaminopropyl)carbodiimide hydrochloride (0.31 g, 1.62 mmol), followed by stirring at 60°C for 4 hours. The reaction mixture was added with a saturated aqueous sodium hydrogencarbonate solution, and was extracted with ethyl acetate. The organic layer was sequentially washed with water and saturated brine, and was dried over anhydrous sodium sulfate, and the solvent was evaporated under reduced pressure. The residue was crystallized to obtain Compound 61 (0.24 g, 55%).
¹H-NMR (270 MHz, CDCl₃) d 2.54 (brt, *J* = 4.6 Hz, 4H), 2.64 (t, *J* = 6.0 Hz, 2H), 3.59 (q, *J* = 5.5 Hz, 2H), 3.76 (brt, *J* = 4.8 Hz, 4H), 6.92 (brm, 1H), 7.26 (t, *J* = 6.6 Hz, 1H), 7.43 (t, *J* = 7.3 Hz, 2H), 7.51 (d, *J* = 8.4 Hz, 2H), 7.63 (d, *J* = 8.4 Hz, 2H), 7.79 (d, *J* = 8.4 Hz, 2H), 8.04 (d, *J* = 8.1 Hz, 1H).
ESI-MS (m/z); 377 [M+H]⁺

### Example 62: (E)-N,N-Diethyl-4-[2-(1H-indazol-3-yl)vinyl]benzamide hydrochloride (Compound 62)

In a similar manner to Example 61, a free base of Compound 62 was obtained using Compound 15 (0.50 g, 1.89 mmol), diethylamine (0.29 mL, 2.80 mmol) and 1-ethyl-3-(3-dimethylaminopropyl)carbodiimide hydrochloride (0.51 g, 2.67 mmol). Then, the free base of Compound 62 was dissolved in acetic acid (5 mL) and was added with a 4 mol/L solution of hydrogen chloride in ethyl acetate (1.00 mL), followed by stirring at room temperature for 30 minutes. The resulting precipitates were collected by filtration to obtain Compound 62 (0.11 g, 18%).
¹H-NMR (270 MHz, DMSO-*d*₆) δ 1.10 (brm, 6H), 3.27-3.37 (brm, 4H), 7.21 (t, *J* = 7.9 Hz, 1H), 7.35 (d, *J* = 8.1 Hz, 2H), 7.38 (t, *J* = 6.9 Hz, 1H), 7.49-7.64 (m, 3H), 7.75 (d, *J* = 8.2 Hz, 2H), 8.19 (d, *J* = 8.2 Hz, 1H), 10.9 (br, 1H).
ESI-MS (m/z); 320 [M+H]⁺

### Example 63: (E)-4-{4-[2-(1H-indazol-3-yl)vinyl]benzoyl}piperazine-1-carboxylic acid 1,1-dimethylethylester (Compound 63)

In a similar manner to Example 61, Compound 63 (0.27 g, 33%) was obtained using Compound 15 (0.50 g, 1.89 mmol), 4-(tert-butoxycarbonyl)piperazine (0.53 g, 2.84 mmol), 1-hydroxybenzotriazole monohydrate (0.33 g, 2.17 mmol) and 1-ethyl-3-(3-dimethylaminopropyl)carbodiimide hydrochloride (0.51 g, 2.66 mmol).
¹H-NMR (270 MHz, DMSO-*d*₆) d 1.41 (s, 9H), 3.23-3.57 (brm, 8H), 7.20 (t, *J* = 7.2 Hz, 1H), 7.39 (t, *J* = 7.2 Hz, 1H), 7.42 (d, *J* = 8.3 Hz, 2H), 7.53 (d, *J* = 16.7 Hz, 1H), 7.55 (d, *J* = 8.2 Hz, 1H), 7.63 (d, *J* = 16.7 Hz, 1H), 7.78 (d, *J* = 8.2 Hz, 2H), 8.19 (d, *J* = 8.2 Hz, 1H), 13.2 (brs, 1H).
ESI-MS (m/z); 431 [M-H]⁻

### Example 64: (E)-1-{4-[2-(1H-indazol-3-yl)vinyl]benzoyl}piperazine (Compound 64)

### Step 1

To the solution of Compound 63 (0.27 g, 0.62 mmol) in methanol (2 mL), 10% hydrogen chloride-methanol solution (2 mL) was added, followed by stirring at 60°C for 30 minutes. After cooling to room temperature, methanol was evaporated to obtain dihydrochloride of Compound 64 (0.21 g, 74%).

### Step 2

Dihydrochloride of Compound 64 (0.10 g) obtained in Step 1 was added with chloroform (20 mL) and saturated aqueous sodium hydrogencarbonate solution (20 mL), followed by stirring at room temperature for 1 hour, then the reaction mixture was extracted with chloroform. The organic layer was washed with saturated brine and dried over anhydrous sodium sulfate, and the solvent was evaporated under reduced pressure. After the obtained residue was crystallized from ethyl acetate, the obtained crystal was washed with mixed solvent of ethyl acetate/hexane (1/1) to obtain Compound 64 (0.022 g, 27%).
¹H-NMR (270 MHz, CDCl₃) d 2.78-3.02 (m, 4H), 3.32-3.89 (m, 4H), 7.20-7.52 (m, 7H), 7.60 (d, *J* = 8.4 Hz, 2H), 8.03 (d, *J* = 8.1 Hz, 1H), 10.8 (brs, 1H).
APCI-MS (m/z); 333 [M+H]⁺

### Example 65: (E)-4-{4-[2-(1H-indazol-3-yl)vinyl]benzoyl}piperazine-1-carbaldehyde (Compound 65)

In a similar manner to Example 61, Compound 65 (0.34 g, 99%) was obtained using Compound 64 (0.40 g, 0.95 mmol), formic acid (0.03 mL), 1-hydroxybenzotriazole monohydrate (0.27 g, 1.96 mmol) and 1-ethyl-3-(3-dimethylaminopropyl)carbodiimide hydrochloride (0.17 g, 0.90 mmol).
¹H-NMR (270 MHz, DMSO-*d*₆) d 3.45-3.57 (brm, 8H), 7.21 (t, *J* = 7.2 Hz, 1H), 7.40 (t, *J* = 7.2 Hz, 1H), 7.45 (d, *J* = 8.3 Hz, 2H), 7.53 (d, *J* = 16.6 Hz, 1H), 7.54 (d, *J* = 8.2 Hz, 1H), 7.64 (d, *J* = 16.6 Hz, 1H), 7.79 (d, *J* = 8.2 Hz, 2H), 8.06 (s, 1H), 8.20 (d, *J* = 8.2 Hz, 1H), 13.2 (brs, 1H).
ESI-MS (m/z); 361 [M+H]⁺

### Example 66: (E)-1-{4-[2-(1H-indazol-3-yl)vinyl]benzoyl}-4-ethoxycarbonylpiperazine (Compound 66)

In a similar manner to Example 61, Compound 66 (346 mg, 45%) was obtained using Compound 15 (500 mg, 1.89 mmol), 1-piperazine carboxylic acid ethyl ester (0.42 mL, 2.84 mmol), 1-hydroxybenzotriazole monohydrate (332 mg, 2.46 mmol), 1-ethyl-3-(3-dimethylaminopropyl)carbodiimide hydrochloride (510 mg, 2.67 mmol) and N-methylmorpholine (0.42 mL, 3.82 mmol).
¹H-NMR (270 MHz, DMSO-*d*₆) d 1.18 (t, *J* = 7.1 Hz, 3H), 3.27-3.43 (m, 8H), 4.05 (q, *J* = 7.1 Hz, 2H), 7.21 (t, *J* = 7.1 Hz, 1H), 7.37-7.45 (m, 3H), 7.53-7.67 (m, 3H), 7.78 (d, *J* = 8.2 Hz, 2H), 8.19 (brd, *J* = 8.2 Hz, 1H), 13.2 (br, 1H).
ESI-MS (m/z); 405 [M+H]⁺

### Example 67: (E)-1-{4-[2-(1H-indazol-3-yl)vinyl]benzoyl}-4-(isonicotinoyl)piperazine (Compound 67)

In a similar manner to Example 61, Compound 67 (108 mg, 13%) was obtained using Compound 15 (477 mg, 1.81 mmol), 4-(isonicotinoyl)piperazine dihydrochloride (617 mg, 2.71 mmol), 1-hydroxybenzotriazole monohydrate (332 mg, 2.46 mmol), 1-ethyl-3-(3-dimethylaminopropyl)carbodiimide hydrochloride (510 mg, 2.67 mmol) and *N*-methylmorpholine (1.00 mL, 9.55 mmol).
¹H-NMR (270 MHz, DMSO-*d*₆) d 3.36-3.70 (m, 8H), 7.22 (t, 1H, *J* = 7.4 Hz), 7.38-7.67 (m, 8H), 7.80 (d, 2H, *J* = 8.1 Hz), 8.20 (d, 1H, *J* = 8.2 Hz), 8.68 (d, 2H, *J* = 5.8 Hz), 13.2 (br, 1H). ESI-MS (m/z); 438 [M+H]⁺

### Example 68: (E)-N-[2-(diethylamino)ethyl]-4-[2-(1H-indazol-3-yl)vinyl]benzamide (Compound 68)

In a similar manner to Example 61, Compound 68 (3.00 g, 55%) was obtained using (*E*)-4-[2-(1*H*-indazol-3-yl)vinyl]-2-methoxybenzoic acid (4.00 g, 15.1 mmol), *N,N-*diethylethylenediamine (2.60 mL, 18.2 mmol), 1-hydroxybenzotriazole monohydrate (3.00 g, 19.70 mmol), 1-ethyl-3-(3-dimethylaminopropyl)carbodiimide hydrochloride (4.10 g, 21.2 mmol) and *N*-methylmorpholine (3.40 mL, 30.3 mmol).
¹H-NMR (300 MHz, DMSO-d₆) d 0.97 (t, *J* = 7.5 Hz, 6H), 2.54 (m, 8H), 7.20 (m, 1H), 7.40 (m, 1H), 7.51-7.69 (m, 3H), 7.81 (dd, *J* = 18.3, 8.3 Hz, 4H), 8.20 (d, *J* = 8.1 Hz, 1H), 8.40 (m, 1H), 13.2 (s, 1H).
ESI-MS (m/z); 363 [M+H]⁺

### Example 69: (E)-4-methoxyacetyl-1-{4-[2-(1H-indazol-3-yl)vinyl]benzoyl}piperazine (Compound 69)

In a similar manner to Example 61, Compound 69 (225 mg, 62%) was obtained using dihydrochloride of Compound 64 (400 mg, 1.09 mmol), methoxyacetic acid (0.069 mL, 0.896 mmol), 1-hydroxybenzotriazole monohydrate (173 mg, 1.28 mmol), 1-ethyl-3-(3-dimethylaminopropyl)carbodiimide hydrochloride (265 mg, 1.38 mmol) and *N*-methylmorpholine (0.40 mL, 3.64 mmol).
¹H-NMR (270 MHz, DMSO-*d*₆) d 3.28 (s, 3H), 3.33-3.49 (m, 8H), 4.10 (s, 2H), 7.21 (t, *J* = 7.3 Hz, 1H), 7.38 (d, *J* = 8.1 Hz, 1H), 7.44 (d, *J* = 8.3 Hz, 2H), 7.51-7.67 (m, 3H), 7.79 (d, *J* = 8.3 Hz, 2H), 8.20 (d, *J* = 8.1 Hz, 1H), 13.2 (br, 1H).
ESI-MS (m/z); 405 [M+H]⁺

### Example 70: (E)-4-{4-[2-(1H-indazol-3-yl)vinyl]benzoyl}piperadin-2-one (Compound 70)

In a similar manner to Example 61, Compound 70 (309 mg, 60%) was obtained using Compound 15 (500 mg, 1.89 mmol), piperadin-2-one (284 mg, 2.83 mmol), 1-hydroxybenzotriazole monohydrate (332 mg, 2.46 mmol), 1-ethyl-3-(3-dimethylaminopropyl)carbodiimide hydrochloride (510 mg, 2.67 mmol) and N-methylmorpholine (850 mL, 7.73 mmol).
¹H-NMR (300 MHz, DMSO-*d*₆) d 3.20-3.28 (m, 2H), 3.55-3.64 (m, 2H), 4.00-4.04 (brs, 2H), 7.21 (t, *J* = 7.3 Hz, 1H), 7.40 (t, *J* = 7.3 Hz, 1H), 7.47 (d, *J* = 8.4 Hz, 2H), 7.52 (d, *J* = 16.1 Hz, 1H), 7.58 (d, *J* = 8.0 Hz, 1H), 7.68 (d, *J* = 16.1 Hz, 1H), 7.80 (d, *J* = 8.1 Hz, 2H), 8.13 (br, 1H), 8.20 (d, *J* = 8.1 Hz, 1H), 13.2 (br, 1H).
ESI-MS (m/z); 347 [M+H]⁺

### Example 71: (E)-1-{4-[2-(1H-indazol-3-yl)vinyl]benzoyl}-3-aminopyrrolidine dihydrochloride (Compound 71)

The product obtained using Compound 15 (800 mg, 2.66 mmol), (pyrrolidin-3-yl)carbamic acid *tert-*butyl ester (1.00 g, 5.32 mmol), 1-hydroxybenzotriazole monohydrate (470 mg, 3.46 mmol), 1-ethyl-3-(3-dimethylaminopropyl)carbodiimide hydrochloride (720 mg, 3.72 mmol) and *N*-methylmorpholine (0.58 mL, 5.32 mmol) in a similar manner to Example 61, was dissolved in methanol (5.00 mL), and the solution was added with 4 moL/L hydrogen chloride-methanol solution (2.00 mL), followed by heating under reflux at 60°C for 1 hour. The reaction mixture was concentrated under reduced pressure, and the residue was crystallized from acetone to obtain Compound 71 (398 mg, 38%).
¹H-NMR (270 MHz, DMSO-d₆) d 2.23 (br, 1H), 3.54-3.87 (m, 6H), 7.22 (dd, *J* = 7.7, 7.7 Hz, 1H), 7.41 (dd, *J* = 7.7, 7.7 Hz, 1H), 7.54 (d, *J* = 8.3 Hz, 2H), 7.55 (d, *J* = 8.3 Hz, 1H), 7.57 (d, *J* = 16.8 Hz, 1H), 7.66 (d, *J* = 16.8 Hz, 1H), 7.80 (d, *J* = 8.3 Hz, 2H), 8.21 (d, *J* = 8.3 Hz, 1H), 8.30 (br, 1H), 8.42 (br, 1H).
APCI-MS (m/z); 333 [M+H]⁺

### Example 72: (S)-(E)-1-{4-[2-(1H-indazol-3-yl)vinyl]benzoyl}-4-(pyrrolidin-3-ylcarbonyl)piperazine dihydrochloride

### (Compound 72)

The product obtained using Compound 64 (300 mg, 0.90 mmol), pyrrolidin-1,3-dicarboxylic acid 1-*tert*-butyl ester (162 mg, 0.76 mmol), 1-hydroxybenzotriazole monohydrate (133 mg, 0.99 mmol), 1-ethyl-3-(3-dimethylaminopropyl)carbodiimide hydrochloride (204 mg, 1.06 mmol) and *N*-methylmorpholine (0.17 mL, 1.52 mmol) in a similar manner to Example 61, was dissolved in methanol (2.00 mL), and the solution was added with 4 moL/L hydrogen chloride-methanol solution (1.50 mL), followed by heating under reflux at 60°C for 30 minutes. The reaction mixture was concentrated under reduced pressure, and the residue was crystallized from acetone to obtain Compound 72 (278 mg, 61%).
¹H-NMR (270 MHz, DMSO-*d*₆) d 1.78-1.94 (m, 2H), 2.40 (br, 1H), 3.15-3.26 (m,2H), 3.58 (br, 8H), 4.60 (br, 2H), 7.22 (dd, *J* = 7.1, 7.1 Hz, 1H), 7.41 (dd, *J* = 8.2, 8.2 Hz, 1H), 7.47 (d, *J* = 8.2 Hz, 2H), 7.54 (d, *J* = 8.2 Hz, 1H), 7.56 (d, *J* = 16.6 Hz, 1H), 7.66 (d, *J* = 16.6 Hz, 1H), 7.82 (d, *J* = 8.2 Hz, 2H), 8.21 (d, *J* = 8.2 Hz, 1H), 8.50 (br, 1H) 9.96-9.98 (m, 1H).
APCI-MS (m/z); 430 [M+H]⁺

### Example 73: (E)-4-[(isobutyrylamino)acetyl]-1-{4-[2-(1H-indazol-3-yl)vinyl]benzoyl}piperazine (Compound 73)

The crude product (174 mg) obtained using Compound 64 (200 mg, 0.602 mmol), N-methylmorpholine (0.132 mL, 1.20 mmol), 1-ethyl-3-(3-dimethylaminopropyl)carbodiimide hydrochloride (161 mg, 0.842 mmol), 1-hydroxybenzotriazole monohydrate (106 mg, 0.783 mmol) and (3-methylbutyrylamino)acetic acid (115 mg, 0.722 mmol) in a similar manner to Example 61, was purified by silica gel column chromatography (chloroform/methanol=80/20), followed by triturating by mixed solvent of ethyl acetate/ethanol to obtain Compound 73 (105 mg, 37%).
¹H-NMR (300 MHz, DMSO-d₆) d 0.89 (d, *J* = 6.0 Hz, 6H), 1.90-2.08 (m, 3H), 3.25-3.75 (br, 8H), 3.80-4.04 (br.s, 2H), 7.22 (t, *J* = 6.9 Hz, 1H), 7.41 (t, *J* = 6.9 Hz, 1H), 7.46 (d, *J* = 8.1 Hz, 2H), 7.56 (d, *J* = 16.8 Hz, 1H), 7.57 (d, *J* = 7.8 Hz, 1H), 7.66 (d, *J* = 16.8 Hz, 1H), 7.80 (d, *J* = 8.1 Hz, 2H), 7.91 (br, 1H), 8.21 (d, *J* = 7.8 Hz, 1H), 13.22 (s, 1H).
APCI-MS (m/z); 474 [M]⁺

### Example 74: (E)-4-(2-amino-2-methylpropionyl)-1-{4-[2-(1H-indazol-3-yl)vinyl]benzoyl}piperazine dihydrochrolide

### (Compound 74)

The product obtained using Compound 64 (300 mg, 0.90 mmol), 2-(*tert*-butoxycarbonyl)amino-2-methylpropionic acid (153 mg, 0.75 mmol), 1-hydroxybenzotriazole monohydrate (132 mg, 0.98 mmol), 1-ethyl-3-(3-dimethylaminopropyl)carbodiimide hydrochloride (202 mg, 1.05 mmol) and *N*-methylmorpholine (0.16 mL, 1.50 mmol) in a similar manner to Example 61, was dissolved in methanol (2.00 mL), and the solution was added with 4 moL/L hydrogen chloride-methanol solution (1.50 mL), followed by heating under reflux at 60°C for 30 minutes. The reaction mixture was concentrated under reduced pressure, and the residue was crystallized from acetone to obtain Compound 74 (0.10 mg, 23%).
¹H-NMR (270 MHz, DMSO-*d*₆) d 1.57 (br, 6H), 3.65 (br, 4H), 4.36 (br, 4H), 7.22 (dd, *J* = 7.9, 7.9 Hz, 1H), 7.41 (dd, *J* = 8.4, 8.4 Hz, 1H), 7.47 (d, *J* = 8.4 Hz, 2H), 7.54 (d, *J* = 7.9 Hz, 1H), 7.55 (d, *J* = 16.6 Hz, 1H), 7.66 (d, *J* = 16.6 Hz, 1H), 7.81 (d, *J* = 8.4 Hz, 2H), 8.21 (d, *J* = 8.4 Hz, 1H), 8.27 (br, 2H).
APCI-MS (m/z); 418 [M+H]⁺

### Example 75: (E)-4-(N-acetyl-N-methylaminoacetyl)-1-{4-[2-(1H-indazol-3-yl)vinyl]benzoyl}piperazine (Compound 75)

The crude product of dihydrochloride of Compound 64 (300 mg, 0.741 mmol), *N*-methylmorpholine (0.163 mL, 1.48 mmol), 1-ethyl-3-(3-dimethylaminopropyl)carbodiimide hydrochloride (199 mg, 1.04 mmol), 1-hydroxybenzotriazole monohydrate (130 mg, 0.963 mmol) and *N*-acetylsarcosine (77.7 mg, 0.593 mmol) in a similar manner to Example 61, was purified by silica gel column chromatography (chloroform/methanol=90/10), followed by triturating by mixed solvent of ethyl acetate/methanol to obtain Compound 75 (54.8 mg, 25%).
¹H-NMR (270 MHz, DMSO-*d*₆) d 2.02 (s, 3H), 2.97 (s, 3H), 3.42-3.60 (br, 8H), 4.18 (s, 2H), 7.22 (t, *J* = 7.6 Hz, 1H), 7.41 (d, *J* = 7.6 Hz, 1H), 7.46 (d, *J* = 8.1 Hz, 2H), 7.55 (d, *J* = 16.7 Hz, 1H), 7.56 (d, *J* = 8.1 Hz, 1H), 7.66 (d, *J* = 16.7 Hz, 1H), 7.80 (d, *J* = 8.1 Hz, 2H), 8.21 (d, *J* = 8.1 Hz, 1H), 13.21 (s, 1H).
APCI-MS (m/z); 446 [M]⁺

### Example 76: (S)-(E)-4-[(pyrrolidin-2-yl)carbonyl]-1-{4-[2-(1H-indazol-3-yl)vinyl]benzoyl}piperazine (Compound 76)

The product obtained using dihydrochloride of Compound 64 (100 mg, 0.25 mmol), *N*-(*tert*-butoxycarbonyl)-L-proline (53 mg, 0.25 mmol), 1-hydroxybenzotriazole monohydrate (43 mg, 0.33 mmol), 1-ethyl-3-(3-dimethylaminopropyl)carbodiimide hydrochloride (66 mg, 0.35 mmol) and N-methylmorpholine (0.06 mL, 0.50 mmol) in a similar manner to Example 61, was dissolved in methanol (2.00 mL), and the solution was added with 4 moL/L hydrogen chloride-methanol solution (1.50 mL) followed by heating under reflux at 60°C for 30 minutes. The reaction mixture was concentrated under reduced pressure, and the residue was added with saturated sodium hydrogencarbonate solution and ethyl acetate, followed by extraction. The crude product was crystallized from acetone to obtain Compound 76 (21 mg, 20%).
¹H-NMR (270 MHz, DMSO-*d*₆) d 1.85-1.91 (m, 4H), 2.32-2.36 (m, 2H), 3.14-3.21 (m, 4H), 3.57 (br, 4H), 4.52 (br, 1H), 7.22 (dd, *J* = 7.4, 7.4 Hz, 1H), 7.41 (dd, *J* = 8.2, 8.2 Hz, 1H), 7.47 (d, *J* = 8.2 Hz, 2H), 7.53 (d, *J* = 7.4 Hz, 1H), 7.56 (d, *J* = 16.6 Hz, 1H), 7.66 (d, *J* = 16.6 Hz, 1H), 7.81 (d, *J* = 8.2 Hz, 2H), 8.21 (d, *J* = 8.2 Hz, 1H), 13.2 (br, 1H).
APCI-MS (m/z); 430 [M+H]⁺

### Example 77: (E)-4-[(1-methylpiperidin-4-yl)carbonyl]-1-{4-[2-(1H-indazol-3-yl)vinyl]benzoyl}piperazine (Compound 77)

In a similar manner to Example 61, Compound 77 (0.02 mg, 9%) was obtained using dihydrochloride of Compound 64 (200 mg, 0.49 mmol), 1-methylpiperidin-4-carboxylic acid (70 mg, 0.49 mmol), 1-hydroxybenzotriazole monohydrate (86 mg, 0.64 mmol), 1-ethyl-3-(3-dimethylaminopropyl)carbodiimide hydrochloride (132 mg, 0.69 mmol) and *N*-methylmorpholine (0.10 mL, 0.98 mmol).
¹H-NMR (270 MHz, DMSO-*d*₆) d 1.58-1.59 (m, 4H), 1.91 (br, 2H), 2.15 (s, 3H), 2.76-2.80 (m, 2H), 3.51 (br, 9H), 7.22 (dd, *J* = 7.7, 7.7 Hz, 1H), 7.39 (dd, *J* = 7.7, 7.7 Hz, 1H), 7.45 (d, *J* = 8.4 Hz, 2H), 7.53 (d, *J* = 8.4 Hz, 1H), 7.55 (d, *J* = 16.6 Hz, 1H), 7.65 (d, *J* = 16.6 Hz, 1H), 7.80 (d, *J*= 8.4 Hz, 2H), 8.21 (d, *J* = 8.4 Hz, 1H), 13.2 (br, 1H).
ESI-MS (m/z); 458 [M+H]⁺

### Example 78: (E)-1-{4-[2-(1H-indazol-3-yl)vinyl]benzoyl}-4-aminopiperidine (Compound 78)

The product obtained using Compound 15 (500 mg, 1.89 mmol), 4-(*tert*-butoxycarbonylamino)piperidine (538 mg, 2.84 mmol), 1-hydroxybenzotriazole monohydrate (332 mg, 2.46 mmol), 1-ethyl-3-(3-dimethylaminopropyl)carbodiimide hydrochloride (510 mg, 2.67 mmol) and *N*-methylmorpholine (0.420 mL, 3.82 mmol) in a similar manner to Example 61, was dissolved in methanol (10.0 mL), and the solution was added with 4 moL/L hydrogen chloride-methanol solution (5.0 mL), followed by heating under reflux at 60°C for 90 minutes. The reaction mixture was concentrated under reduced pressure, the residue was added with saturated aqueous potassium carbonate solution and ethyl acetate and then extracted. The crude product was crystallized from ethyl acetate to obtain Compound 78 (435 mg, 66%).
¹H-NMR (270 MHz, DMSO-*d*₆) d 1.15-1.20 (m, 2H), 1.72-2.00 (m, 2H), 2.79-3.60 (m, 7H), 7.22 (dd, *J* = 7.9, 7.9 Hz, 1H), 7.38 (d, *J* = 8.3 Hz, 2H), 7.37-7.43 (m, 1H), 7.54 (d, *J* = 16.9 Hz, 1H), 7.56 (d, *J* = 7.9 Hz, 1H), 7.63 (d, *J* = 16.9 Hz, 1H), 7.78 (d, *J* = 8.3 Hz, 2H), 8.21 (d, *J* = 8.3 Hz, 1H).
ESI-MS (m/z); 347 [M+H]⁺

### Example 79: Preparation Example (Tablet)

Tablets having the following composition are prepared according to a conventional procedure.

| | | |
|---|---|---|
| Compound 2 | 5 mg | |
| Lactose | 60 mg | |
| Potato starch | 30 mg | |
| Poly(vinyl alcohol) | 2 mg | |
| Magnesium stearate | 1 mg | |
| Tar pigment | trace amount | |

### Industrial Applicability

The present invention provides protein kinase inhibitor and the like which comprises, as an active ingredient, indazole derivatives or pharmaceutically acceptable salts thereof.

## Claims

1. A protein kinase inhibitor (excluding c-jun N-terminal kinase inhibitor) which comprises, as an active ingredient, an indazole derivative represented by Formula (I) 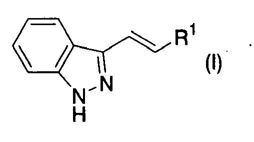 (wherein R¹ represents substituted or unsubstituted aryl or a substituted or unsubstituted heterocyclic group) or a pharmaceutically acceptable salt thereof.

2. A protein kinase inhibitor (excluding c-jun N-terminal kinase inhibitor) which comprises, as an active ingredient, an indazole derivative represented by Formula (Ia) 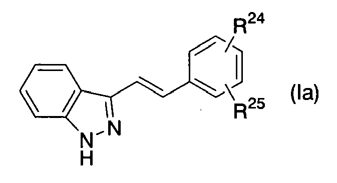 [wherein R²⁴ represents CONR^{24a}R^{24b} (wherein R^{24a} and R^{24b} may be the same or different and each represents a hydrogen atom, substituted or unsubstituted lower alkyl, substituted or unsubstituted aryl, substituted or unsubstituted aralkyl, or a substituted or unsubstituted heterocyclic group, or R^{24a} and R^{24b} are combined together with the adjacent nitrogen atom thereto to form a substituted or unsubstituted heterocyclic group) or NR^{24c}R^{24d} (wherein R^{24c} represents substituted or unsubstituted lower alkylsulfonyl or substituted or unsubstituted arylsulfonyl and R^{24d} represents a hydrogen atom or substituted or unsubstituted lower alkyl) and R²⁵ represents a hydrogen atom, halogen, cyano, nitro, hydroxy, carboxy, lower alkoxycarbonyl, substituted or unsubstituted lower alkyl, substituted or unsubstituted lower alkoxy, substituted or unsubstituted lower alkanoyl, CONR ^{25a}R^{25b} (wherein R^{25a} and R^{25b} may be the same or different and each represents a hydrogen atom, substituted or unsubstituted lower alkyl, substituted or unsubstituted aryl, substituted or unsubstituted aralkyl or a substituted or unsubstituted heterocyclic group, or R^{25a} and R^{25b} are combined together with the adjacent nitrogen atom thereto to form a substituted or unsubstituted heterocyclic group) or NR^{25c}R^{25d} (wherein R^{25c} and R^{25d} may be the same or different and each represents a hydrogen atom, substituted or unsubstituted lower alkyl, substituted or unsubstituted lower alkanoyl, substituted or unsubstituted aroyl, substituted or unsubstituted heteroaroyl, substituted or unsubstituted aralkyl, substituted or unsubstituted lower alkylsulfonyl or substituted or unsubstituted arylsulfonyl)], or a pharmaceutically acceptable salt thereof.

3. The protein kinase inhibitor (excluding c-jun N-terminal kinase inhibitor) according to Claim 2, wherein R²⁴ is CONR^{24a}R^{24b} (wherein R^{24a} and R^{24b} have the same meanings as defined above, respectively) and R²⁵ is a hydrogen atom.

4. The protein kinase inhibitor (excluding c-jun N-terminal kinase inhibitor) according to Claim 2, wherein R²⁴ is NR^{24c}R^{24d} (wherein R^{24c} and R^{24d} have the same meanings as defined above, respectively) and R²⁵ is substituted or unsubstituted lower alkoxy.

5. The protein kinase inhibitor (excluding c-jun N-terminal kinase inhibitor) according to Claim 2, wherein R²⁴ is NR^{24c}R^{24d} (wherein R^{24c} and R^{24d} have the same meanings as defined above, respectively) and R²⁵ is a hydrogen atom.

6. The protein kinase inhibitor according to any of Claims 1 to 5, wherein protein kinase is Fms like tyrosine kinase 3.

7. An anticancer agent which comprises, as an active ingredient, the indazole derivative or the pharmaceutically acceptable salt thereof described in Claim 1.

8. The anticancer agent according to Claim 7, wherein cancer is cancer of hematopoietic tumor, mammary cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, oral cavity and pharynx cancer, osteosarcoma, melanoma or brain neoplasm.

9. The anticancer agent according to Claim 7, wherein cancer is leukemia, myeloma or lymphoma.

10. The anticancer agent according to Claim 7, wherein cancer is solid carcinoma.

11. An antitumor agent, which comprises, as an active ingredient, the indazole derivative or the pharmaceutically acceptable salt thereof described in Claim 1.

12. The antitumor agent according to Claim 11, wherein tumor is hematopoietic tumor.

13. The antitumor agent according to Claim 11, wherein tumor is solid tumor.

14. Use of the indazole derivative or the pharmaceutically acceptable salt thereof described in any of Claims 1 to 5 for the manufacture of a protein kinase inhibitor (excluding c-jun N-terminal kinase inhibitor).

15. Use of the indazole derivative or the pharmaceutically acceptable salt thereof described in any of Claims 1 to 5 for the manufacture of an Fms like tyrosine kinase 3 inhibitor.

16. Use of the indazole derivative or the pharmaceutically acceptable salt thereof described in Claim 1 for the manufacture of an anticancer agent.

17. Use of the indazole derivative or the pharmaceutically acceptable salt thereof described in Claim 1 for the manufacture of a therapeutic agent for cancer of hematopoietic tumor, mammary cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, oral cavity and pharynx cancer, osteosarcoma, melanoma or brain neoplasm.

18. Use of the indazole derivative or the pharmaceutically acceptable salt thereof described in Claim 1 for the manufacture of a therapeutic agent for leukemia, myeloma or lymphoma.

19. Use of the indazole derivative or the pharmaceutically acceptable salt thereof described in Claim 1 for the manufacture of a therapeutic agent for solid carcinoma.

20. Use of the indazole derivative or the pharmaceutically acceptable salt thereof described in Claim 1 for the manufacture of an antitumor agent.

21. Use of the indazole derivative or the pharmaceutically acceptable salt thereof described in Claim 1 for the manufacture of a therapeutic agent for hematopoietic tumor.

22. Use of the indazole derivative or the pharmaceutically acceptable salt thereof described in Claim 1 for the manufacture of a therapeutic agent for solid tumor.

23. A method for inhibiting protein kinase (excluding c-jun N-terminal kinase), comprising a step of administering an effective amount of the indazole derivative or the pharmaceutically acceptable salt thereof described in any of Claims 1 to 5.

24. A method for inhibiting Fms like tyrosine kinase 3, comprising a step of administering an effective amount of the indazole derivative or the pharmaceutically acceptable salt thereof described in any of Claims 1 to 5.

25. A method for treating cancer, comprising a step of administering an effective amount of the indazole derivative or the pharmaceutically acceptable salt thereof described in Claim 1.

26. A method for treating cancer of hematopoietic tumor, mammary cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, oral cavity and pharynx cancer, osteosarcoma, melanoma or brain neoplasm, comprising a step of administering an effective amount of the indazole derivative or the pharmaceutically acceptable salt thereof described in Claim 1.

27. A method for treating leukemia, myeloma or lymphoma, comprising a step of administering an effective amount of the indazole derivative or the pharmaceutically acceptable salt thereof described in Claim 1.

28. A method for treating solid carcinoma, comprising a step of administering an effective amount of the indazole derivative or the pharmaceutically acceptable salt thereof described in Claim 1.

29. A method for treating tumor, comprising a step of administering an effective amount of the indazole derivative or the pharmaceutically acceptable salt thereof described in Claim 1.

30. A method for treating hematopoietic tumor, comprising a step of administering an effective amount of the indazole derivative or the pharmaceutically acceptable salt thereof described in Claim 1.

31. A method for treating solid tumor, comprising a step of administering an effective amount of the indazole derivative or the pharmaceutically acceptable salt thereof described in Claim 1.

32. An indazole derivative represented by Formula (II) 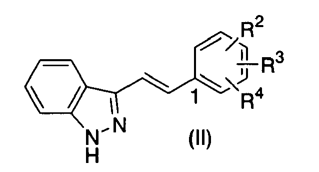 {wherein R², R³ and R⁴ may be the same or different and each represents a hydrogen atom, halogen, nitro, nitroso, carboxy, cyano, substituted or unsubstituted lower alkyl, substituted or unsubstituted lower alkanoyl, substituted or unsubstituted lower alkoxycarbonyl, substituted or unsubstituted aryl, NR⁵R⁶ (wherein R⁵ and R⁶ may be the same or different and each represents a hydrogen atom, substituted or unsubstituted lower alkyl, substituted or unsubstituted lower alkenyl, substituted or unsubstituted lower alkynyl, substituted or unsubstituted lower alkoxy, substituted or unsubstituted lower alkanoyl, substituted or unsubstituted aryl, substituted or unsubstituted aroyl, a substituted or unsubstituted heterocyclic group or substituted or unsubstituted heteroaroyl, or R⁵ and R⁶ are combined together with the adjacent nitrogen atom thereto to form a substituted or unsubstituted heterocyclic group) or OR⁷ (wherein R⁷ represents a hydrogen atom, substituted or unsubstituted lower alkyl, substituted or unsubstituted aryl, substituted or unsubstituted aroyl or a substituted or unsubstituted heterocyclic group) with the proviso that when two of R², R³ and R⁴ are hydrogen atoms, another one is not nitro, hydroxy, methoxy or 2-(dimethylamino)ethoxy bonding to 4-position of benzene ring [1-position of benzene ring is the position combined with (E)-2-(1H-indazol-3-yl)vinyl group]}, or a pharmaceutically acceptable salt thereof.

33. The indazole derivative or the pharmaceutically acceptable salt thereof according to Claim 32, wherein at least one of R², R³ and R⁴ is NR⁵R⁶ (wherein R⁵ and R⁶ have the same meanings as defined above, respesctively).

34. The indazole derivative or the pharmaceutically acceptable salt thereof according to Claim 32, wherein at least one of R², R³ and R⁴ is NR^{5a}R^{6a} (wherein R^{5a} and R^{6a} may be the same or different and each represents a hydrogen atom or substituted or unsubstituted lower alkanoyl, or R^{5a} and R^{6a} are combined together with adjacent nitrogen atom thereto to form a substituted or unsubstituted heterocyclic group).

35. The indazole derivative or the pharmaceutically acceptable salt thereof according to Claim 32, wherein at least one of R², R³ and R⁴ is OR⁷ (wherein R⁷ has the same meaning as defined above).

36. The indazole derivative or the pharmaceutically acceptable salt thereof according to Claim 32, wherein at least one of R², R³ and R⁴ is OR^{7a} (wherein R^{7a} represents substituted or unsubstituted lower alkyl).

37. An indazole derivative represented by Formula (II-1) 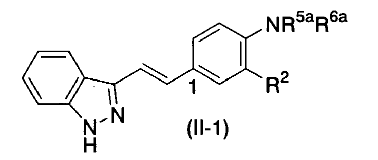 (wherein R², R^{5a} and R^{6a} have the same meanings as defind above, respectively) or a pharmaceutically acceptable salt thereof.

38. An indazole derivative represented by Formula (II-2) 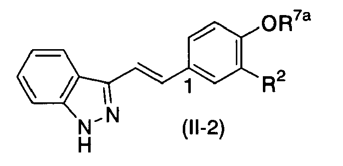 (wherein R² and R^{7a} have the same meanings as defind above, respectively) or a pharmaceutically acceptable salt thereof with the proviso that when R² is a hydrogen atom, R^{7a} is neither methyl nor 2-(dimethylamino)ethyl.

39. A protein kinase inhibitor (excluding c-jun N-terminal kinase inhibitor) which comprises, as an active ingredient, the indazol derivative or the pharmaceutically acceptable salt thereof according to any of Claims 32 to 38.

40. The protein kinase inhibitor according to Claim 39, wherein protein kinase is Fms like tyrosine kinase 3.

41. A pharmaceutical composition which comprises, as an active ingredient, the indazol derivative or the pharmaceutically acceptable salt thereof according to any of Claims 32 to 38.

42. An anticancer agent which comprises, as an active ingredient, the indazol derivative or the pharmaceutically acceptable salt thereof according to any of Claims 32 to 38.

43. The anticancer agent according to Claim 42, wherein cancer is cancer of hematopoietic tumor, mammary cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, oral cavity and pharynx cancer, osteosarcoma, melanoma or brain neoplasm.

44. The anticancer agent according to Claim 42, wherein cancer is leukemia, myeloma or lymphoma.

45. The anticancer agent according to Claim 42, wherein cancer is solid carcinoma.

46. An antitumor agent, which comprises, as an active ingredient, the indazole derivative or the pharmaceutically acceptable salt thereof according to any of Claims 32 to 38.

47. The antitumor agent according to Claim 46, wherein tumor is hematopoietic tumor.

48. The antitumor agent according to Claim 46, wherein tumor is solid tumor.

49. Use of the indazole derivative or the pharmaceutically acceptable salt thereof according to any of Claims 32 to 38 for the manufacture of a protein kinase inhibitor (excluding c-jun N-terminal kinase inhibitor).

50. Use of the indazole derivative or the pharmaceutically acceptable salt thereof according to any of Claims 32 to 38 for the manufacture of an Fms like tyrosine kinase 3 inhibitor.

51. Use of the indazole derivative or the pharmaceutically acceptable salt thereof according to any of Claims 32 to 38 for the manufacture of an anticancer agent.

52. Use of the indazole derivative or the pharmaceutically acceptable salt thereof according to any of Claims 32 to 38 for the manufacture of a therapeutic agent for cancer of hematopoietic tumor, mammary cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, oral cavity and pharynx cancer, osteosarcoma, melanoma or brain neoplasm.

53. Use of the indazole derivative or the pharmaceutically acceptable salt thereof according to any of Claims 32 to 38 for the manufacture of a therapeutic agent for leukemia, myeloma or lymphoma.

54. Use of the indazole derivative or the pharmaceutically acceptable salt thereof according to any of Claims 32 to 38 for the manufacture of a therapeutic agent for solid carcinoma.

55. Use of the indazole derivative or the pharmaceutically acceptable salt thereof according to any of Claims 32 to 38 for the manufacture of an antitumor agent.

56. Use of the indazole derivative or the pharmaceutically acceptable salt thereof according to any of Claims 32 to 38 for the manufacture of a therapeutic agent for hematopoietic tumor.

57. Use of the indazole derivative or the pharmaceutically acceptable salt thereof according to any of Claims 32 to 38 for the manufacture of a therapeutic agent for solid tumor.

58. A method for inhibiting protein kinase (excluding c-jun N-terminal kinase), comprising a step of administering an effective amount of the indazole derivative or the pharmaceutically acceptable salt thereof according to any of Claims 32 to 38.

59. A method for inhibiting Fms like tyrosine kinase 3, comprising a step of administering an effective amount of the indazole derivative or the pharmaceutically acceptable salt thereof according to any of Claims 32 to 38.

60. A method for treating cancer, comprising a step of administering an effective amount of the indazole derivative or the pharmaceutically acceptable salt thereof according to any of Claims 32 to 38.

61. A method for treating cancer of hematopoietic tumor, mammary cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, oral cavity and pharynx cancer, osteosarcoma, melanoma or brain neoplasm, comprising a step of administering an effective amount of the indazole derivative or the pharmaceutically acceptable salt thereof according to any of Claims 32 to 38.

62. A method for treating leukemia, myeloma or lymphoma, comprising a step of administering an effective amount of the indazole derivative or the pharmaceutically acceptable salt thereof according to any of Claims 32 to 38.

63. A method for treating solid carcinoma, comprising a step of administering an effective amount of the indazole derivative or the pharmaceutically acceptable salt thereof according to any of Claims 32 to 38.

64. A method for treating tumor, comprising a step of administering an effective amount of the indazole derivative or the pharmaceutically acceptable salt thereof according to any of Claims 32 to 38.

65. A method for treating hematopoietic tumor, comprising a step of administering an effective amount of the indazole derivative or the pharmaceutically acceptable salt thereof according to any of Claims 32 to 38.

66. A method for treating solid tumor, comprising a step of administering an effective amount of the indazole derivative or the pharmaceutically acceptable salt thereof according to any of Claims 32 to 38.

67. An indazole derivative represented by Formula (III) 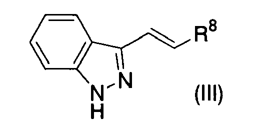 {wherein R⁸ represents a substituted or unsubstituted heterocyclic group [substituents in the substituted heterocyclic group may be the same or different, are 1 to 3 in number, and include oxo, formyl, carboxy, lower alkoxycarbonyl, substituted or unsubstituted lower alkyl, substituted or unsubstituted lower alkoxy, CONR⁹R¹⁰ (wherein R⁹ and R¹⁰ may be the same or different and each represents a hydrogen atom or substituted or unsubstituted lower alkyl), NR¹¹R¹² (wherein R¹¹ and R¹² may be the same or different and each represents a hydrogen atom, lower alkanoyl, lower alkoxycarbonyl, aralkyl, substituted or unsubstituted lower alkyl, substituted or unsubstituted aryl, substituted or unsubstituted aroyl or a substituted or unsubstituted heterocyclic group) or -O(CR¹³R¹⁴)ₙO- (wherein R¹³ and R¹⁴ may be the same or different and each represents a hydrogen atom or lower alkyl, n represents 2 or 3, and two terminal oxygen atoms are combined on the same carbon atom in the substituted heterocyclic group)]} or a pharmaceutically acceptable salt thereof.

68. The indazole derivative or the pharmaceutically acceptable salt thereof according to Claim 67, wherein substituents in the substituted heterocyclic group are amino, oxo, lower alkoxy, lower alkoxycarbonylamino, aroylamino or lower alkoxycarbonyl-lower alkyl.

69. The indazole derivative or the pharmaceutically acceptable salt thereof according to Claim 67, wherein R⁸ is 3-pyridyl.

70. A protein kinase inhibitor (excluding c-jun N-terminal kinase inhibitor) which comprises, as an active ingredient, the indazol derivative or the pharmaceutically acceptable salt thereof according to any of Claims 67 to 69.

71. The protein kinase inhibitor according to Claim 70, wherein protein kinase is Fms like tyrosine kinase 3.

72. A pharmaceutical composition which comprises, as an active ingredient, the indazol derivative or the pharmaceutically acceptable salt thereof according to any of Claims 67 to 69.

73. An anticancer agent which comprises, as an active ingredient, the indazol derivative or the pharmaceutically acceptable salt thereof according to any of Claims 67 to 69.

74. The anticancer agent according to Claim 73, wherein cancer is cancer of hematopoietic tumor, mammary cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, oral cavity and pharynx cancer, osteosarcoma, melanoma or brain neoplasm.

75. The anticancer agent according to Claim 73, wherein cancer is leukemia, myeloma or lymphoma.

76. The anticancer agent according to Claim 73, wherein cancer is solid carcinoma.

77. An antitumor agent, which comprises, as an active ingredient, the indazole derivative or the pharmaceutically acceptable salt thereof according to any of Claims 67 to 69.

78. The antitumor agent according to Claim 77, wherein tumor is hematopoietic tumor.

79. The antitumor agent according to Claim 77, wherein tumor is solid tumor.

80. Use of the indazole derivative or the pharmaceutically acceptable salt thereof according to any of Claims 67 to 69 for the manufacture of a protein kinase inhibitor (excluding c-jun N-terminal kinase inhibitor).

81. Use of the indazole derivative or the pharmaceutically acceptable salt thereof according to any of Claims 67 to 69 for the manufacture of an Fms like tyrosine kinase 3 inhibitor.

82. Use of the indazole derivative or the pharmaceutically acceptable salt thereof according to any of Claims 67 to 69 for the manufacture of an anticancer agent.

83. Use of the indazole derivative or the pharmaceutically acceptable salt thereof according to any of Claims 67 to 69 for the manufacture of a therapeutic agent for cancer of hematopoietic tumor, mammary cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, oral cavity and pharynx cancer, osteosarcoma, melanoma or brain neoplasm.

84. Use of the indazole derivative or the pharmaceutically acceptable salt thereof according to any of Claims 67 to 69 for the manufacture of a therapeutic agent for leukemia, myeloma or lymphoma.

85. Use of the indazole derivative or the pharmaceutically acceptable salt thereof according to any of Claims 67 to 69 for the manufacture of a therapeutic agent for solid carcinoma.

86. Use of the indazole derivative or the pharmaceutically acceptable salt thereof according to any of Claims 67 to 69 for the manufacture of an antitumor agent.

87. Use of the indazole derivative or the pharmaceutically acceptable salt thereof according to any of Claims 67 to 69 for the manufacture of a therapeutic agent for hematopoietic tumor.

88. Use of the indazole derivative or the pharmaceutically acceptable salt thereof according to any of Claims 67 to 69 for the manufacture of a therapeutic agent for solid tumor.

89. A method for inhibiting protein kinase (excluding c-jun N-terminal kinase), comprising a step of administering an effective amount of the indazole derivative or the pharmaceutically acceptable salt thereof according to any of Claims 67 to 69.

90. A method for inhibiting Fms like tyrosine kinase 3, comprising a step of administering an effective amount of the indazole derivative or the pharmaceutically acceptable salt thereof according to any of Claims 67 to 69.

91. A method for treating cancer, comprising a step of administering an effective amount of the indazole derivative or the pharmaceutically acceptable salt thereof according to any of Claims 67 to 69.

92. A method for treating cancer of hematopoietic tumor, mammary cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, oral cavity and pharynx cancer, osteosarcoma, melanoma or brain neoplasm, comprising a step of administering an effective amount of the indazole derivative or the pharmaceutically acceptable salt thereof according to any of Claims 67 to 69.

93. A method for treating leukemia, myeloma or lymphoma, comprising a step of administering an effective amount of the indazole derivative or the pharmaceutically acceptable salt thereof according to any of Claims 67 to 69.

94. A method for treating solid carcinoma, comprising a step of administering an effective amount of the indazole derivative or the pharmaceutically acceptable salt thereof according to any of Claims 67 to 69.

95. A method for treating tumor, comprising a step of administering an effective amount of the indazole derivative or the pharmaceutically acceptable salt thereof according to any of Claims 67 to 69.

96. A method for treating hematopoietic tumor, comprising a step of administering an effective amount of the indazole derivative or the pharmaceutically acceptable salt thereof according to any of Claims 67 to 69.

97. A method for treating solid tumor, comprising a step of administering an effective amount of the indazole derivative or the pharmaceutically acceptable salt thereof according to any of Claims 67 to 69.
